(19) Europäisches Patentamt
European Patent Office
Office européen des brevets

(11) EP 4 706 644 A1

(12) **EUROPEAN PATENT APPLICATION**
published in accordance with Art. 153(4) EPC

(43) Date of publication:
**11.03.2026 Bulletin 2026/11**

(21) Application number: **24800277.6**

(22) Date of filing: **03.05.2024**

(51) International Patent Classification (IPC):
*A61K 9/51* (2006.01) *A61K 31/575* (2006.01)
*A61P 35/00* (2006.01) *A61P 25/28* (2006.01)

(52) Cooperative Patent Classification (CPC):
**A61K 9/51; A61K 31/575; A61P 25/28; A61P 35/00**

(86) International application number:
**PCT/KR2024/006052**

(87) International publication number:
**WO 2024/228596 (07.11.2024 Gazette 2024/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.05.2023 KR 20230058677**

(71) Applicant: **Mepsgen Co., Ltd.**
**Seoul 05836 (KR)**

(72) Inventors:
- **LEE, Su Ji**
**Namyangju-si Gyeonggi-do 12275 (KR)**
- **KIM, Jin Young**
**Seongnam-si Gyeonggi-do 13313 (KR)**
- **KIM, Yong Tae**
**Seoul 05855 (KR)**

(74) Representative: **Mullholland, Lewis Paul**
**WP Thompson**
**44 Southampton Buildings**
**London WC2A 1AP (GB)**

(54) **RECONSTITUTED HIGH-DENSITY LIPOPROTEIN NANOPARTICLES COMPRISING CHOLESTEROL**

(57) The present invention relates to: reconstituted high-density lipoprotein nanoparticles comprising cholesterol; and a composition for preventing or treating Alzheimer's disease and cancer, the composition comprising the nanoparticles. Specifically, the reconstituted high-density lipoprotein nanoparticles comprising cholesterol according to the present invention have an excellent cell influx rate and promote cholesterol efflux from cells, thus having a cancer cell killing effect, and can therefore be used for preventing or treating cancer.

**Figure 1**

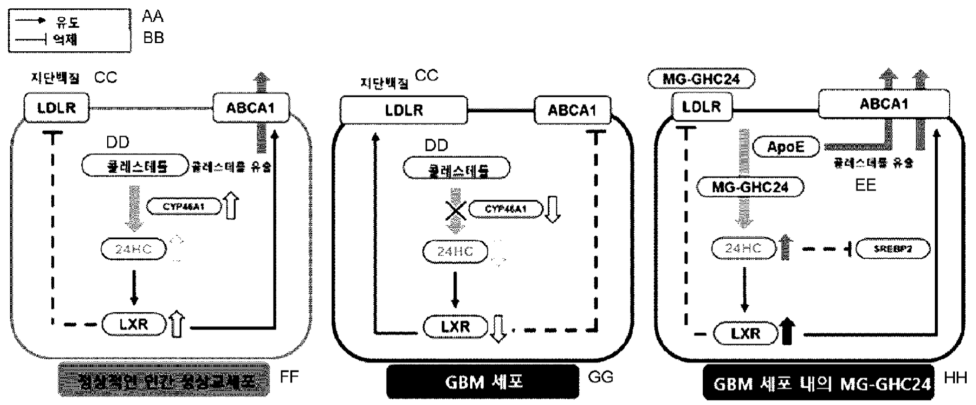


AA ... Induction
BB ... Inhibition
CC ... Lipoprotein
DD ... Cholesterol
EE ... Cholesterol efflux
FF ... Normal human astrocytes
GG ... GBM cells
HH ... MG-GHC24 in GBM cells

EP 4 706 644 A1

## Description

## Technical Field

The present invention relates to: reconstituted high-density lipoprotein (rHDL) nanoparticles comprising cholesterol; and a composition for preventing and treating Alzheimer's disease or cancer, the composition comprising the nanoparticles.

Specifically, the present invention relates to rHDL nanoparticles comprising a cholesterol, a phospholipid, and an apolipoprotein and the rHDL nanoparticles according to the present invention promote cholesterol efflux from cells, thus having a cancer cell killing effect.

## Background Art

According to global cancer statistics, 19.3 million new cancer cases and 10 million cancer-related deaths occurred in 2020. Research on the treatment of cancer, which ranks as the leading cause of death in modern society, has been regarded as essential, and representative cancer treatments modalities include surgical treatment, biotherapy, radiotherapy, chemotherapy through the administration of anticancer agents, and targeted therapy. However, due to side effects that damage normal organ and adversely affect the quality of life, continuous efforts have been made to develop more effective anticancer therapies. Among these, glioblastoma (GBM) is the most common and aggressive primary brain tumor and represents one of the most lethal forms of cancer. The current standard of care (SOC) for GBM is the Stupp protocol, which comprises localized radiotherapy, surgical resection, and temozolomide. However, complete surgical resection is difficult due to the delicate anatomy of the brain and the highly invasive nature of GBM. In addition, the limited blood-brain barrier (BBB) permeability of existing chemotherapeutic agents, such as temozolomide, restricts effective treatment. Despite recent advances in surgery, chemotherapy, and immunotherapy, the median survival time for patients diagnosed with GBM remains less than two years. The development of alternative and targeted delivery approaches for the effective treatment of GBM represents an urgent challenge in GBM therapy. Moreover, understanding the biology of GBM is essential for identifying novel treatments strategies.

Pancreatic cancer is another malignant tumor that presents a challenging therapeutic approach. The poor prognosis, with a median 5-year survival rate of approximately 5-10%, is attributed to difficulties in early diagnosis, treatment intolerance, and a hostile tumor microenvironment that promotes the rapid development of resistance mechanisms. Transcriptome analyses have revealed that pancreatic ductal adenocarcinoma (PDAC) exhibits enhanced lipid metabolism pathways compared with normal cells, and in particular, cholesterol uptake processes, including the expression of the low-density lipoprotein receptor (LDLR), are highly activated (Raimund O, Life Sci Alliance, 2022; Fabienne G, PNAS, 2015).

Cholesterol is a crucial nutrient for normal cellular function and survival. It plays a a key role in the plasma membrane and lipid rafts, and serves as a precursor to steroid hormones, bile acids, and vitamin D. Because both excess and deficiency of intracellular cholesterol can be toxic, cells regulate intracellular cholesterol homeostasis through cholesterol uptake, efflux, and endogenous synthesis. In addition, cells control cholesterol biosynthesis and elimination to maintain consistent and appropriate levels of cholesterol for homeostasis regulation. Cholesterol metabolism is regulated by the coordinated action of the sterol regulatory element-binding protein (SREBP) and the liver X receptor (LXR) transcription factors. SREBP enhances cholesterol uptake by inducing cholesterol biosynthetic genes and the low-density lipoprotein receptor (LDLR). LXR activates cholesterol efflux genes (ABCA1, ABCG1) to promote cholesterol efflux and downregulates cholesterol uptake by inducing the synthesis of IDOL (LDLR-inducible degrader), an E3 ubiquitin ligase that mediates LDLR degradation.

However, a common feature of various cancers is dysregulation of cholesterol homeostasis. Abnormally increased cholesterol levels in many cancers, including liver cancer, gastric cancer, colorectal cancer, pancreatic cancer, and prostate cancer, influence cancer cell proliferation, metastasis, and invasion. In particular, GBM depends on exogenous cholesterol uptake through upregulation of LDLR, while reduced cholesterol metabolism and its metabolites suppress cholesterol efflux, resulting in excessive intracellular cholesterol accumulation (Leila Pirmoradi, J. Investig. Med., 2019). Excessive intracellular cholesterol can be eliminated by conversion to oxysterols or 24S-hydroxycholesterol (24HC) via the brain-specific protein cholesterol 24-hydroxylase (CYP46A1). However, GBM exhibits lower expression levels of CYP46A1 compared with normal human astrocytes, thereby showing a reduced capacity to convert cholesterol to 24HC (Mingzhi Han, EMBO Mol. Med., 2020).

Several endogenous oxysterols, including 22HC, 25HC, 27HC, and 24HC, act as LXR agonists that strongly induce LXR transcription. Among them, 24HC reduces intracellular cholesterol levels through upregulation of ABCA1 in GBM, thereby inhibiting cell growth and ultimately inducing cell death (Sigrid Nachterhaele, Nat. chem. biol., 2012). Although novel cancer treatment approaches targeting pathways associated with cholesterol homeostasis are being investigated, the limited permeability of the blood-brain barrier (BBB) renders targeted delivery of LXR agonists to GBM

difficult. Accordingly, there is a need to develop a delivery system capable of targeting GBM and effectively crossing the BBB to deliver sufficient therapeutic agents.

While increased ABCA1 expression enhances intracellular cholesterol efflux and contribute to cancer cell death, increased ABCA1 expression has also been linked to improvement in Alzheimer's disease (AD). In the brain, ABCA1 mediates the lipidation of apolipoprotein E, promotes the clearance of amyloid-β (Aβ), and participates in BBB maintenance via an apolipoprotein E-mediated pathway. Increased ABCA1 expression can positively affect apolipoprotein E lipidation, insulin sensitivity, peripheral vascular and BBB integrity, and anti-inflammatory signaling. Conversely, reduced ABCA1 expression is associated with decreased apolipoprotein E levels in cerebrospinal fluid and plasma, increased brain amyloid burden, and decreased apolipoprotein E levels are associated with BBB disruption (Radosveta Koldamova, J Biol Chem., 2005).Accordingly, formulations that increase ABCA1 levels may provide positive effects not only in anticancer treatment but also in Alzheimer's disease.

It has been reported that treatment of PDAC cells with the LXR agonist T0901317 increased the expression of the ABCA1 protein, which promotes cholesterol efflux and induces cancer cell death (Raimund O, Life Sci Alliance, 2022). Accordingly, inducing cancer cell death by delivering LXR agonists intracellularly through overexpressed LDLR could be a promising approach for pancreatic cancer therapy.

High-density lipoprotein (HDL) is a natural nanoparticle composed of apolipoprotein and lipid components that transport cholesterol. Based on studies demonstrating that HDL and reconstituted high-density lipoprotein (rHDL) can deliver drugs across the blood-brain barrier (BBB), we developed ApoE3-based rHDL nanoparticles containing 24HC, which possesses LDLR binding capacity.

The present inventors have confirmed that rHDL nanoparticles incorporating apolipoprotein E3 and comprising 24HC exhibit remarkable therapeutic effects on tumors through cholesterol regulation. Accordingly, the present inventors have completed the present invention.

## Prior Art Document

### Non-Patent Document

(Non-Patent Document 0001) Leila Pirmoradi, Nayer Seyfizadeh, Saeid Ghavami, Amir A Zeki, Shahla Shojaei, Targeting cholesterol metabolism in glioblastoma: a new therapeutic approach in cancer therapy, J. Investig. Med. 2019 67(4):715-719.

(Non-Patent Document 0002) Mingzhi Han, Shuai Wang, Ning Yang, Xu Wang, Wenbo Zhao, Halala Sdik Saed, Thomas Daubon, Bin Huang, Anjing Chen, Gang Li, Hrvoje Miletic, Frits Thorsen, Rolf Bjerkvig, Xingang Li, Jian Wang, Therapeutic implications of altered cholesterol homeostasis mediated by loss of CYP46A1 in human glioblastoma, EMBO Mol. Med. 2020 Jan 9;12(1):e10924.

(Non-Patent Document 0003) Sigrid Nachtergaele, Laurel K Mydock, Kathiresan Krishnan, Jayan Rammohan, Paul H Schlesinger, Douglas F Covey, Rajat Rohatgi, Oxysterols are allosteric activators of the oncoprotein smoothened, Nat. Chem. Biol. 2012 Jan 8;8(2):211-220.

(Non-Patent Document 0004) Radosveta Koldamova, Matthias Staufenbiel, Iliya Lefterov, Lack of ABCA1 Considerably Decreases Brain ApoE Level and Increases Amyloid Deposition in APP23 Mice, J Biol Chem., 2005 Dec 30;280(52):43224-43235.

(Non-Patent Document 0005) Raimund Oberle, Kristina Kuhrer, et al. The HDL particle composition determines its antitumor activity in pancreatic cancer, Life Sci Alliance. 2022 May 16;5(9):e202101317.

(Non-Patent Document 0006) Fabienne Guillaumond, Ghislain Bidaut, et al. Cholesterol uptake disruption, in association with chemotherapy, is a promising combined metabolic therapy for pancreatic adenocarcinoma, PNAS. 2015 Feb 124;112(8):2473-8.


## Detailed Description of Invention

### Technical Problem

An object of the present invention is to provide a reconstituted high-density lipoprotein nanoparticle comprising cholesterol.

Another object of the present invention is to provide a composition for preventing or treating Alzheimer's disease or cancer, comprising reconstituted high-density lipoprotein nanoparticles comprising cholesterol.

Another object of the present invention is to provide a method for producing reconstituted high-density lipoprotein nanoparticles comprising cholesterol.

**Solution to Problem**

The present invention provides a reconstituted high-density lipoprotein (rHDL) nanoparticle comprising a phospholipid, a cholesterol, and apolipoprotein E.

The cholesterol may be at least one selected from the group consisting of cholesterol, 7-ketocholesterol, 7α-hydroxycholesterol, 7β-hydroxycholesterol, 4β-hydroxycholesterol, α-5,6-epoxycholesterol, β-5,6-epoxycholesterol, 24S, 25-epoxycholesterol, 25-epoxycholesterol, 7-hydroxycholesterol, 20(S)-hydroxycholesterol, 22(S)-hydroxycholesterol, 22(R)-hydroxycholesterol, 24(S)-hydroxycholesterol, 24(R)-hydroxycholesterol, 25-hydroxycholesterol, 27-hydroxycholesterol, 5β-epoxycholesterol, 6β-epoxycholesterol, 7-dehydrocholesterol, (25R)26-hydroxycholesterol, 7α,(25R)26-dihydroxycholesterol, 5β-cholestane-3α,7α,12α,(25R)26-tetrol, 7α,25-dihydroxycholesterol, 7α,24S-dihydroxycholesterol, 3β,5α,6β-cholestane-3,5,6,26-tetrol, lanosterol, desmosterol, 7α-hydroxydesmosterol, (24Z),26S-hydroxydesmosterol, (24E),26S-hydroxydesmosterol, 24S,25-epoxylanosterol, 26(Z)-hydroxydesmosterol, 25-hydroxy-8-dehydrocholesterol, 24-hydroxy-8-dehydrocholesterol, 7-dehydrocholesterol, 8-dehydrocholesterol, (25R)26-hydroxy-8-dehydrocholesterol, 25-hydroxy-7-dehydrocholesterol, 4α-hydroxy-7-dehydrocholesterol, 4β-hydroxy-7-dehydrocholesterol, 7,8-epoxycholesterol, 3α,5β,6α-cholestane-3,6-diol, cholestane-3β, 5α, 6β-triol, Oxy34, Oxy49, and Oxy133, but is not limited thereto.

In one embodiment, the cholesterol may be 24-hydroxycholesterol (24HC).

In one embodiment, the nanoparticle according to the present invention may further comprise a liver X receptor (LXR) agonist.

In one embodiment, the LXR agonist may be at least one selected from the group consisting of T0901317, GW3965, GW6340, DMHCA, LXR-623, WYE-672, and AZ876, but is not limited thereto.

When synthesizing the reconstituted high-density lipoprotein (rHDL) nanoparticles, the synthetic mixing weight ratio of apolipoprotein E and cholesterol may be 1:2 to 1:4, preferably 1:3.

In one embodiment, the apolipoprotein E may be apolipoprotein E2, E3, or E2 and E3.

In one embodiment, the reconstituted high-density lipoprotein (rHDL) nanoparticle may further comprise apolipoprotein A1.

In one embodiment, the phospholipid may be at least one selected from the group consisting of 1,2-dioleoyl-sn-glycero-3-phosphatidylcholine (DOPC), egg phosphatidylcholine (EPC), dilauroylphosphatidylcholine (DLPC), 1,2-dimyristoyl-sn-glycero-3-phosphocholine (DMPC), dipalmitoylphosphatidylcholine (DPPC), distearoylphosphatidylcholine (DSPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoyl phosphatidylcholine (SPPC), 1,2-diarachidoyl-sn-glycero-3-phosphocholine (DAPC), 1,2-dibehenoyl-sn-glycero-3-phosphocholine (DBPC), 1,2-dierucoyl-sn-glycero-3-phosphocholine (DEPC), palmitoyloleoylphosphatidylcholine (POPC), lysophosphatidylcholine, dilinoleoylphosphatidylcholine, distearoylphosphatidylethanolamine (DSPE), dimyristoylphosphatidylethanolamine (DMPE), dipalmitoylphosphatidylethanolamine (DPPE), palmitoyloleoylphosphatidylethanolamine (POPE), lysophosphatidylethanolamine, N1-[2-((1S)-1-[(3-aminopropyl)amino]-4-[di(3-aminopropyl)amino]butylcarboxamido)ethyl]-3,4-di[oleoyloxy]-benzamide) , dioctadecylamidoglycylspermine 4-trifluoroacetic acid (DOGS), 3β-[N-(N',N'-dimethylaminoethane)-carbamoyl]cholesterol (DC-Chol), 1,2-di-O-octadecenyl-3-trimethylammonium propane (DOTMA), 1,2-dioleoyl-3-trimethylammonium-propane (DOTAP), (1,2-dioleoyloxypropyl)-3-dimethylhydroxyethyl ammonium bromide (DORIE), 1,2-dimyristyloxy-propyl-3-dimethyl-hydroxyethyl ammonium bromide (DMRIE), 2,3-dioleoyloxy-N-[2-(sperminecarboxamido)ethyl]-N,N-dimethyl-1-propanaminium trifluoroacetate (DOSPA), N-(3-aminopropyl)-N,N-dimethyl-2,3-bis(dodecyloxy)-1-propaneammonium bromide (GAP-DLRIE), N-t-butyl-N'-tetradecyl-3-tetradecylaminopropionamidine (diC14-amidine), ethylphosphocholine (Ethyl PC), dimethyldioctadecylammonium bromide (DDAB), N4-cholesteryl-spermine (GL67), 1,2-dioleyloxy-3-dimethylaminopropane (DODMA), D-Lin-MC3-DMA (MC3, DLin-MC3-DMA), DLin-KC2-DMA, and DLin-DMA, but is not limited thereto.

In one embodiment, the reconstituted high-density lipoprotein (rHDL) nanoparticle may have a diameter of 25 nm or less.

In one embodiment, the reconstituted high-density lipoprotein (rHDL) nanoparticle may increase the expression of ABCA1.

In one embodiment, the reconstituted high-density lipoprotein (rHDL) nanoparticle may increase the cholesterol efflux.

In one embodiment, the reconstituted high-density lipoprotein (rHDL) nanoparticle may kill cancer cells.

In one embodiment, the reconstituted high-density lipoprotein (rHDL) nanoparticle may be influxed into cells via LDLR.

The present invention provides a composition for preventing or treating Alzheimer's disease or cancer, comprising the reconstituted high-density lipoprotein (rHDL) nanoparticles.

The present invention provides a use of a composition for preventing or treating Alzheimer's disease or cancer, comprising the reconstituted high-density lipoprotein (rHDL) nanoparticles.

The present invention provides a method for treating Alzheimer's disease or cancer, comprising administering the reconstituted high-density lipoprotein (rHDL) nanoparticles to a subject.

The cancer may be at least one selected from the group consisting of carcinoma, sarcoma, lymphoma, leukemia, germ cell tumor, brain tumor corresponding to blastoma, cervical cancer, ovarian cancer, prostate cancer, lung cancer, bile duct cancer, kidney cancer, gastric cancer, liver cancer, retinoblastoma, choriocarcinoma, small intestine cancer, colorectal cancer and rectal cancer, non-small cell lung cancer, gastric adenocarcinoma, acute lymphocytic leukemia, acute myeloid leukemia, breast cancer, bone sarcoma, bladder cancer, anaplastic astrocytoma, head and neck cancer, pancreatic cancer, and esophageal cancer, but is not limited thereto. Preferably, the cancer may be brain tumor.

The present invention provides a method for producing cholesterol-containing rHDL nanoparticles, wherein the method comprises: a first step of injecting a phospholipid solution into a second inlet located at the center of a microfluidic device comprising three inlets and one outlet; a second step of injecting an apolipoprotein solution into a first and a third inlets located on either side to produce rHDL nanoparticles comprising phospholipid and apolipoprotein E; and a third step of adding cholesterol dropwise onto the rHDL nanoparticles.

In one embodiment, the microfluidic device may comprise a micropillar.

In one embodiment, the production method may further comprise a step of removing an organic solvent after the second step.

In one embodiment, the cholesterol of the production method may be at least one selected from the group consisting of cholesterol, 7-ketocholesterol, 7α-hydroxycholesterol, 7β-hydroxycholesterol, 4β-hydroxycholesterol, α-5,6-epoxycholesterol, β-5,6-epoxycholesterol, 24S, 25-epoxycholesterol, 25-epoxycholesterol, 7-hydroxycholesterol, 20(S)-hydroxycholesterol, 22(S)-hydroxycholesterol, 22(R)-hydroxycholesterol, 24(S)-hydroxycholesterol, 24(R)-hydroxycholesterol, 25-hydroxycholesterol, 27-hydroxycholesterol, 5β-epoxycholesterol, 6β-epoxycholesterol, 7-dehydrocholesterol, (25R)26-hydroxycholesterol, 7α,(25R)26-dihydroxycholesterol, 5β-cholestane-3α,7α,12α,(25R)26-tetrol, 7α,25-dihydroxycholesterol, 7α,24S-dihydroxycholesterol, 3β,5α,6β-cholestane-3,5,6,26-tetrol, lanosterol, desmosterol, 7α-hydroxydesmosterol, (24Z),26S-hydroxydesmosterol, (24E),26S-hydroxydesmosterol, 24S,25-epoxylanosterol, 26(Z)-hydroxydesmosterol, 25-hydroxy-8-dehydrocholesterol, 24-hydroxy-8-dehydrocholesterol, 7-dehydrocholesterol, 8-dehydrocholesterol, (25R)26-hydroxy-8-dehydrocholesterol, 25-hydroxy-7-dehydrocholesterol, 4α-hydroxy-7-dehydrocholesterol, 4β-hydroxy-7-dehydrocholesterol, 7,8-epoxycholesterol, 3α,5β,6α-cholestane-3,6-diol, cholestane-3β, 5α, 6β-triol, Oxy34, Oxy49, and Oxy133, but is not limited thereto.

In one embodiment, the cholesterol may be 24-hydroxycholesterol (24HC).

In one embodiment, the apolipoprotein may be apolipoprotein E.

In one embodiment, the apolipoprotein E may be apolipoprotein E2, E3, or E2 and E3.

In one embodiment, the phospholipid may be at least one selected from the group consisting of 1,2-dioleoyl-sn-glycero-3-phosphatidylcholine (DOPC), egg phosphatidylcholine (EPC), dilauroylphosphatidylcholine (DLPC), 1,2-dimyristoyl-sn-glycero-3-phosphocholine (DMPC), dipalmitoylphosphatidylcholine (DPPC), distearoylphosphatidylcholine (DSPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoyl phosphatidylcholine (SPPC), 1,2-diarachidoyl-sn-glycero-3-phosphocholine (DAPC), 1,2-dibehenoyl-sn-glycero-3-phosphocholine (DBPC), 1,2-dierucoyl-sn-glycero-3-phosphocholine (DEPC), palmitoyloleoylphosphatidylcholine (POPC), lysophosphatidylcholine, dilinoleoylphosphatidylcholine, distearoylphosphatidylethanolamine (DSPE), dimyristoylphosphatidylethanolamine (DMPE), dipalmitoylphosphatidylethanolamine (DPPE), palmitoyloleoylphosphatidylethanolamine (POPE), lysophosphatidylethanolamine, N1-[2-((1S)-1-[(3-aminopropyl)amino]-4-[di(3-aminopropyl)amino]butylcarboxamido)ethyl]-3,4-di[oleoyloxy]-benzamide) , dioctadecylamidoglycylspermine 4-trifluoroacetic acid (DOGS), 3β-[N-(N',N'-dimethylaminoethane)-carbamoyl]cholesterol (DC-Chol), 1,2-di-O-octadecenyl-3-trimethylammonium propane (DOTMA), 1,2-dioleoyl-3-trimethylammonium-propane (DOTAP), (1,2-dioleoyloxypropyl)-3-dimethylhydroxyethyl ammonium bromide (DORIE), 1,2-dimyristyloxy-propyl-3-dimethyl-hydroxyethyl ammonium bromide (DMRIE), 2,3-dioleyloxy-N-[2-(sperminecarboxamido) ethyl]-N,N-dimethyl-1-propanaminium trifluoroacetate (DOSPA), N-(3-aminopropyl)-N,N-dimethyl-2,3-bis(dodecyloxy)-1-propaneammonium bromide (GAP-DLRIE), N-t-butyl-N'-tetradecyl-3-tetradecylaminopropionamidine (diC14-amidine), ethylphosphocholine (Ethyl PC), dimethyldioctadecylammonium bromide (DDAB), N4-cholesteryl-spermine (GL67), 1,2-dioleyloxy-3-dimethylaminopropane (DODMA), D-Lin-MC3-DMA (MC3, DLin-MC3-DMA), DLin-KC2-DMA, and DLin-DMA, but is not limited thereto.

## Effects of Invention

The cholesterol-containing rHDL nanoparticles according to the present invention have treatment and prevention effects against cancer. In particular, the nanoparticles can be applied to cancers in which LDLR is overexpressed, including pancreatic cancer, head and neck cancer, esophageal cancer, gastric cancer, and brain tumor and are preferably applied to brain tumors. In addition, the nanoparticles are capable of crossing the BBB and may be used for the treatment and prevention of Alzheimer's disease.

The cholesterol-containing rHDL nanoparticles according to the present invention target LDLR in glioblastoma and have the effect of inducing cholesterol efflux within glioblastoma.

The cholesterol-containing rHDL nanoparticles according to the present invention induce cholesterol efflux in glioblastoma, thereby inducing cell death in glioblastoma cells.

The cholesterol-containing rHDL nanoparticles according to the present invention are non-toxic to BBB constituent cells and are capable of crossing the BBB, thereby enabling the treatment of glioblastoma.

Therefore, the cholesterol-containing rHDL nanoparticles according to the present invention can be used to treat brain tumors and Alzheimer's disease.

In addition, since the rHDL nanoparticles according to the present invention can be produced using a microfluidic device, mass production of nanoparticles with consistent properties (no batch-to-batch variability) is possible. Compared to conventional rHDL, the rHDL nanoparticles do not comprise additional components such as surfactants, so the rHDL nanoparticles exhibit no in vivo toxicity and demonstrate excellent safety.

**Brief Description of Drawings**

Figure 1 is a schematic diagram illustrating cholesterol metabolism in normal brain cells, cholesterol metabolism in GBM cells, and the mechanism of GBM death induced by 24HC-containing rHDL nanoparticles.

Figure 2 shows a comparison CYP46A1 RNA levels in normal and GBM brain samples based on TCGA (The Cancer Genome Atlas) and GTEx (Genotype-tissue expression).

Figure 3 shows a comparison LDLR RNA levels in normal and GBM brain samples based on TCGA and GTEx.

Figure 4 shows 24HC levels in astrocytes and GBM.

Figure 5 shows cholesterol levels in astrocytes and GBM.

Figure 6 is a schematic diagram of the microfluidic device used for producing rHDL nanoparticles and the corresponding production method.

Figure 7 shows the size distribution of rHDL nanoparticles by volume as measured by DLS.

Figure 8 shows the size distribution of nanoparticles by volume according to the synthetic mixing molar ratio of apolipoprotein E3:24HC when producing 24HC-containing rHDL nanoparticles as measured by DLS.

Figure 9 shows the size distribution of nanoparticles by intensity according to the synthetic mixing molar ratio of apolipoprotein E3:24HC when producing 24HC-containing rHDL nanoparticles as measured by DLS.

Figure 10 shows the size distribution of nanoparticles by number according to the synthetic mixing molar ratio of apolipoprotein E3:24HC when producing 24HC-containing rHDL nanoparticles as measured by DLS.

Figure 11 shows the amount of 24HC encapsulated in 24HC-containing rHDL nanoparticles according to the synthetic mixing molar ratio of apolipoprotein E3:24HC.

Figure 12 shows the size distribution of 24HC-containing rHDL nanoparticles produced at a synthetic mixing molar ratio of 1:3 apolipoprotein E3:24HC by volume as measured by DLS.

Figure 13 shows the compositional ratio of apolipoprotein E3 and phospholipid in the rHDL nanoparticles.

Figure 14 shows the compositional ratio of apolipoprotein E3, phospholipid, and 24HC in 24HC-containing rHDL nanoparticles.

Figure 15 shows particle size changes over 24 hours to measure the stability of rHDL nanoparticles and 24HC-containing rHDL nanoparticles under serum conditions.

Figure 16 shows particle size changes over 24 hours to measure the stability of rHDL nanoparticles and 24HC-containing rHDL nanoparticles under GBM tissue pH conditions.

Figure 17 shows particle size changes over 24 hours to measure the stability of rHDL nanoparticles and 24HC-containing rHDL nanoparticles under body fluid pH conditions.

Figure 18 shows particle size changes over 60 days to measure the stability of rHDL nanoparticles and 24HC-containing rHDL nanoparticles under four conditions.

Figure 19 shows the PDI stability of rHDL nanoparticles and 24HC-containing rHDL nanoparticles over 60 days at 4 °C.

Figure 20 is a photograph showing the cellular uptake of rHDL nanoparticles and 24HC-containing rHDL nanoparticles in hBMEC cells using CLSM.

Figure 21 shows the quantification of CLSM data on the cellular uptake of rHDL nanoparticles and 24HC-containing rHDL nanoparticles in hBMEC cells using ImageJ.

Figure 22 is a photograph showing the cellular uptake of rHDL nanoparticles and 24HC-containing rHDL nanoparticles in iBMEC cells using CLSM.

Figure 23 shows the quantificationof CLSM data on the cellular uptake of rHDL nanoparticles and 24HC-containing rHDL nanoparticles in iBMEC cells using ImageJ.

Figure 24 shows the cell permeability in hBMEC cells when treated with rHDL nanoparticles and 24HC-containing rHDL nanoparticles.

Figure 25 shows the cytotoxicity of hBMEC cells when treated with rHDL nanoparticles, 24HC, and 24HC-containing rHDL nanoparticles.

Figure 26 shows the cytotoxicity of iBMEC cells when treated with rHDL nanoparticles, 24HC, and 24HC-containing rHDL nanoparticles.

Figure 27 shows the cytotoxicity of pericyte when treated with rHDL nanoparticles, 24HC, and 24HC-containing rHDL nanoparticles.

Figure 28 shows the cytotoxicity of astrocyte when treated with rHDL nanoparticles, 24HC, and 24HC-containing rHDL nanoparticles.

Figure 29 is a photograph showing the LDLR-mediated cellular uptake of rHDL nanoparticles and 24HC-containing rHDL nanoparticles in U87MG cells using CLSM.

Figure 30 shows the quantification of CLSM data on the LDLR-mediated cellular uptake of rHDL nanoparticles and 24HC-containing rHDL nanoparticles in U87MG cells using ImageJ.

Figure 31 is a photograph showing the LDLR-mediated cellular uptake of rHDL nanoparticles and 24HC-containing rHDL nanoparticles in LN229 cells using CLSM.

Figure 32 shows the quantification of CLSM data on the LDLR-mediated cellular uptake of rHDL nanoparticles and 24HC-containing rHDL nanoparticles in LN229 cells using ImageJ.

Figure 33 shows the changes in 24HC levels in U87MG and LN229 cells when treated with 24HC and 24HC-containing rHDL nanoparticles, relative to the 24HC reference level.

Figure 34 shows the changes in LDLR mRNA expression in U87MG and LN229 cells when treated with rHDL nanoparticles, 24HC, and 24HC-containing rHDL nanoparticles.

Figure 35 shows the changes in ABCA1 mRNA expression in U87MG and LN229 cells when treated with rHDL nanoparticles, 24HC, and 24HC-containing rHDL nanoparticles.

Figure 36 shows the changes in cholesterol efflux in U87MG and LN229 cells when treated with rHDL nanoparticles, 24HC, and 24HC-containing rHDL nanoparticles.

Figure 37 shows the changes in cholesterol efflux in U87MG and LN229 cells when treated with rHDL nanoparticles and 24HC-containing rHDL nanoparticles, in the presence or absence of an ABCA1 inhibitor.

Figure 38 shows the killing efficacy of rHDL nanoparticles, 24HC, and 24HC-containing rHDL nanoparticles in U87MG cells.

Figure 39 shows the killing efficacy of rHDL nanoparticles, 24HC, and 24HC-containing rHDL nanoparticles in LN229 cells.

Figure 40 is a photograph showing the cellular uptake of rHDL nanoparticles and 24HC-containing rHDL nanoparticles in GBM#P1 and GBM#P2 cells using CLSM.

Figure 41 shows the quantification of CLSM data on the cellular uptake of rHDL nanoparticles and 24HC-containing rHDL nanoparticles in GBM#P1 cells using ImageJ.

Figure 42 shows the quantification of CLSM data on the cellular uptake of rHDL nanoparticles and 24HC-containing rHDL nanoparticles in GBM#P2 cells using ImageJ.

Figure 43 shows the changes in LDLR mRNA expression in GBM#P1 and GBM#P2 cells when treated with rHDL nanoparticles, 24HC, and 24HC-containing rHDL nanoparticles.

Figure 44 shows the changes in ABCA1 mRNA expression in GBM#P1 and GBM#P2 cells when treated with rHDL nanoparticles, 24HC, and 24HC-containing rHDL nanoparticles.

Figure 45 shows the changes in cholesterol efflux in GBM#P1 and GBM#P2 cells when treated with rHDL nanoparticles, 24HC, and 24HC-containing rHDL nanoparticles.

Figure 46 shows the changes in cholesterol efflux in GBM#P1 and GBM#P2 cells when treated with rHDL nanoparticles and 24HC-containing rHDL nanoparticles, in the presence or absence of an ABCA1 inhibitor.

Figure 47 shows the killing efficacy of rHDL nanoparticles, 24HC, and 24HC-containing rHDL nanoparticles in GBM#P1 cells.

Figure 48 shows the killing efficacy of rHDL nanoparticles, 24HC, and 24HC-containing rHDL nanoparticles in GBM#P2 cells.

## Best Mode for Carrying out the Invention

As used herein, the term "reconstituted high-density lipoprotein (rHDL)" refers to artificially produced HDL-mimetic particles that is designed to mimic the biological effects of naturally occurring high-density lipoprotein (HDL).

As used herein, the term "24-hydroxycholesterol" is a type of oxysterol and refers to an oxidized derivative of cholesterol which is generated through endogenous cholesterol metabolism in tissues and participates in the regulation of cholesterol metabolism. The term "24-hydroxtcholesterol" may also be referred to as "24HC".

As used herein, the term "phospholipid" may be specifically at least one selected from the group consisting of 1,2-dioleoyl-sn-glycero-3-phosphatidylcholine (DOPC), egg phosphatidylcholine (EPC), dilauroylphosphatidylcholine (DLPC), 1,2-dimyristoyl-sn-glycero-3-phosphocholine (DMPC), dipalmitoylphosphatidylcholine (DPPC), distearoylphosphatidylcholine (DSPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoyl phosphatidylcholine (SPPC), 1,2-diarachidoyl-sn-glycero-3-phosphocholine (DAPC), 1,2-dibehenoyl-sn-glycero-3-phosphocholine (DBPC), 1,2-dierucoyl-sn-glycero-3-phosphocholine (DEPC), palmitoyloleoylphosphatidylcholine (POPC), lysophosphatidylcholine, dilinoleoylphosphatidylcholine, distearoylphosphatidylethanolamine (DSPE), dimyristoylphosphatidylethanolamine (DMPE), dipalmitoylphosphatidylethanolamine (DPPE), palmitoyloleoylphosphatidylethanolamine (POPE), lysophosphatidylethanolamine, N1-[2-((1S)-1-[(3-aminopropyl)amino]-4-[di(3-aminopropyl)amino]butylcarboxamido)ethyl]-3,4-di[oleoyloxy]-benzamide) , dioctadecylamidoglycylspermine 4-trifluoroacetic acid (DOGS), 3β-[N-(N',N'-dimethylaminoethane)-carbamoyl]cholesterol (DC-Chol), 1,2-di-O-octadecenyl-3-trimethylammonium propane (DOTMA), 1,2-dioleoyl-3-trimethylammonium-propane (DOTAP), (1,2-dioleoyloxypropyl)-3-dimethylhydroxyethyl ammonium bromide (DORIE), 1,2-dimyristyloxy-propyl-3-dimethyl-hydroxyethyl ammonium bromide (DMRIE), 2,3-dioleoyloxy-N-[2-(sperminecarboxamido)ethyl]-N,N-dimethyl-1-propanaminium trifluoroacetate (DOSPA), N-(3-aminopropyl)-N,N-dimethyl-2,3-bis(dodecyloxy)-1-propaneammonium bromide (GAP-DLRIE), N-t-butyl-N'-tetradecyl-3-tetradecylaminopropionamidine (diC14-amidine), ethylphosphocholine (Ethyl PC), dimethyldioctadecylammonium bromide (DDAB), N4-cholesteryl-spermine (GL67), 1,2-dioleyloxy-3-dimethylaminopropane (DODMA), or D-Lin-MC3-DMA (MC3, DLin-MC3-DMA), DLin-KC2-DMA, DLin-DMA, but is not limited thereto.

As used herein, the term "cholesterol" is a type of sterol, a steroid alcohol, and refers to a lipid component that constitutes cell membranes. Preferably, it may be at least one selected from the group consisting of cholesterol, 7-ketocholesterol, 7α-hydroxycholesterol, 7β-hydroxycholesterol, 4βhydroxycholesterol (4β-hydroxycholesterol), α-5,6-epoxycholesterol, β-5,6-epoxycholesterol, 24S, 25-epoxycholesterol, 25-epoxycholesterol, 7-hydroxycholesterol, 20(S)-hydroxycholesterol, 22(S)-hydroxycholesterol, 22(R)-hydroxycholesterol, 24(S)-hydroxycholesterol, 24(R)-hydroxycholesterol, 25-hydroxycholesterol, 27-hydroxycholesterol, 5β-epoxycholesterol, 6β-epoxycholesterol, 7-dehydrocholesterol, (25R)26-hydroxycholesterol, 7α,(25R)26-dihydroxycholesterol, 5β-cholestane-3α,7α,12α,(25R)26-tetrol, 7α,25-dihydroxycholesterol, 7α,24S-dihydroxycholesterol, 3β,5α,6β-cholestane-3,5,6,26-tetrol, lanosterol, desmosterol, 7α-hydroxydesmosterol, (24Z),26S-hydroxydesmosterol, (24E),26S-hydroxydesmosterol, 24S,25-epoxylanosterol, 26(Z)-hydroxydesmosterol, 25-hydroxy-8-dehydrocholesterol, 24-hydroxy-8-dehydrocholesterol, 7-dehydrocholesterol, 8-dehydrocholesterol, (25R)26-hydroxy-8-dehydrocholesterol, 25-hydroxy-7-dehydrocholesterol, 4α-hydroxy-7-dehydrocholesterol, 4β-hydroxy-7-dehydrocholesterol, 7,8-epoxycholesterol, 3α,5β,6α-cholestane-3,6-diol, cholestane-3β, 5α, 6β-triol, Oxy34, Oxy49, and Oxy133, but is not limited thereto.

As used herein, the term "LXR agonist" refers to a substance that promotes transcription of the liver X receptor (LXR). An LXR agonist promotes LXR transcription and increases its expression, thereby activating cholesterol efflux genes (ABCA1, ABCG1) to induce excessive cholesterol efflux and downregulate cholesterol uptake. Preferably, the LXR agonist may be at least one selected from the group consisting of T0901317, GW3965, GW6340, DMHCA, LXR-623, WYE-672, and AZ876, but is not limited thereto.

The above T0901317 is an LXR agonist and has Formula 1 below:

[Formula 1]

$C_{17}H_{12}NSO_3F_9$ (T0901317)

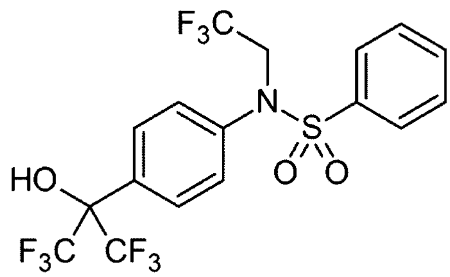

The above GW3965 is an LXR agonist and has Formula 2 below.

[Formula 2]

$C_{33}H_{31}F_3ClNO_3$ (GW3965)

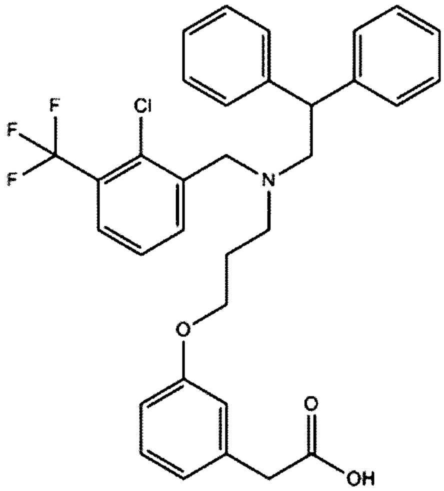

The above GW6340 is an LXR agonist and has Formula 3 below.

[Formula 3]

$C_{33}H_{32}ClF_3N_2O$ (GW6340)

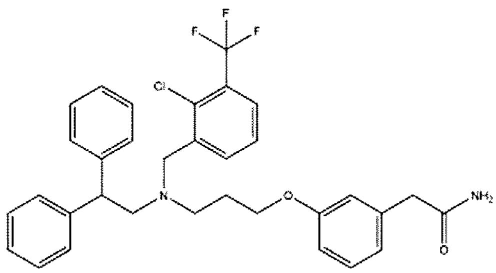

The above DMHCA is an LXR agonist and has Formula 4 below.

[Formula 4]

$C_{26}H_{43}NO_2$ (DMHCA)

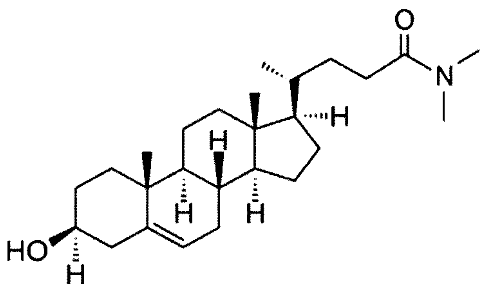

The above LXR-623 is an LXR agonist and has Formula 5 below.

[Formula 5]

$C_{21}H_{12}ClF_5N_2$ (LXR-623)

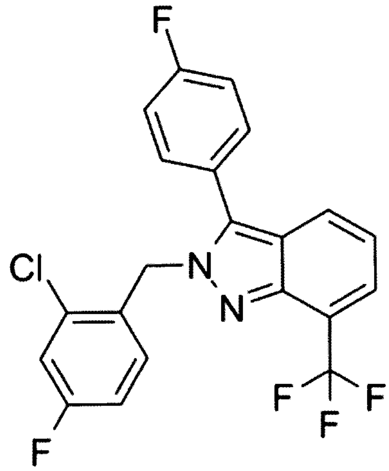

The above WYE-672 is an LXR agonist and has Formula 6 below.

[Formula 6]

$C_{23}H_{17}F_3N_2O_2S$ (WYE-672)

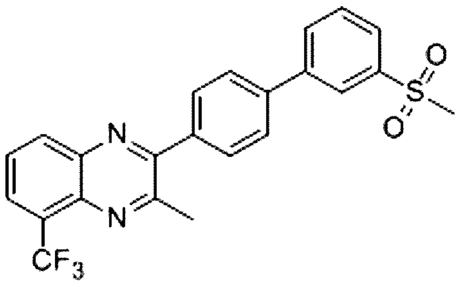

The above AZ876 is an LXR agonist and has Formula 7 below.

[Formula 7]

$C_{24}H_{29}N_3O_3S$ (AZ876)

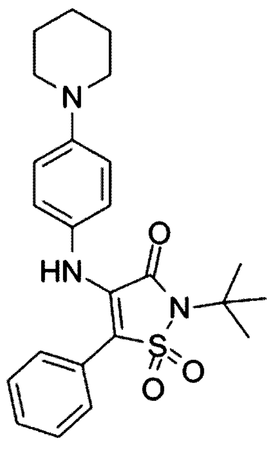

As used herein, the term "ShK" refers to Stichodactyl toxin or a derivative thereof, including forms in which the terminus of the Stichodactyla toxin is substituted with a functional group such as an NHS group. ShK also refers to a

peptide or derivative thereof that functions to restore neuronal cells by inhibiting intracellular potassium channels. The above ShK has the amino acid sequence of Chemical Formula 8 set forth below.

[Formula 8]

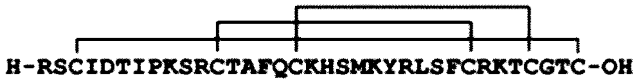

H-RSCIDTIPKSRCTAFQCKHSMKYRLSFCRKTCGTC-OH

As used herein, the term "apolipoprotein E" refers to a mammalian protein encoded by the APOE gene or a functional variant thereof. In a preferred embodiment, the apolipoprotein E is a human protein encoded by the human APOE gene on chromosome 19. The apolipoprotein E may be any one of the isoforms of the APOE gene product, such as apolipoprotein E2 ("APOE2"), apolipoprotein E3 ("APOE3"), and apolipoprotein E4 (APOE4). APOE is a polymorphism having three major alleles (epsilon 2, epsilon 3, and epsilon 4). Any of the alleles may be used in various embodiments of the present invention. A "functional variant" of the present invention refers to a variant of a mammalian protein encoded by an APOE gene that retains the same or similar biological function as the APOE gene product. In some cases, the functional variant comprises amino acid insertions, deletions, and/or substitutions compared to the protein encoded by the human APOE gene. In some cases, the functional variant is a fragment of the protein encoded by the human APOE gene.

In some embodiments, the apolipoprotein E2 is a protein disclosed in GenBank under the accession number ARQ79459 or a protein having at least 95% sequence identity thereto, preferably at least 98% or 99% sequence identity thereto. In some embodiments, the apolipoprotein E3 is a protein disclosed in GenBank under the accession number ARQ79461.1 or a protein having at least 95% sequence identity thereto, preferably at least 98% or 99% sequence identity thereto.

In some embodiments, the apolipoprotein E in the reconstituted high-density lipoprotein (rHDL) is a recombinant protein produced by genetic engineering, or a synthetic protein produced by chemical synthesis.

The term "apolipoprotein A1" refers to a mammalian protein encoded by the APOA1 gene or a functional variant thereof. In a preferred embodiment, the apolipoprotein A1 is a human protein encoded by the human APOA1 gene located on chromosome 11. A "functional variant" thereof refers to a variant of a mammalian protein encoded by the APOA1 gene that retains the same or similar biological function as the APOA1 gene product. In some cases, the functional variant comprises amino acid insertions, deletions, and/or substitutions compared to the protein encoded by the human APOA1 gene. In some cases, the functional variant is a fragment of the protein encoded by the human APOA1 gene.

In some embodiments, the apolipoprotein A1 is a protein disclosed in GenBank under the accession number AAS68227.1 or a protein having at least 95% sequence identity thereto, preferably at least 98% or 99% sequence identity thereto.

As used herein, the term "apolipoprotein E" is meant to encompass a fragment and a functional variant thereof.

As used herein, the term "microfluidic device" refers to a device including a microchannel, etc., which is provided to allow a fluid to flow on a substrate made of various materials including plastic, glass, metal, or silicon comprising an organic polymer material.

As used herein, the term "micropillar" or "micropillar structure" refers to a pillar structure that forms a vortex within a microfluidic device to efficiently mix different types of fluids injected into the microfluidic device.

As used herein, the term "prevention" refers to any action of inhibiting or delaying the onset of a disease by administration of a composition, and "treatment" refers to any action in which symptoms of a subject suspected of and suffering from a disease are improved or beneficially changed by administration of a composition.

As used herein, the term "cancer" may be at least one selected from the group consisting of carcinoma, sarcoma, lymphoma, leukemia, germ cell tumor, brain tumor corresponding to blastoma, cervical cancer, ovarian cancer, prostate cancer, lung cancer, bile duct cancer, kidney cancer, gastric cancer, liver cancer, retinoblastoma, choriocarcinoma, small intestine cancer, colorectal cancer and rectal cancer, non-small cell lung cancer, gastric adenocarcinoma, acute lymphocytic leukemia, acute myeloid leukemia, breast cancer, bone sarcoma, bladder cancer, anaplastic astrocytoma, head and neck cancer, pancreatic cancer, and esophageal cancer, but is not limited thereto.

Hereinafter, the present invention will be described in more detail through the examples, but the following examples are for illustrative purposes only and are not intended to limit the scope of the present invention.

**[Example 1]**

**Comparison of cholesterol metabolism-related factors and intracellular cholesterol in normal brain astrocytes and GBM cells**

Cholesterol is a crucial nutrient for normal cellular function and survival. It plays an important role in the plasma

membrane and lipid rafts, and serves as a precursor for steroid hormones, bile acids, and vitamin D. When the intracellular cholesterol level is excessively high or excessively low, cytotoxicity may occur. Therefore, cells regulate cholesterol homeostasis by controlling cholesterol uptake, efflux, and endogenous synthesis. Sterol-responsive nuclear liver X receptors (LXRs) regulate cholesterol homeostasis through activation of cholesterol efflux genes (ABCA1, ABCG1) as well as induction of low-density lipoprotein receptor (LDLR) degradation.

Dysregulation of cholesterol homeostasis is one of the characteristics of cancer. In many cancers such as liver cancer, gastric cancer, colorectal cancer, pancreatic cancer, and prostate cancer, abnormal high cholesterol levels affect cancer progression, including proliferation, metastasis, and invasion. Excessive cholesterol levels in cancer cells occur through increased LDLR-mediated exogenous cholesterol uptake, inhibition of ABCA1-mediated cholesterol efflux, or increased cholesterol synthesis.

Figure 1 is a schematic diagram of cholesterol metabolism in normal brain astrocytes and GBM cells, and of the mechanism of GBM death induced by 24HC-containing rHDL nanoparticles. Normal brain astrocytes (normal human astrocytes) maintain intracellular cholesterol homeostasis by converting excessive intracellular cholesterol to 24HC by the cytochrome P450 enzyme cholesterol 24-hydroxylase (Cytochrome P450 Family 46 Subfamily A Member 1, CYP46A1). 24HC functions as an agonist of LXR, and LXR activation suppresses the expression of LDLR, which is involved in cholesterol uptake, and increases the expression of ABCA1 and ABCG1, which are involved in cholesterol efflux, thereby reducing intracellular cholesterol. GBM cells are known to have relatively low levels of CYP46A1 compared to normal cells, resulting in low intracellular 24HC levels. Accordingly, the present invention was designed to deliver 24HC-containing rHDL nanoparticles into GBM cells in order to increase the intracellular level of 24HC, an LXR agonist, thereby inducing cell death byintracellular cholesterol depletion.

Interestingly, GBM cells are unable to synthesize new cholesterol via the mevalonate pathway and rely on cholesterol uptake through LDLR. We investigated RNA gene expression in GBM and normal brain samples using data from The Cancer Genome Atlas (TCGA) and Genotype-Tissue Expression (GTEx).

As shown in Figure 2, analysis of RNA genes related to cholesterol metabolism enzymes in TCGA revealed that the expression of CYP46A1, which converts cholesterol to 24HC, was lower in GBM compared to normal brain tissue. This suggests that GBM cells have a diminished ability to convert cholesterol to 24HC compared with normal cells.

As shown in Figure 3, analysis of LDLR RNA genes from TCGA revealed that the expression of LDLR RNA was higher in GBM compared to normal brain tissue. Because GBM cells are unable to synthesize intracellular cholesterol, they rely on exogenous cholesterol through LDLR, and therefore LDLR is overexpressed in GBM compared with normal brain tissue.

Astrocytes, which can represent normal brain cells, and representative GBM cell lines U87MG and LN229 were compared the intracellular levels of 24HC and cholesterol. Astrocytes were cultured on poly-L-lysine-coated plastic flask using AM medium. U87MG cells were cultured in MEM medium, and LN229 cells were cultured in DMEM medium, each medium supplemented with 10% FBS and 1% penicillin-streptomycin (P/S). Cells were incubated at 37 °C in a 5% $CO_2$ atmosphere and 95% relative humidity. In fact, we found that the intracellular 24HC level in U87MG and LN229 cells were significantly lower than those in astrocyte cells (Figure 4), and this was shown to actually induce excessive accumulation of cholesterol in GBM cells (Figure 5).

**[Example 2]**

**Method for producing 24HC-containing rHDL nanoparticles**

A microfluidic device was designed to produce rHDL nanoparticles by mixing phospholipids and apolipoproteins (Figure 6). The microfluidic device comprises three inlets and one outlet. Phospholipids, which are hydrophobic, were injected into an inlet located in the center of the microfluidic device, whereas apolipoproteins, which are hydrophilic, were injected into two inlets located on either side. In addition, a micropillar structure was introduced within the microfluidic device to effectively mix lipids and apolipoproteins.

The nanoparticles comprising phospholipids and apolipoproteins were produced using the microfluidic device as follows. A solution of DMPC (1,2-dimyristoyl-sn-glycero-3-phosphocholine) in absolute ethanol and a solution of apolipoprotein E3 in PBS were prepared. Thereafter, approximately 0.8 mL of the DMPC solution at a concentration of 0.83 mg/mL in absolute ethanol was filled into one syringe, and approximately 1.25 mL (a total of about 2.5 mL) of the apolipoprotein solution in PBS at a concentration of 0.2 mg/mL was filled into each of two other syringes. After filling, all air bubbles were removed from the syringes.

Each syringe needle was connected to the inlet of the microfluidic device using a tube. PBS was introduced through the outlet at a flow rate of 1 mL/min to wash the microfluidic device. Thereafter, the injection flow rate of the DMPC solution was adjusted to 0.8 mL/min, and the injection flow rate of the apolipoprotein E3 solution was adjusted to 4.4 mL/min using a syringe pump.

The rHDL nanoparticles were collected through the outlet of the microfluidic device. The collected rHDL

nanoparticles were mixed with PBS and purified three times using a 10K filter by centrifugation at maximum speed for 20 minutes at 4 °C.

A 24HC solution (a molecular weight of 402.7 g/mol) in DMSO was prepared at a concentration of 1 mg/mL. The concentration of apolipoprotein E3 in the collected rHDL nanoparticles was measured, and 24HC was added dropwise to each nanoparticle such that the molar ratios of apolipoprotein E3:24HC were 1:2, 1:3, and 1:4. At this time, the amount of DMSO did not exceed 10% of the total amount of 24HC-containing rHDL nanoparticles to be finally produced. After storing at room temperature for 12-16 hours, it was mixed with PBS and then purified using a 10K filter by centrifugation at maximum speed for 20 minutes at 4 °C.

**[Example 3]**

**Method for producing rHDL nanoparticles comprising ShK**

The rHDL nanoparticles were obtained in the same manner as in Example 2. The obtained rHDL nanoparticles were mixed with PBS and then purified three times using a 10K filter by centrifugation at maximum speed for 20 minutes at 4 °C.

A solution of hydroxysuccinimide cholesterol in anhydrous acetonitrile and a solution of ShK (6-FAM-AEEAc-Stichodacty1a helianthus neurotoxin (ShK) trifluoroacetate salt) in PBS were prepared. The cholesterol solution and the ShK solution were mixed at a molar ratio of 1:2 and allowed to react at room temperature for 4 hours. Thereafter, the mixture was purified three times using a 3K filter by centrifugation at maximum speed for 20 minutes at 4 °C to prepare a ShK-cholesterol solution.

The prepared rHDL nanoparticle solution and the ShK-cholesterol solution were mixed using a vortex mixer at a molar ratio of 1:2 and then allowed to react at room temperature for 4 hours. Thereafter, the mixture was purified once using a 10K filter by centrifugation at maximum speed for 20 minutes at 4 °C to obtain rHDL nanoparticles comprising ShK-cholesterol.

**[Example 4]**

**Optimization of the molar mixing ratio of apolipoprotein E3 and 24HC for synthesis of 24HC-containing rHDL nanoparticles**

In order to synthesize 24HC-containing rHDL nanoparticles, the following experiment was conducted to determind the synthetic mixing molar ratio of apolipoprotein and cholesterol in which 24HC is optimally contained. The 24HC-containing rHDL nanoparticles were synthesized at apolipoprotein E3:24HC molar ratios of 1:2, 1:3, and 1:4, respectively, and the average particle size and particle size distribution were compared depending on the molar mixing ratio.

The 24HC-containing rHDL nanoparticles, prepared in the same manner as in Example 2 above with varying molar ratios of apolipoprotein E3:24HC, were suspended in PBS. Thereafter, the changes in particle size distribution due to aggregation of the nanoparticles was measured by means of dynamic light scattering (DLS) using Zetasizer Nano ZS.

[Table 1]

| Volume-based (n=3) | | |
|---|---|---|
| ApoE3:24HC (molar ratio) | Size (nm) | Volume (%) |
| 1:2 | 18.2 ± 5.4 | 16.9 ± 1.1 |
| 1:3 | 19.7 + 7.3 | 18.6 + 3.4 |
| 1:4 | 21.7 ± 1.9 | 19.2 + 1.8 |

As shown in Table 1 above and Figures 8 to 10, it was confirmed that the 24HC-containing rHDL nanoparticles synthesized at each molar ratio had a size of 25 nm or less.

To confirm the amount of 24HC encapsulated in the 24HC-containing rHDL nanoparticles, 24HC was quantified using a cholesterol quantification kit. The results are shown in Table 2 below.

[Table 2]

| No. | ApoE3:24HC (molar ratio) | Amount of ApoE3 injected (mg) | Amount of 24HC injected (μg) | Final encapsulated 24HC (μg) | 24HC encapsulation rate (%) |
|---|---|---|---|---|---|
| 1 | 1:2 | 0.7 | 14.4 | 9.6 | 66.7 |
| 2 | 1:3 | 0.7 | 24 | 19.9 | 83.0 |
| 3 | 1:4 | 0.7 | 32 | 19.5 | 61.0 |

Encapsulation rate = ((actual amount of 24HC in nanoparticles) / (amount of 24HC injected when producing nanoparticles)) × 100

As shown in Table 2 above, it was confirmed that when the synthetic mixing molar ratio of apolipoprotein E3:24HC in rHDL nanoparticles was 1:2 and 1:4, the encapsulation rate of 24HC was 61-67%, while when the synthetic mixing molar ratio was 1:3, the encapsulation rate was 83%, which was the highest rate (Figure 11). It can be seen that there is a limit to the encapsulation of 24HC even when 24HC is injected to rHDL nanoparticles at a synthetic mixing molar ratio of 1:3 or higher. Therefore, 24HC-containing rHDL nanoparticles with a synthetic mixing molar ratio of apolipoprotein E3:24HC in rHDL nanoparticles of 1:3, which resulted in the highest 24HC encapsulation rate, were selected as the final particles.

While rHDL nanoparticles had a particle size of 16.8 nm (Figure 7), when the synthetic mixing molar ratio of apolipoprotein E3:24HC in rHDL nanoparticles was 1:3 or higher, the particle size increased to 18.6 nm (Figure 12). It was confirmed that the particle size increased due to 24HC encapsulation.

In order to confirm the final composition ratio of apolipoprotein E3, phospholipid, and 24HC in 24HC-containing rHDL nanoparticles with a synthetic mixing molar ratio of apolipoprotein E3:24HC of 1:3 according to the production method of Example 2 above, the final composition ratio was quantified using a BCA assay, a lipid quantification kit, and a cholesterol quantification kit. The results are shown in Table 3 and Figures 13 and 14.

[Table 3]

| | ApoE3 (%) | DMPC (%) | 24HC (%) |
|---|---|---|---|
| rHDL nanoparticle | 67.0 | 33.0 | - |
| 24HC-containing rHDL nanoparticles | 64.8 | 33.1 | 2.2 |

As shown in Table 3 above, it was confirmed that both rHDL nanoparticles and 24HC-containing rHDL nanoparticles had similar lipid contents of 33%, but the protein composition of the 24HC-containing rHDL nanoparticles was lower than that of rHDL nanoparticles due to the presence of 24HC.

**[Example 5]**

**Confirmation of stability of rHDL nanoparticles and 24HC-containing rHDL nanoparticles**

Stability tests were conducted on rHDL nanoparticles and 24HC-containing rHDL nanoparticles in serum and under high ionic strength conditions. Both nanoparticles were diluted in serum (FBS, fatal bovine serum) (Figure 15), phosphate-buffered saline (pH 6.8; GBM pH) (Figure 16), and saline (pH 7.0; body fluid pH) (Figure 17) and then incubated at 37 °C. Nanoparticles at a predetermined amounts were dissolved in PBS at designated times points (0, 2, 4, 8, and 24 hours), and then the particle size was measured. It was confirmed that both nanoparticles maintained their initial particle size for up to 24 hours under the three conditions described above. In addition, it was confirmed that both nanoparticles stably maintained their initial particle size (Figure 18) and particle dispersity index (Figure 19) for 60 days under the conditions of 4 °C. Based on these results, it was confirmed that both nanoparticles are stable under in vivo conditions and exhibit excellent storage stability.

**[Example 6]**

**Evaluation and comparison of blood-brain barrier cell influx and permeability of rHDL nanoparticles and 24HC-containing rHDL nanoparticles**

The ability of rHDL nanoparticles and 24HC-containing rHDL nanoparticles produced according to the method of Example 2 above to penetrate the blood-brain barrier (BBB) and to be delivered to the brain was confirmed. The cell influx experiment was performed using human brain microvascular endothelial cells (hBMEC) and human iPSC-derived brain microvascular endothelial cells (iBMEC) as follows. hBMEC and iBMEC cells were cultured on collagen IV/fibronectin-coated flasks. hBMEC cells were cultured in ECM medium, and iBMEC cells were cultured in hESFM+B27+bFGF+RA medium. After 24 hours of incubation, the medium for iBMEC cells was replaced with hESFM+B27 medium. All cells were cultured at 37 °C in a 5% $CO_2$ atmosphere and 95% relative humidity. hBMEC and iBMEC cells were seeded in confocal dishes at 2 x $10^5$/dish and 1.2 x $10^5$ cells/dish, respectively. After 24 hours of incubation, the cells were treated with DiD-labeled rHDL nanoparticles and DiD-labeled 24HC-containing rHDL nanoparticles for 4 hours, respectively, using medium without FBS and P/S. After treatment, the supernatant was completely removed and washed with PBS, and the cells were fixed with a 4% paraformaldehyde (PFA) solution for 10 minutes. Thereafter, the 4% PFA solution was removed, and mounting medium with DAPI and Microtubule Stain reagent was added. The samples were covered with cover glass and observed using a confocal laser scanning microscopy (CLSM). The influx of both DiD-rHDL nanoparticles and DiD-24HC-containing rHDL nanoparticles into hBMECs (Figure 20) and iBMECs (Figure 22) was confirmed (scale bar: 50 μm). Quantification of rHDL nanoparticles (Figure 21) and 24HC-containing rHDL nanoparticles (Figure 23) delivered into the cells using ImageJ showed no difference in the amounts of the two nanoparticles delivered into the cells. These results indicate that the inclusion of 24HC in rHDL nanoparticles does not affect the delivery of rHDL across the BBB.

In order to confirm the BBB permeability efficiency of rHDL nanoparticles and 24HC-containing rHDL nanoparticles, a BBB permeability experiment was performed using hBMECs as follows. hBMECs were cultured in the insert well of a collagen IV/fibronectin-coated transwell at a density of 3.3 x $10^5$ cells/well for 48 hours. The cells were then treated with DiD-labeled rHDL nanoparticles and DiD-labeled 24HC-containing rHDL nanoparticles, respectively, for 24 hours. Thereafter, quantitative analysis of the nanoparticles penetrated into the bottom well was performed. As shown in Figure 24, it was confirmed that the hBMEC permeability of rHDL nanoparticles and 24HC-containing rHDL nanoparticles was high, with no difference observed between the two. This confirms that the BBB permeability of 24HC-containing rHDL nanoparticles is the same as that of rHDL, and that ability of rHDL to penetrate the BBB is not diminished by the inclusion of 24HC. In conclusion, it was confirmed that both rHDL nanoparticles and 24HC-containing rHDL nanoparticles penetrate the BBB and are delivered to the brain.

**[Example 7]**

**Evaluation of safety of 24HC-containing rHDL nanoparticles in blood-brain barrier cells (hBMECs, iBMECs, pericytes) and brain astrocytes**

The toxicity of 24HC-containing rHDL nanoparticlestoward hBMECs, iBMECs, pericytes, and normal brain astrocytes, which constitute the BBB until the nanoparticles penetrate the BBB and reach the GBM, was evaluated. hBMECs, iBMECs, pericytes, and astrocytes were seeded into a 96-well cell culture plate. Pericytes were cultured in PM medium, astrocytes were cultured in the same culture medium as described in Example 1, and hBMEC and iBMEC cells were cultured in the same culture medium as described in Example 6. After 24 hours of incubation, the cells were treated with the nanoparticles according to the present invention at concentrations ranging from 0to 5 μg/mL, and the cell survival rate (%) was evaluated after 8 hours of incubation. As shown in Figures 25 to 28, none of the rHDL nanoparticles, 24HC, or 24HC-containing rHDL nanoparticles exhibited toxicity in hBMECs, iBMECs, pericytes, or astrocytes. Based on these results, it was confirmed that the nanoparticles according to the present invention can be delivered across the BBB without toxicity. In addition, it was confirmed that the nanoparticles are not toxic to normal brain cells other than GBM.

**[Example 8]**

**Confirmation of influx of 24HC-containing rHDL nanoparticles into GBM cells via LDLR**

ApoE consists of two independently folded structural domains: the high-lipid binding domain (C-terminal domain) and the LDLR binding domain (N-terminal domain). Interestingly, the ApoE3-lipid complex, i.e., rHDL, exhibits stronger binding affinity to the LDLR binding domain (residues 140-150) than ApoE alone. Therefore, based on the finding that lipid binding induces a conformational change and confers LDLR recognition properties, we hypothesized that rHDL would be a suitable nanoparticle for delivering 24HC to GBM cells overexpressing LDLR.

The cellular uptake mechanism of 24HC-containing rHDL nanoparticles was evaluated in U87MG and LN229 cells, which are GBM cells overexpressing LDLR. In order to determine the influx of the rHDL nanoparticles and the 24HC-containing rHDL nanoparticles according to the present invention into GBM cells via LDLR, LDL (low density lipoprotein, human) was used as a competitive inhibitor. U87MG and LN228 cells were cultured in the same medium and under the same conditions as described in Example 1 above. After 24 hours of incubation, the cells were pretreated with LDL at a concentration of 50 μg/mL for 2 hours in medium without FBS and P/S. The cells were treated with Cy5.5-labeled rHDL nanoparticles (Cy5.5-rHDL nanoparticles) and Cy5.5-labeled 24HC-containing rHDL nanoparticles (Cy5.5-24HC-containing rHDL nanoparticles) for 4 hours, respectively. After treatment, the supernatant was completely removed and washed with PBS, and the cells were fixed with a 4% paraformaldehyde (PFA) solution for 10 minutes. Thereafter, the 4% PFA solution was removed, and mounting medium with DAPI reagent was added. The samples were covered with cover glass and observed using a confocal laser scanning microscopy (CLSM).

The cellular uptake efficiency of rHDL nanoparticles and 24HC-containing rHDL nanoparticles was quantitatively analyzed using ImageJ software. In order to confirm the cellular fluorescence level in the fluorescence microscopy images, corrected total cell fluorescence (CTCF) was performed for each cell. Individual cells were selected using the freehand drawing tool to create a ROI. The average background level was obtained by measuring the fluorescence intensity in three different regions outside the cells and averaging the values. The CTCF for each cell was calculated using the following formula:

CTCF = Integrated density of cell ROI - (ROI region × average fluorescence of background)

As shown in Figures 29 and 31, the intracellular influx of Cy5.5-rHDL nanoparticles and Cy5.5-24HC-containing rHDL nanoparticles was compared (scale bar: 50 μm). Both Cy5.5-rHDL nanoparticles and Cy5.5-24HC-containing rHDL nanoparticles were influxed into the cytoplasm. Interestingly, the intracellular influx of Cy5.5-rHDL nanoparticles and Cy5.5-24HC-containing rHDL nanoparticles was inhibited by pretreatment with LDL, which serves as a competitive inhibitor of LDLR. As shown in Figures 30 and 32, as a result of quantifying intracellularly delivered nanoparticles, it was confirmed that the intracellular influx of nanoparticles was inhibited in the group pretreated with LDLR. Based on these results, it was confirmed that the nanoparticles according to the present invention are influxed into the cells via LDLR, and that even when 24HC is contained, the intracellular influx occurs via LDLR.

In order to confirm that treatment of GBM with 24HC-containing rHDL nanoparticles delivers 24HC into cells more efficiently via LDLR than treatment with 24HC solution alone, the cells were treated with 24HC solution and 24HC-containing rHDL nanoparticles, and the amount of intracellular 24HC was quantified using a 24(S)-hydroxycholesterol ELISA kit. It was confirmed that a greater amount of intracellular 24HC was delivered in the group treated with 24HC-containing rHDL nanoparticles compared to the group treated with 24HC solution (Figure 33). The amount of intracellular 24HC was increased by 40% and 20% in U87MG and LN229 cells, respectively, in the group treated with 24HC-containing rHDL nanoparticles compared to the group treated with 24HC. While 24HC is transported vertically from the outer layer to the inner layer of the cell membrane via local diffusion, 24HC-containing rHDL nanoparticles rapidly delivered 24HC into cells via LDLR. Consistent with our results, 24HC-containing rHDL nanoparticles targeted LDLR overexpression in GBM and increased 24HC uptake and accumulation compared to 24HC solution. Based on the above, it was confirmed that the rHDL nanoparticles according to the present invention serve as drug delivery vehicles for more effectively delivering 24HC into GBM cells than treatment with 24HC solution alone.

**[Example 9]**

**Confirmation of changes in LDLR and ABCA1 mRNA expression in GBM by treatment with 24HC-containing rHDL nanoparticles**

24HC is an endogenous LXR agonist and is known to induce the expression of genes responsible for cholesterol efflux, such as ABCA1 and ABCG1, thereby inducing intracellular cholesterol efflux, and to inhibit exogenous cholesterol uptake by downregulating LDLR. Accordingly, we anticipated that treatment of GBM with 24HC-containing rHDL nanoparticles would result in a greater decrease in LDLR expression and an increase in ABCA1 expression due to the action of 24HC, compared to the treatment with 24HC alone. Therefore, U87MG and LN229 cells were treated with 24HC, 24HC-containing rHDL nanoparticles, and rHDL nanoparticles, and the changes in LDLR and ABCA1 mRNA expression were confirmed using qPCR (real-time polymerase chain reaction).

U87MG and LN229 cells were seeded at 0.5 x $10^5$ cells/well in a 6-well cell culture plate and cultured for 24 hours under the conditions described in Example 1 above. In qPCR experiments, U87MG cells were treated with 1 μg/mL of 24HC for 24 hours, and LN229 cells were treated with 5 μg/mL of 24HC for 24 hours, and changes in LDLR mRNA were confirmed. Changes in ABCA1 mRNA expression were confirmed after 4 hours of treatment. In the case of rHDL nanoparticles, the amount of apolipoprotein E3 in the 24HC-containing rHDL nanoparticles was quantified using a BCA assay, and the same amount of apolipoprotein E3 corresponding to the 24HC treatment concentration was calculated prior

to treatment with rHDL nanoparticles.

Figures 34 and 35 show the results of the changes in LDLR and ABCA1 mRNA expression in each cell type according to treatment with the nanoparticles according to the present invention. When U87MG and LN229 cells were treated with 24HC and 24HC-containing rHDL nanoparticles, LDLR mRNA expression was decreased compared to the control group (Figure 34). In U87MG cells, the level of LDLR mRNA expression was reduced to 0.5-fold in the group treated with 24HC-containing rHDL nanoparticles compared to the control group, and to 0.8-fold compared to the group treated with 24HC (o in Figure 34). In LN229 cells, the level of LDLR mRNA expression was reduced to 0.2-fold in the group treated with 24HC-containing rHDL nanoparticles compared to the control group, and to 0.3-fold lower compared to the group treated with 24HC (△ in Figure 34) (U87MG, LN229; ****$p \leq 0.0001$).

Conversely, it was confirmed that in U87MG and LN229 cells, the level of ABCA1 mRNA expression was increased in the group treated with 24HC and 24HC-containing rHDL nanoparticles compared to the control group (Figure 35). In U87MG cells, the level of ABCA1 mRNA expression was 2.7-fold higher in the group treated with 24HC-containing rHDL nanoparticles compared to the control group, and 1.6-fold higher compared to the group treated with 24HC (o in Figure 35). In LN229 cells, the level of ABCA1 mRNA expression was 3.8-fold higher in the group treated with 24HC-containing rHDL nanoparticles compared to the control group, and 1.1-fold higher compared to the group treated with 24HC (△ in Figure 35) (U87MG, LN229; ****$p \leq 0.0001$).

As a result of comprehensive verification, it was confirmed that, compared to treatment with 24HC alone, treatment with 24HC-containing rHDL nanoparticles resulted in a greater reduction in LDLR mRNA expression and a greater increas in ABCA1 mRNA expression in cell. This suggests that, as confirmed in Example 8, 24HC-containing rHDL nanoparticles exhibited higher influx into GBM cells via LDLR compared to with 24HC, and therefore a stronger LXR agonist effect of 24HC is achieved when treated with 24HC-containing rHDL nanoparticles.

**[Example 10]**

**Confirmation of increased cholesterol efflux in GBM by 24HC-containing rHDL nanoparticles**

We confirmed whether 24HC-containing rHDL nanoparticles induced substantial intracellular cholesterol efflux by increasing ABCA1 expression. U87MG and LN229 cells were treated with rHDL nanoparticles, 24HC, and 24HC-containing rHDL nanoparticles, and the level of intracellular cholesterol efflux was quantified using a cholesterol efflux kit. U87MG and LN229 cells were seeded at $1.2 \times 10^5$ cells/well in a 96-well cell culture plate, and the experimental protocol for the cholesterol efflux kit was followed. Each cell line was treated with 24HC or 24HC-containing rHDL nanoparticles at a concentration of 1 μg/mL for U87MG cells and 5 μg/mL for LN229 cells for 24 hours. In the case of rHDL nanoparticles, the amount of apolipoprotein E3 in the 24HC-containing rHDL nanoparticles was quantified using a BCA assay, and then the same amount of apolipoprotein E3 corresponding to the 24HC treatment concentration was calculated prior to treatment with rHDL nanoparticles. After 24 hours, the fluorescence values of cholesterol effluxed from the cells and intracellular cholesterol were measured, respectively. The cholesterol efflux rate (%) was calculated by dividing the effluxed extracellular cholesterol in each treatment group by the intracellular cholesterol in the control group.

Figure 36 shows the results of intracellular cholesterol efflux in each cell when treated with the nanoparticles according to the present invention. In U87MG cells, the cholesterol efflux (%) was 8.4-fold higher in the group treated with 24HC-containing rHDL nanoparticles compared to the control group and was 4.7-fold higher compared to the group treated with 24HC (o in Figure 36). In LN229 cells, the cholesterol efflux (%) was 20.3-fold higher in the group treated with 24HC-containing rHDL nanoparticles compared to the control group and was 15.5-fold higher compared to the group treated with 24HC (△ in Figure 36) (U87MG, LN229; ***$p \leq 0.001$, ****$p \leq 0.0001$).

In conclusion, the treatment with 24HC-containing rHDL nanoparticles resulted in the greatest cholesterol efflux in all cells. This suggests that the higher cholesterol efflux (%) in the group treated with 24HC-containing rHDL nanoparticles compared to other treatment groups was due to the effect of increasing intracellular cholesterol efflux by the induction of the increased level of ABCA1 mRNA expression, as shown in Example 9.

Using probucol, an ABCA1 inhibitor, we confirmed whether 24HC-containing rHDL nanoparticles increased cholesterol efflux through the increased ABCA1 expression. U87MG and LN229 cells were pretreated with 40 μM Probucol for 4 hours and treated with 24HC-containing rHDL nanoparticles and rHDL nanoparticles at a concentration of 5 μg/mL of 24HC for 4 hours, and the changes in cholesterol efflux were quantified using a cholesterol efflux kit. In the case of rHDL nanoparticles, the amount of apolipoprotein E3 in the 24HC-containing rHDL nanoparticles was quantified using a BCA assay, and then the same amount of apolipoprotein E3 corresponding to the 24HC treatment concentration was calculated prior to treatment with rHDL nanoparticles.

Figure 37 shows the results obtained by confirming cholesterol efflux from each cell when treated with the nanoparticles according to the present invention after treatment with an ABCA1 inhibitor. It was confirmed that cholesterol efflux was reduced in the group treated with nanoparticles after treatment with the ABCA1 inhibitor, probucol, compared to the group not treated with probucol. In U87MG cells, the cholesterol efflux (%) in the group treated with 24HC-containing

rHDL nanoparticles was reduced by 32.27% in the group treated with probucol (+) compared to the untreated group (-) (**$p \leq 0.01$). The cholesterol efflux (%) in the group treated with rHDL nanoparticles was reduced by 29.3% in the group treated with probucol (+) compared to the untreated group (-) (o in Figure 37) (*$p \leq 0.05$).

In LN229 cells, the cholesterol efflux (%) in the group treated with 24HC-containing rHDL nanoparticles was reduced by 45.7% in the group treated with probucol (+) compared to the untreated group (-), and the cholesterol efflux (%) in the group treated with rHDL nanoparticles was reduced by 50.0% in the group treated with probucol (+) compared to the untreated group (-) (△ in Figure 37) (LN229; ***$p \leq 0.001$). Based on these results, it can be seen that ABCA1, which is involved in cholesterol efflux, was inhibited by probucol, thereby inhibiting cholesterol efflux of rHDL nanoparticles and 24HC-containing rHDL nanoparticles.

In conclusion, 24HC-containing rHDL nanoparticles exhibited ABCA1-mediated cholesterol efflux. These data demonstrate that 24HC loaded on 24HC-containing rHDL nanoparticles activates LXR and induces cholesterol efflux through the increase in ABCA1. Interestingly, rHDL nanoparticles did not increase ABCA1 mRNA expression, but induced cholesterol efflux. These results are supported by previous studies demonstrating that ApoE induces cholesterol efflux through ABCA1.

**[Example 11]**

**Evaluation of the effect of treatment with 24HC-containing rHDL nanoparticles on GBM cell death induced by cholesterol depletion**

In order to determine whether cholesterol depletion caused by intracellular cholesterol efflux inhibits GBM cell proliferation and induces cell death, U87MG and LN229 cells were treated with rHDL nanoparticles, 24HC, and 24HC-containing rHDL nanoparticles, and the cell survival rate was evaluated. After 24 hours of incubation, the cells were treated with the nanoparticles according to the present invention at concentrations of 0-50 μg/mL, and the cell survival rate (%) was evaluated after 48 hours of incubation. As shown in Table 4 and Figures 38 and 39, the group treated with 24HC-containing rHDL nanoparticles exhibited $IC_{50}$ values of 0.4 and 0.9 μg/mL in U87MG and LN229 cells, respectively. The group treated with 24HC alone exhibited $IC_{50}$ values of 6.7 and 12.8 μg/mL in U87MG and LN229 cells, respectively. The group treated with 24HC-containing rHDL nanoparticles exhibited an $IC_{50}$ value approximately 14-fold lower compared to the group treated with 24HC alone. These results are considered to be due to the higher cellular uptake efficiency of 24HC-containing rHDL nanoparticles compared to 24HC, the increased expression of ABCA1, and the greater intracellular cholesterol depletion caused by increased cholesterol efflux. In conclusion, we demonstrated that 24HC-containing rHDL nanoparticles deplete intracellular cholesterol in GBM cells, resulting in high cytotoxicity.

[Table 4]

| Cell | Formulation | IC50 values (unit: μg/mL) |
|---|---|---|
| | | 48 h |
| U87MG | rHDL nanoparticles | 64.4 |
| | 24HC | 6.7 |
| | 24HC-containing rHDL nanoparticles | 0.4 |
| LN229 | rHDL nanoparticles | 48.9 |
| | 24HC | 12.8 |
| | 24HC-containing rHDL nanoparticles | 0.9 |

**[Example 12]**

**Confirmation of influx of 24HC-containing rHDL nanoparticles into patient-derived GBM cells**

Using 24HC-containing rHDL nanoparticles produced according to the production method of Example 2 above, the influx of the nanoparticles into patient-derived GBM cells was confirmed as follows. GBM#P1 and GBM#P2 cells were cultured in DMEM/F12 medium supplemented with 1% P/S, 15% FBS, EGF (50 ng/mL), and FGF-10 (50 ng/mL). Each cell was cultured at 37 °C in a 5% $CO_2$ atmosphere and 95% relative humidity. The cells were processed and quantified as described in Example 8 above.

Figure 40 shows the results of intracellular influx of Cy5.5-rHDL nanoparticles and Cy5.5-24HC-containing rHDL nanoparticles according to the present invention (scale bar: 50 μm). It was confirmed that both Cy5.5-rHDL nanoparticles

and Cy5.5-24HC-containing rHDL nanoparticles were influxed into the cytoplasm, similar to U87MG and LN229 cells. Figure 41 shows the quantitative analysis of influx of each nanoparticle into GBM#P1 cells, and Figure 42 shows the quantitative analysis of influx of each nanoparticle into GBM#P2 cells. Based on the intracellular experimental results, it was quantitatively confirmed that the rHDL nanoparticles according to the present invention were delivered into GBM#P1 and GBM#P2 cells, and that even when 24HC was contained in the rHDL nanoparticles, the influx efficiency into GBM cells remained unchanged.

**[Example 13]**

**Confirmation of changes in LDLR and ABCA1 mRNA expression in patient-derived GBM cells by 24HC-containing rHDL nanoparticles**

GBM#P1 and GBM#P2 cells were treated with 24HC, 24HC-containing rHDL nanoparticles, and rHDL nanoparticles, and the changes in LDLR and ABCA1 mRNA expression were confirmed using qPCR. GBM#P1 and GBM#P2 cells were seeded at $0.5 \times 10^5$ cells/well in a 6-well cell culture plate and were cultured for 24 hours. The cells were cultured under the conditions as described in Example 12 above. GBM#P1 and GBM#P2 cells were treated with 24HC and 24HC-containing rHDL nanoparticles at a concentration of 2 μg/mL of 24HC for 4 hours. In the case of rHDL nanoparticles, the amount of apolipoprotein E3 in the 24HC-containing rHDL nanoparticles was quantified using a BCA assay, and then the same amount of apolipoprotein E3 corresponding to the 24HC treatment concentration was calculated prior to treatment with rHDL nanoparticles.

When GBM#P1 and GBM#P2 cells were treated with 24HC and 24HC-containing rHDL nanoparticles, the level of LDLR mRNA expression was significantly reduced compared to the control group, and this reduction was statistically significant (Figure 43). The level of LDLR mRNA expression was 0.5-fold lower in the group treated with 24HC-containing rHDL nanoparticles compared to the control group, and was 1.3-fold lower compared to the group treated with 24HC (GBM#P1, GBM#P2; ****$p \leq 0.0001$). In addition, the level of ABCA1 mRNA expression was significantly increased in the group treated with 24HC-containing rHDL nanoparticles compared to the control group. In the group treated with 24HC-containing rHDL nanoparticles, the level of ABCA1 mRNA expression was increased 5.1-fold compared to the control group, 2.1-fold compared to the group treated with 24HC, and 5.7-fold compared to the group treated with rHDL nanoparticles (Figure 44). In particular, the group treated with 24HC-containing rHDL nanoparticles showed a significantly difference compared to the control group and the group treated with 24HC (GBM#P1, GBM#P2; ****$p \leq 0.0001$). However, this statistical significance was calculated by integrating the raw data values of the control and experimental groups for GBM#P1 and GBM#P2 cells.

In patient-derived GBM cells, the level of ABCA1 mRNA expression was increased in the group treated with 24HC-containing rHDL nanoparticles compared to the group treated with 24HC alone, in a similar manner to U87MG and LN229 cells. This indicates that 24HC-containing rHDL nanoparticles comprehensively induce intracellular cholesterol depletion in patient-derived GBM cells.

**[Example 14]**

**Confirmation of changes in cholesterol efflux in patient-derived GBM cells by 24HC-containing rHDL nanoparticles**

GBM#P1 and GBM#P2 cells were treated with rHDL nanoparticles, 24HC, and 24HC-containing rHDL nanoparticles, and the level of intracellular cholesterol efflux was quantified using a cholesterol efflux kit. GBM#P1 cells were seeded at $0.2 \times 10^5$ cells/well and GBM#P2 cells were seeded at $0.15 \times 10^5$ cells/well, and the experimental protocol for the cholesterol efflux kit was followed. Fluorescently labeled cholesterol was influxed into the cells, and the cells were cultured. Thereafter, each cell group was treated with rHDL nanoparticles, 24HC, and 24HC-containing rHDL nanoparticles. GBM#P1 and GBM#P2 cells were treated with 24HC or 24HC-containing rHDL nanoparticles at a concentration of 10 μg/mL of 24HC 24 hours. In the case of rHDL nanoparticles, the amount of apolipoprotein E3 in the 24HC-containing rHDL nanoparticles was quantified using a BCA assay, and then the same amount of apolipoprotein E3 corresponding to the 24HC treatment concentration was calculated before treatment with rHDL nanoparticles. After 24 hours, the fluorescence values of cholesterol effluxed from the cells and intracellular cholesterol were measured, respectively. The cholesterol efflux rate (%) was calculated by dividing the extracellular cholesterol in each treatment group by the intracellular cholesterol in the control group.

When GBM#P1 and GBM#P2 were treated with the nanoparticles according to the present invention, both the group treated with rHDL nanoparticles and the group treated with 24HC-containing rHDL nanoparticles showed higher cholesterol efflux (%) compared to the group treated with 24HC, and the group treated with 24HC-containing rHDL nanoparticles effluxed 4.4-fold higher cholesterol efflux compared with the group treated with 24HC (Figure 45) (GBM#P1,

GBM#P2; ***p ≤ 0.001). This suggests that the higher cholesterol efflux (%) in the group treated with 24HC-containing rHDL nanoparticles compared to other treatment groups was due to the high intracellular delivery efficiency of 24HC-containing rHDL nanoparticles as shown in Example 8 and the increased ABCA1 mRNA expression in cells after treatment with 24HC-containing rHDL nanoparticles as shown in Example 13.

In order to confirm whether 24HC-containing rHDL nanoparticles induce cholesterol efflux via ABCA1 in patient-derived GBM cells, GBM#P1 and GBM#P2 cells (0.15 x $10^5$ cells/well) were pretreated with 40 μM probucol, an ABCA1 inhibitor, for 4 hours. The cells were then treated with 24HC-containing rHDL nanoparticles and rHDL nanoparticles for 24 hours, and the changes in cholesterol efflux were confirmed using a cholesterol efflux kit. GBM#P1 and GBM#P2 cells were treated with 24HC-containing rHDL nanoparticles at a concentration of 10 μg/mL of 24HC. It was confirmed that cholesterol efflux was reduced in the nanoparticles-treated groups after treatment with probucol, an ABCA1 inhibitor, compared to the group not treated with probucol (Figure 46). The cholesterol efflux (%) in the group treated with 24HC-containing rHDL nanoparticles was reduced by 34.9% in the group treated with probucol (+) compared to the untreated group (-), and the cholesterol efflux (%) in the group treated with rHDL nanoparticles was reduced by 36.7% in the group treated with probucol (+) compared with the untreated group (-) (GBM#P1, GBM#P2; ****p ≤ 0.0001). However, this significance was calculated by integrating the raw data values of the control and experimental groups for GBM#P1 and GBM#P2 cells.

**[Example 15]**

**Evaluation of efficacy of 24HC-containing rHDL nanoparticles on patient-derived GBM cell death**

In order to determine whether cholesterol depletion due to intracellular cholesterol efflux inhibits GBM cell proliferation and induces cell death, GBM#P1 and GBM#P2 cells were treated with rHDL nanoparticles, 24HC, and 24HC-containing rHDL nanoparticles, and cell viability was evaluated. After 24 hours of incubation, the cells were treated with 0-50 μg/mL of the nanoparticles, and the cell viability (%) was assessed after 48 hours of incubation. As shown in Table 5 and Figures 47 and 48, the group treated with 24HC-containing rHDL nanoparticles exhibited IC50 values of 2.5 and 1.0 μg/mL in GBM#P1 and GBM#P2 cells, respectively. The group treated with 24HC alone showed IC50 values of 40.2 and 13.8 μg/mL in GBM#P1 and GBM#P2 cells, respectively. These results can be interpreted as a consequence of the higher cellular uptake efficiency of 24HC-containing rHDL nanoparticles compared with 24HC, the higher ABCA1 expression, and the increased intracellular cholesterol depletion resulting from enhanced cholesterol efflux. In conclusion, we demonstrated that 24HC-containing rHDL nanoparticles deplete intracellular cholesterol in patient-derived GBM cells, resulting in high cytotoxicity.

[Table 5]

| Cell | Formulation | $IC_{50}$ values (unit: μg/mL) |
|---|---|---|
| | | 48 h |
| GBM#P1 | rHDL nanoparticles | 37.05 |
| | 24HC | 40.2 |
| | 24HC-containing rHDL nanoparticles | 2.5 |
| GBM#P2 | rHDL nanoparticles | 12.0 |
| | 24HC | 13.8 |
| | 24HC-containing rHDL nanoparticles | 1.0 |

## Claims

1. A reconstituted high-density lipoprotein (rHDL) nanoparticle comprising a phospholipid, a cholesterol, and apolipoprotein E.

2. The reconstituted high-density lipoprotein (rHDL) nanoparticle according to claim 1, wherein the cholesterol is at least one selected from the group consisting of cholesterol, 7-ketocholesterol, 7α-hydroxycholesterol, 7β-hydroxycholesterol, 4β-hydroxycholesterol, α-5,6-epoxycholesterol, β-5,6-epoxycholesterol, 24S, 25-epoxycholesterol, 25-epoxycholesterol, 7-hydroxycholesterol, 20(S)-hydroxycholesterol, 22(S)-hydroxycholesterol, 22(R)-hydroxycholesterol, 24(S)-hydroxycholesterol, 24(R)-hydroxycholesterol, 25-hydroxycholesterol, 27-hydroxycholesterol, 5β-epoxycholesterol, 6β-epoxycholesterol, 7-dehydrocholesterol, (25R)26-hydroxycholesterol, 7α,(25R)26-dihydroxycholesterol, 5β-cholestane-3α,7α,12α,(25R)26-tetrol, 7α,25-dihydroxycholesterol, 7α,24S-dihydroxycholesterol, 3β,5α,6β-cholestane-3,5,6,26-tetrol, lanosterol, desmosterol, 7α-hydroxydesmosterol, (24Z),26S-hydroxydesmosterol, (24E),26S-hydroxydesmosterol, 24S,25-epoxylanosterol, 26(Z)-hydroxydesmosterol, 25-hydroxy-8-dehydrocholesterol, 24-hydroxy-8-dehydrocholesterol, 7-dehydrocholesterol, 8-dehydrocholesterol, (25R)26-hydroxy-8-dehydrocholesterol, 25-hydroxy-7-dehydrocholesterol, 4α-hydroxy-7-dehydrocholesterol, 4β-hydroxy-7-dehydrocholesterol, 7,8-epoxycholesterol, 3α,5β,6α-cholestane-3,6-diol, cholestane-3β, 5α, 6β-triol, Oxy34, Oxy49, and Oxy133.

**3.** The reconstituted high-density lipoprotein (rHDL) nanoparticle according to claim 1, wherein the cholesterol is 24-hydroxycholesterol (24HC).

**4.** The reconstituted high-density lipoprotein (rHDL) nanoparticle according to claim 1, wherein the nanoparticle further comprises a liver X receptor (LXR) agonist.

**5.** The reconstituted high-density lipoprotein (rHDL) nanoparticle according to claim 4, wherein the liver X receptor agonist is at least one selected from the group consisting of T0901317, GW3965, GW6340, DMHCA, LXR-623, WYE-672, and AZ876.

**6.** The reconstituted high-density lipoprotein (rHDL) nanoparticle according to claim 1, wherein the nanoparticle further comprises ShK.

**7.** The reconstituted high-density lipoprotein (rHDL) nanoparticle according to claim 1, wherein the synthetic mixing molar ratio of apolipoprotein E and cholesterol is 1:2 to 1:4.

**8.** The reconstituted high-density lipoprotein (rHDL) nanoparticle according to claim 1, wherein the synthetic mixing molar ratio of apolipoprotein E and cholesterol is 1:3.

**9.** The reconstituted high-density lipoprotein (rHDL) nanoparticle according to claim 1, wherein the apolipoprotein E is apolipoprotein E2, E3, or E2 and E3.

**10.** The reconstituted high-density lipoprotein (rHDL) nanoparticle according to claim 1, wherein the reconstituted high-density lipoprotein (rHDL) nanoparticle may further comprise apolipoprotein A1.

**11.** The reconstituted high-density lipoprotein (rHDL) nanoparticle according to claim 1, wherein the phospholipid is at least one selected from the group consisting of 1,2-dioleoyl-sn-glycero-3-phosphocholine (DOPC), egg phosphatidylcholine (EPC), dilauroylphosphatidylcholine (DLPC), 1,2-dimyristoyl-sn-glycero-3-phosphocholine (DMPC), dipalmitoylphosphatidylcholine (DPPC), distearoylphosphatidylcholine (DSPC), 1-myristoy1-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoyl phosphatidylcholine (SPPC), 1,2-diarachidoyl-sn-glycero-3-phosphocholine (DAPC), 1,2-dibehenoyl-sn-glycero-3-phosphocholine (DBPC), 1,2-dierucoyl-sn-glycero-3-phosphocholine (DEPC), palmitoyloleoylphosphatidylcholine (POPC), lysophosphatidylcholine, dilinoleoylphosphatidylcholine, distearoylphosphatidylethanolamine (DSPE), dimyristoylphosphatidylethanolamine (DMPE), dipalmitoylphosphatidylethanolamine (DPPE), palmitoyloleoylphosphatidylethanolamine (POPE), lysophosphatidylethanolamine, N1-[2-((1S)-1-[(3-aminopropyl)amino]-4-[di(3-amino-propyl)amino]butylcarboxamido)ethyl]-3,4-di[oleoyloxy]-benzamide) , dioctadecylamidoglycylspermine 4-trifluoroacetic acid (DOGS), 3β-[N-(N',N'-dimethylaminoethane)-carbamoyl]cholesterol (DC-Chol), 1,2-di-O-octadecenyl-3-trimethylammonium propane (DOTMA), 1,2-dioleoyl-3-trimethylammonium-propane (DOTAP), (1,2-dioleoyloxypropyl)-3-dimethylhydroxyethyl ammonium bromide (DORIE), 1,2-dimyristyloxy-propyl-3-dimethyl-hydroxyethyl ammonium bromide (DMRIE), 2,3-dioleoyloxy-N-[2-(sperminecarboxamido)ethyl]-N,N-dimethyl-1-propanaminium trifluoroacetate (DOSPA), N-(3-aminopropyl)-N,N-dimethyl-2,3-bis(dodecyloxy)-1-propaneammonium bromide (GAP-DLRIE), N-t-butyl-N'-tetradecyl-3-tetradecylaminopropionamidine (diC14-amidine), ethylphosphocholine (Ethyl PC), dimethyldioctadecylammonium bromide (DDAB), N4-cholesteryl-spermine (GL67), 1,2-dioleoyloxy-3-dimethylaminopropane (DODMA), D-Lin-MC3-DMA (MC3, DLin-MC3-DMA), DLin-KC2-DMA, and DLin-DMA.

**12.** The reconstituted high-density lipoprotein (rHDL) nanoparticle according to claim 1, wherein it has a diameter of 25 nm or less.

**13.** The reconstituted high-density lipoprotein (rHDL) nanoparticle according to claim 3, wherein the nanoparticle increases the expression of ABCA1.

**14.** The reconstituted high-density lipoprotein (rHDL) nanoparticle according to claim 3, wherein the nanoparticle increases the cholesterol efflux.

**15.** The reconstituted high-density lipoprotein (rHDL) nanoparticle according to claim 3, wherein the nanoparticle kills cancer cells.

**16.** The reconstituted high-density lipoprotein (rHDL) nanoparticle according to claim 1, wherein the nanoparticle is influxed into cells via LDLR.

**17.** A composition for preventing or treating Alzheimer's disease or cancer, comprising the nanoparticles according to claim 3.

**18.** The composition for preventing or treating cancer according to claim 17, wherein the cancer is selected from the group consisting of carcinoma, sarcoma, lymphoma, leukemia, germ cell tumor, brain tumor corresponding to blastoma, cervical cancer, ovarian cancer, prostate cancer, lung cancer, bile duct cancer, kidney cancer, gastric cancer, liver cancer, retinoblastoma, choriocarcinoma, small intestine cancer, colorectal cancer and rectal cancer, non-small cell lung cancer, gastric adenocarcinoma, acute lymphocytic leukemia, acute myeloid leukemia, breast cancer, bone sarcoma, bladder cancer, anaplastic astrocytoma, head and neck cancer, pancreatic cancer, and esophageal cancer.

**19.** The composition for preventing or treating cancer according to claim 18, wherein the cancer is a brain tumor.

**20.** A method for producing reconstituted high-density lipoprotein (rHDL) nanoparticles comprising a phospholipid, cholesterol, and an apolipoprotein, wherein the method comprises:

a first step of injecting a phospholipid solution into a second inlet located in the center of a microfluidic device comprising three inlets and one outlet;
a second step of injecting an apolipoprotein into the first and third inlets located on either side, thereby producing nanoparticles; and
a third step of adding cholesterol dropwise to the produced nanoparticles.

**21.** The method for producing reconstituted high-density lipoprotein (rHDL) nanoparticles according to claim 20, wherein the microfluidic device has a micropillar structure.

**22.** The method for producing reconstituted high-density lipoprotein (rHDL) nanoparticles according to claim 20, wherein the method further comprises a step of removing an organic solvent after the second step.

**23.** The method for producing reconstituted high-density lipoprotein (rHDL) nanoparticles according to claim 20, wherein the cholesterol is 24-hydroxycholesterol.

**24.** The method for producing reconstituted high-density lipoprotein (rHDL) nanoparticles according to claim 20, wherein the apolipoprotein is apolipoprotein E.

**25.** The method for producing reconstituted high-density lipoprotein (rHDL) nanoparticles according to claim 24, wherein the apolipoprotein E is apolipoprotein E2, E3, or E2 and E3.

**26.** The method for producing reconstituted high-density lipoprotein (rHDL) nanoparticles according to claim 20, wherein the phospholipid is at least one selected from the group consisting of 1,2-dioleoyl-sn-glycero-3-phosphatidylcholine (DOPC), egg phosphatidylcholine (EPC), dilauroylphosphatidylcholine (DLPC), 1,2-dimyristoyl-sn-glycero-3-phosphocholine (DMPC), dipalmitoylphosphatidylcholine (DPPC), distearoylphosphatidylcholine (DSPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoyl phosphatidylcholine (SPPC), 1,2-diarachidoyl-sn-glycero-3-phosphocholine (DAPC), 1,2-dibehenoyl-sn-glycero-3-phosphocholine (DBPC), 1,2-dierucoyl-sn-glycero-3-phosphocholine (DEPC), palmitoyloleoylphosphatidylcholine (POPC), lysophosphatidylcholine, dilinoleoylphosphatidylcholine, distearoylphosphatidylethanolamine (DSPE), dimyristoylphosphatidylethanolamine (DMPE), dipalmitoylphosphatidylethanolamine (DPPE), palmitoyloleoylphosphatidylethanolamine (POPE), lysophosphatidylethanolamine, N1-[2-((1S)-1-[(3-aminopropyl)amino]-4-[di(3-aminopropyl)amino]butylcarboxamido)ethyl]-3,4-di[oleoyloxy]-benzamide) , dioctadecylamidoglycylspermine 4-trifluoroacetic acid (DOGS), 3β-[N-(N',N'-dimethylaminoethane)-carbamoyl]cholesterol (DC-Chol), 1,2-di-O-octadecenyl-3-trimethylammonium propane (DOTMA), 1,2-dioleoyl-3-trimethylammonium-propane (DOTAP), (1,2-dioleoyloxypropyl)-3-dimethylhydroxyethyl ammonium bromide (DORIE), 1,2-dimyristyloxy-propyl-3-dimethyl-hydroxyethyl ammonium bromide (DMRIE), 2,3-dioleoyloxy-N-[2-(sperminecarboxamido)ethyl]-N,N-dimethyl-1-propanaminium trifluoroacetate (DOSPA), N-(3-aminopropyl)-N,N-dimethyl-2,3-bis(dodecyloxy)-1-propaneammonium bromide (GAP-DLRIE), N-t-butyl-N'-tetradecyl-3-tetradecylaminopropionamidine (diC14-amidine), ethylphosphocholine (Ethyl PC), dimethyldioctadecylammonium bromide (DDAB), N4-cholesteryl-spermine (GL67), 1,2-dioleyloxy-3-dimethylaminopropane (DODMA), D-Lin-MC3-DMA (MC3, DLin-MC3-DMA), DLin-KC2-DMA, and DLin-DMA.

**27.** The method for producing reconstituted high-density lipoprotein (rHDL) nanoparticles according to claim 20, wherein the cholesterol is at least one selected from the group consisting of cholesterol, 7-ketocholesterol, 7α-hydroxycholesterol, 7β-hydroxycholesterol, 4β-hydroxycholesterol, α-5,6-epoxycholesterol, β-5,6-epoxycholesterol, 24S, 25-epoxycholesterol, 25-epoxycholesterol, 7-hydroxycholesterol, 20(S)-hydroxycholesterol, 22(S)-hydroxycholesterol, 22(R)-hydroxycholesterol, 24(S)-hydroxycholesterol, 24(R)-hydroxycholesterol, 25-hydroxycholesterol, 27-hydroxycholesterol, 5β-epoxycholesterol, 6β-epoxycholesterol, 7-dehydrocholesterol, (25R)26-hydroxycholesterol, 7α,(25R)26-dihydroxycholesterol, 5β-cholestane-3α,7α,12α,(25R)26-tetrol, 7α,25-dihydroxycholesterol, 7α,24S-dihydroxycholesterol, 3β,5α,6β-cholestane-3,5,6,26-tetrol, lanosterol, desmosterol, 7α-hydroxydesmosterol, (24Z),26S-hydroxydesmosterol, (24E),26S-hydroxydesmosterol, 24S,25-epoxylanosterol, 26(Z)-hydroxydesmosterol, 25-hydroxy-8-dehydrocholesterol, 24-hydroxy-8-dehydrocholesterol, 7-dehydrocholesterol, 8-dehydrocholesterol, (25R)26-hydroxy-8-dehydrocholesterol, 25-hydroxy-7-dehydrocholesterol, 4α-hydroxy-7-dehydrocholesterol, 4β-hydroxy-7-dehydrocholesterol, 7,8-epoxycholesterol, 3α,5β,6α-cholestane-3,6-diol, cholestane-3β, 5α, 6β-triol, Oxy34, Oxy49, and Oxy133.

**Figure 1**

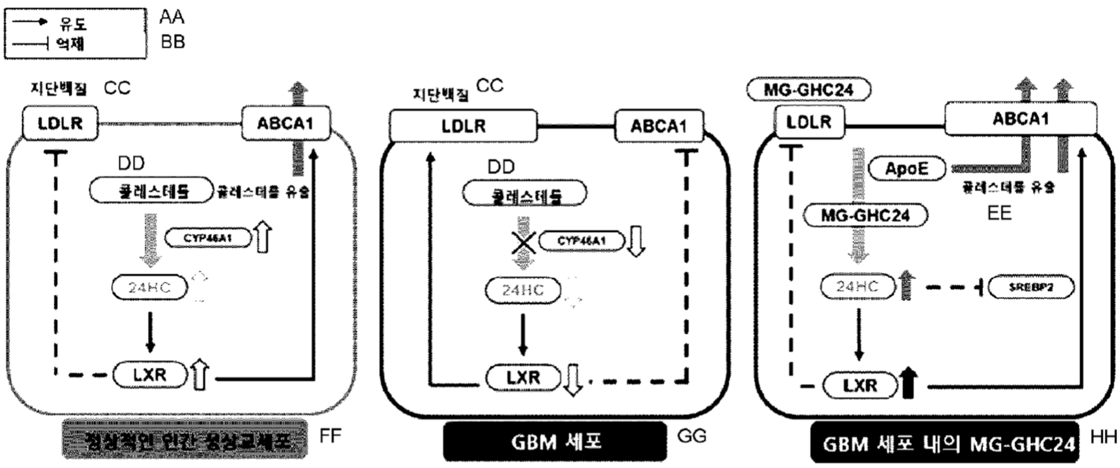


AA ... Induction
BB ... Inhibition
CC ... Lipoprotein
DD ... Cholesterol
EE ... Cholesterol efflux
FF ... Normal human astrocytes
GG ... GBM cells
HH ... MG-GHC24 in GBM cells

Figure 2

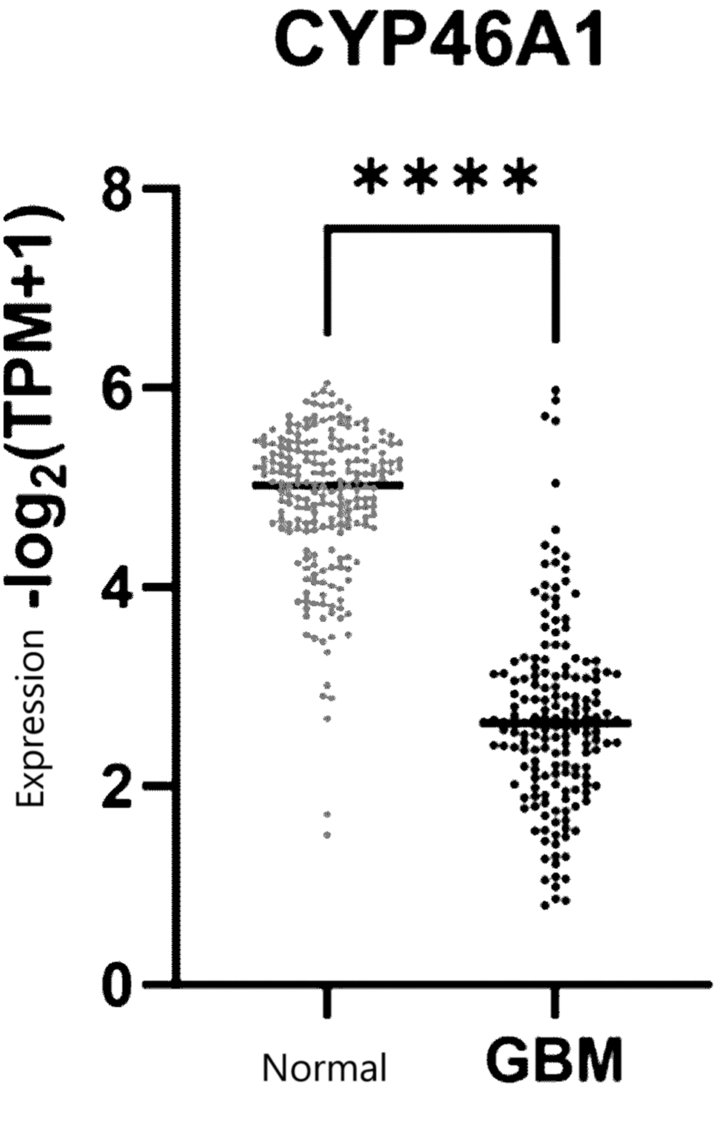

Figure 3

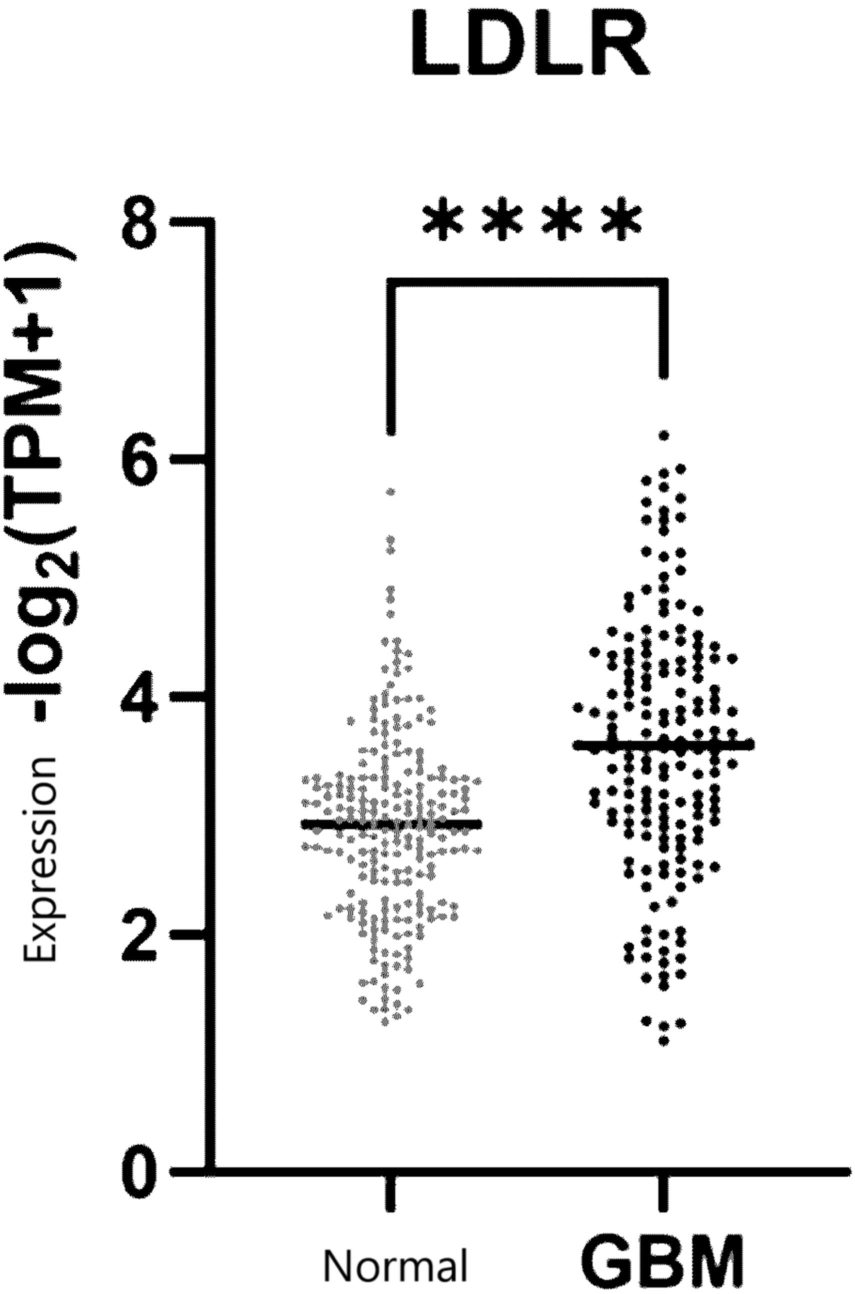

Figure 4

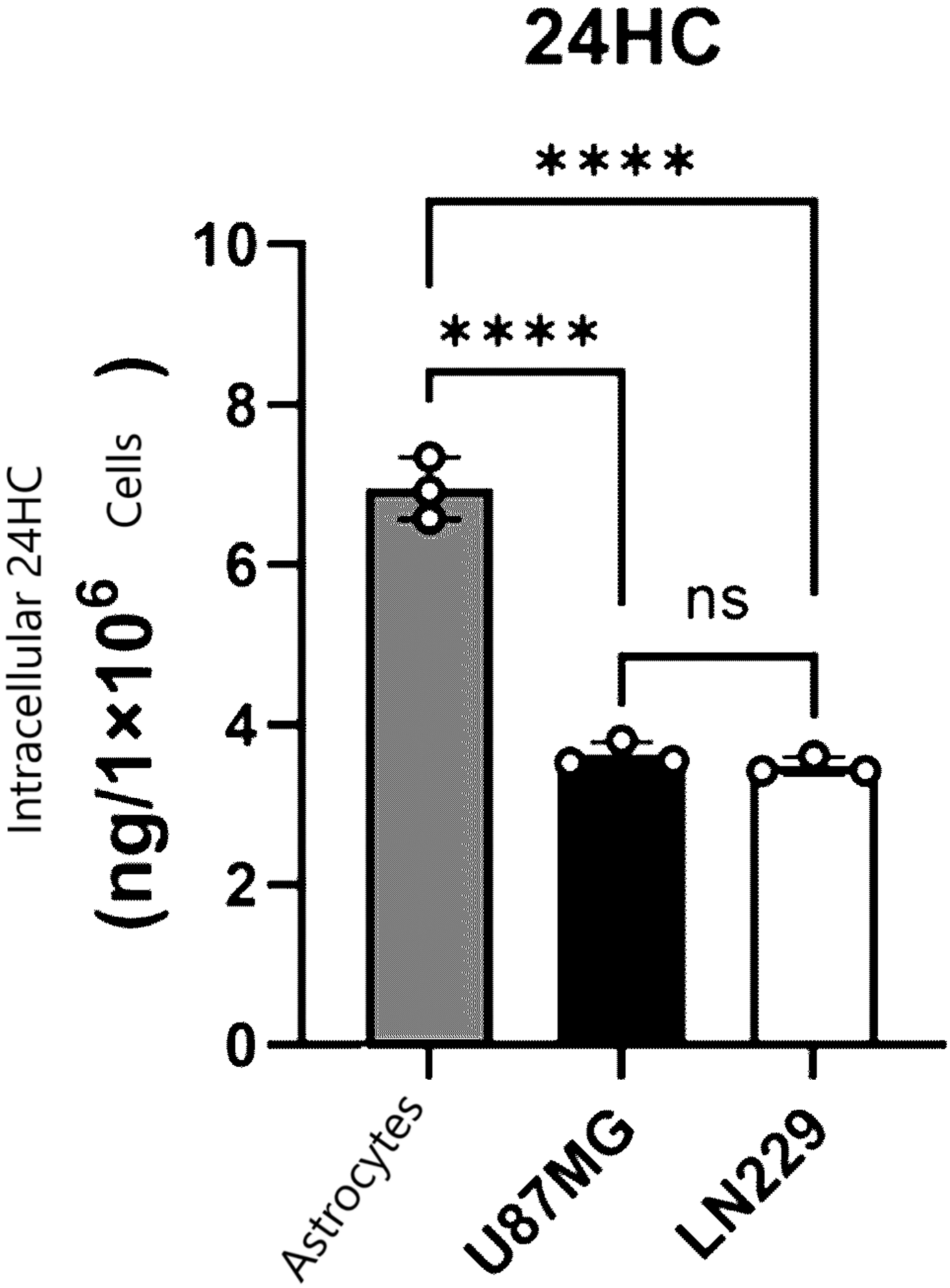
24HC
****
****
ns
10
8
6
4
2
0
Intracellular 24HC
(ng/1×10^6 Cells )
Astrocytes
U87MG
LN229

Figure 5

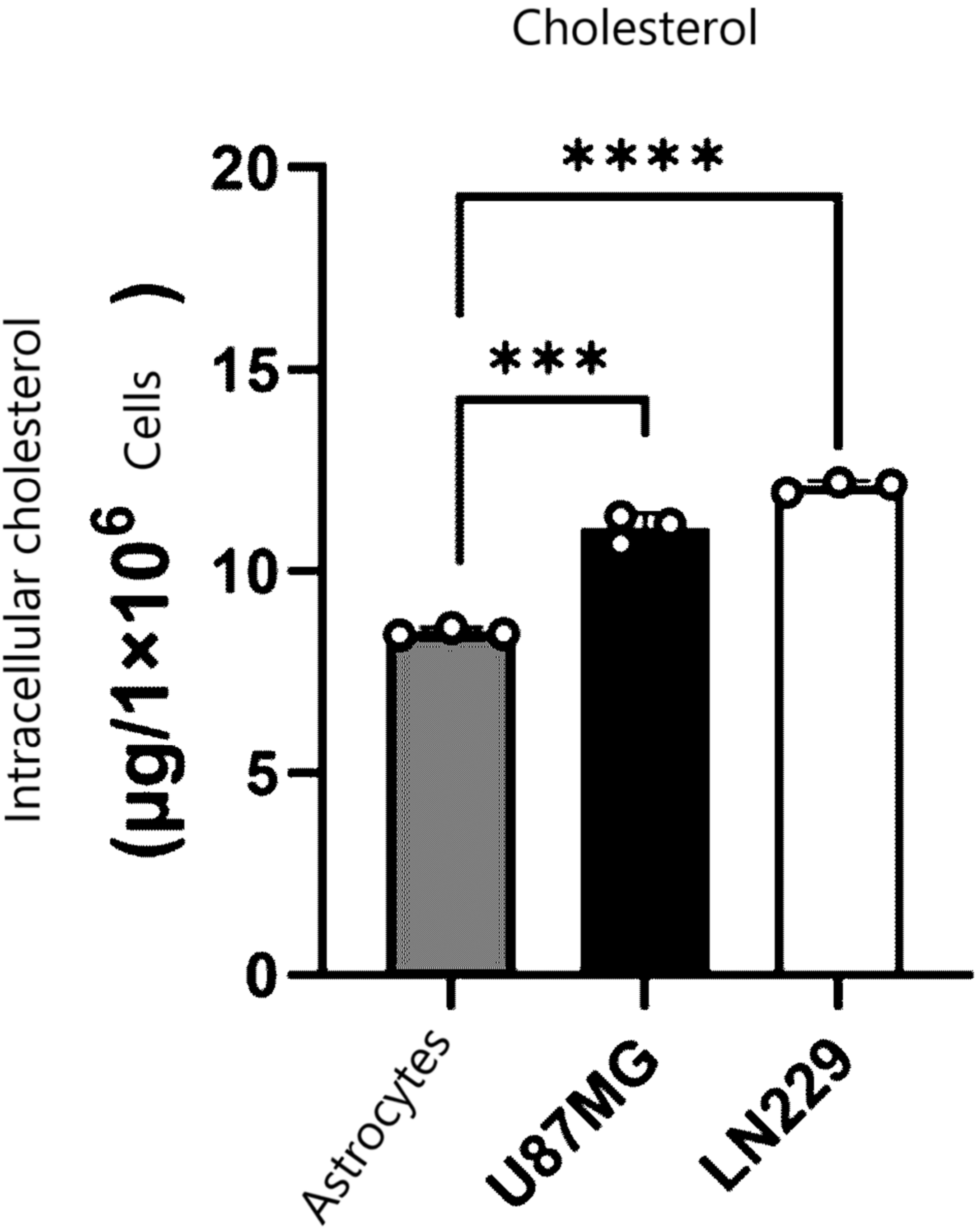
Cholesterol
Intracellular cholesterol
(μg/1×10^6 Cells)
20
15
10
5
0
****
***
Astrocytes
U87MG
LN229

**Figure 6**

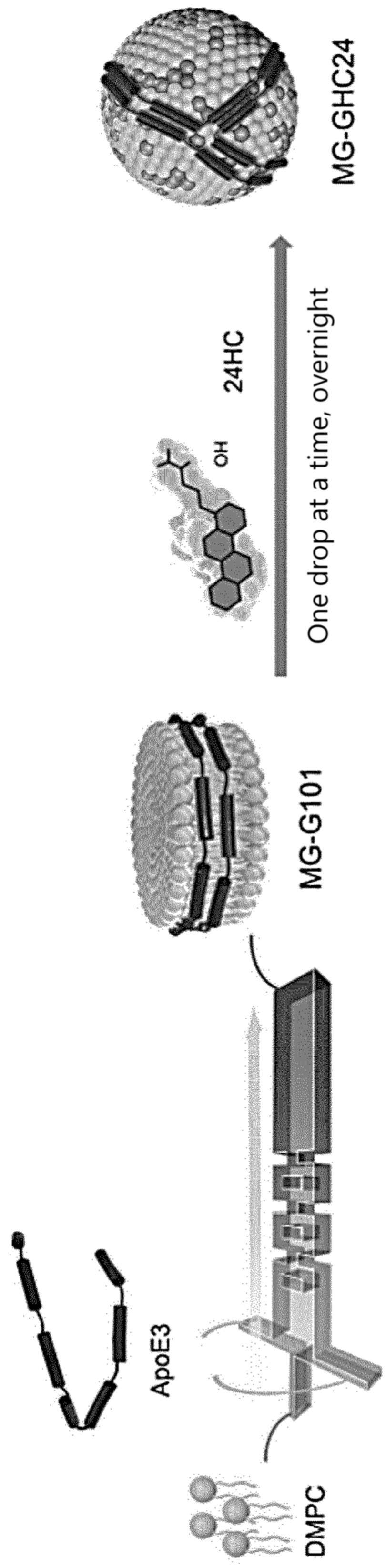

Figure 7

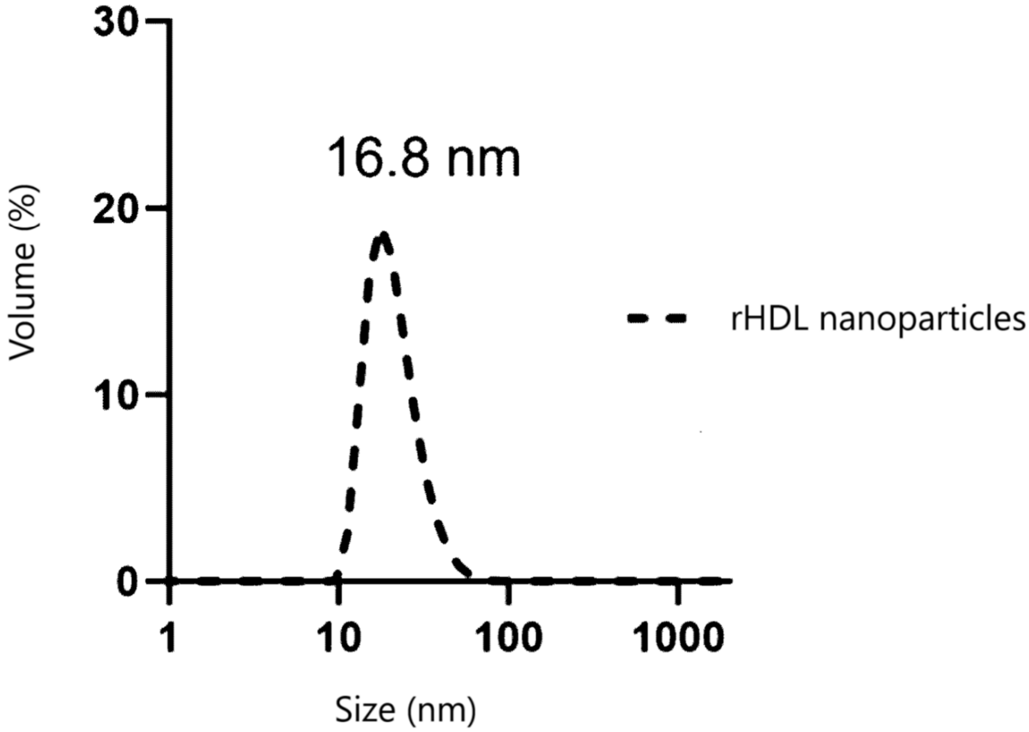
30
20
10
0
Volume (%)
16.8 nm
rHDL nanoparticles
1
10
100
1000
Size (nm)

Figure 8

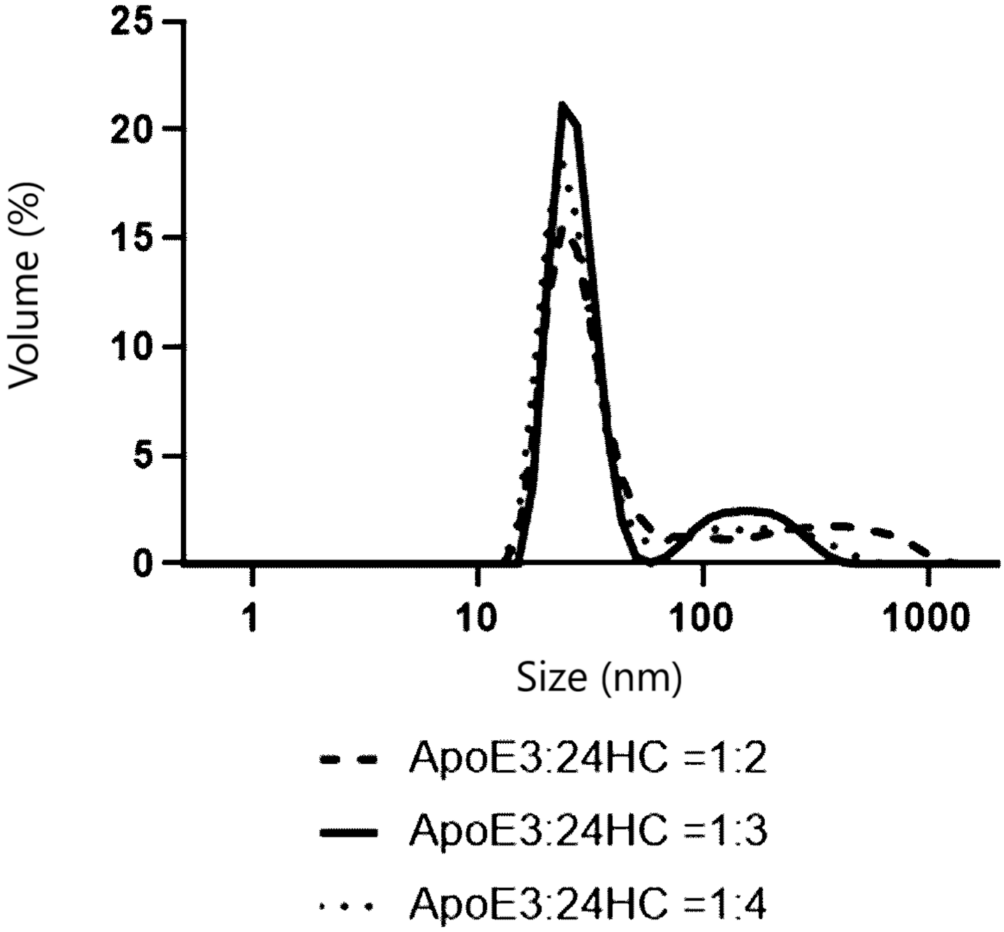
25
20
15
10
5
0
Volume (%)
1
10
100
1000
Size (nm)
ApoE3:24HC =1:2
ApoE3:24HC =1:3
ApoE3:24HC =1:4

**Figure 9**

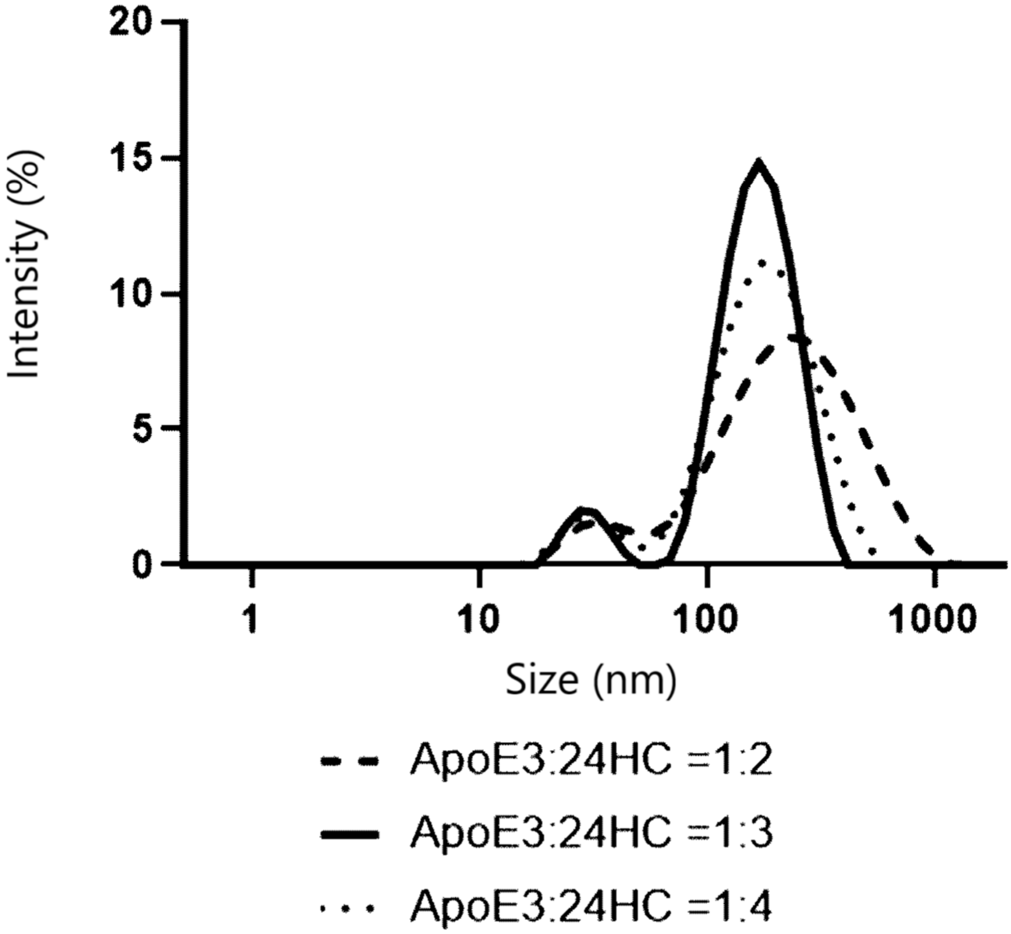
20
15
10
5
0
Intensity (%)
1
10
100
1000
Size (nm)
ApoE3:24HC =1:2
ApoE3:24HC =1:3
ApoE3:24HC =1:4

**Figure 10**

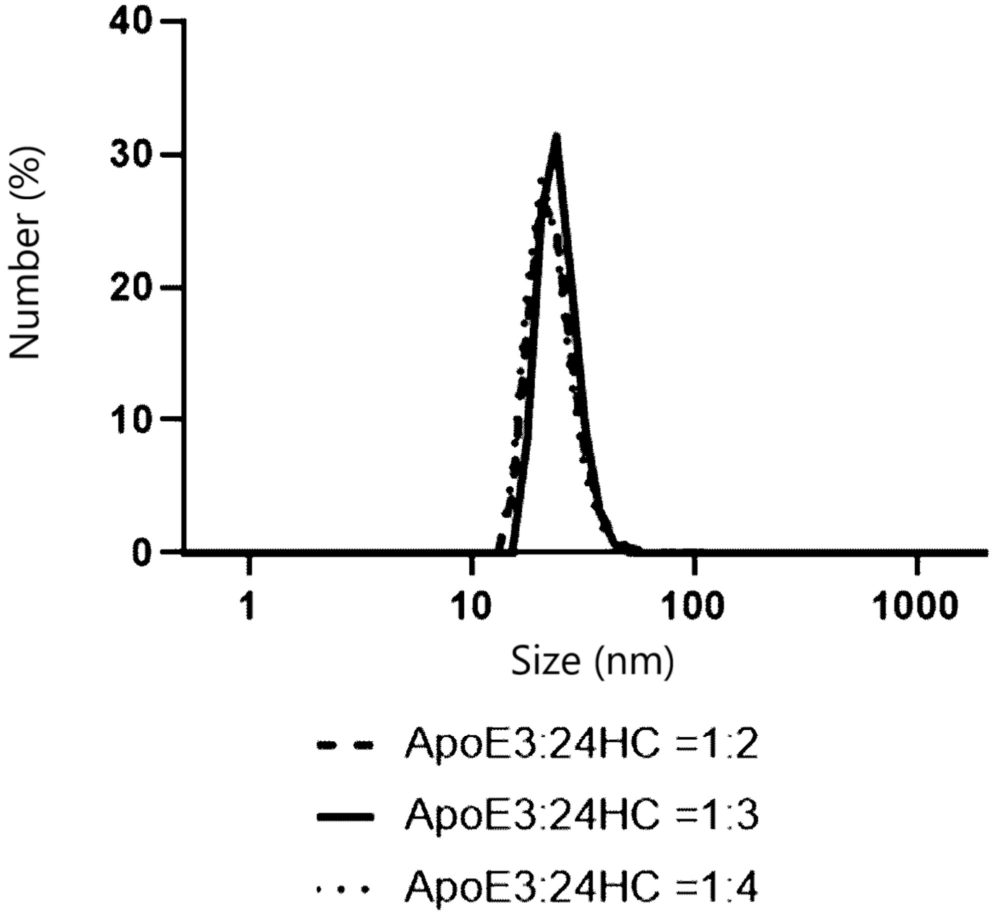
40
30
20
10
0
Number (%)
1
10
100
1000
Size (nm)
ApoE3:24HC =1:2
ApoE3:24HC =1:3
ApoE3:24HC =1:4

Figure 11

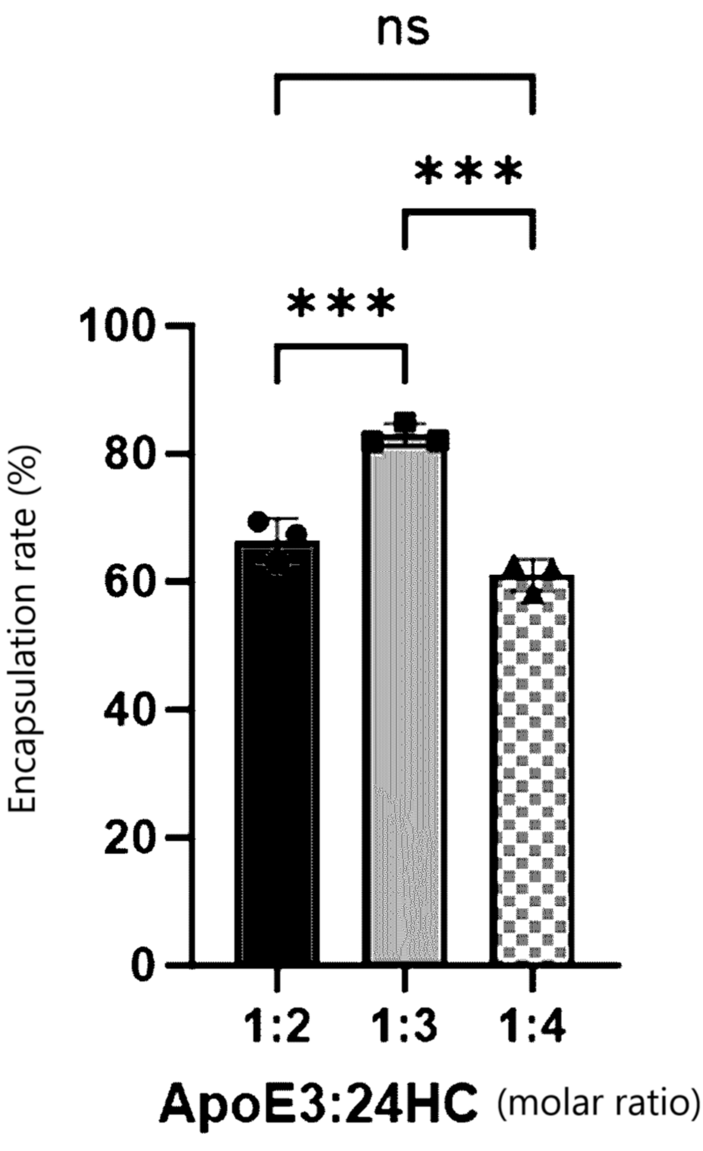
ns
***
***
100
80
60
40
20
0
Encapsulation rate (%)
1:2
1:3
1:4
ApoE3:24HC (molar ratio)

Figure 12

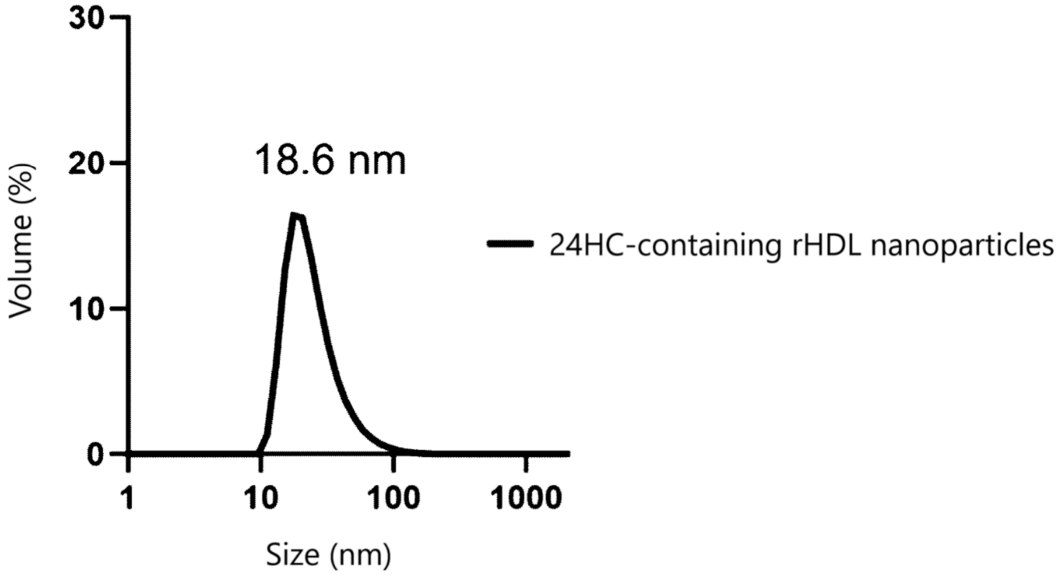


Figure 13

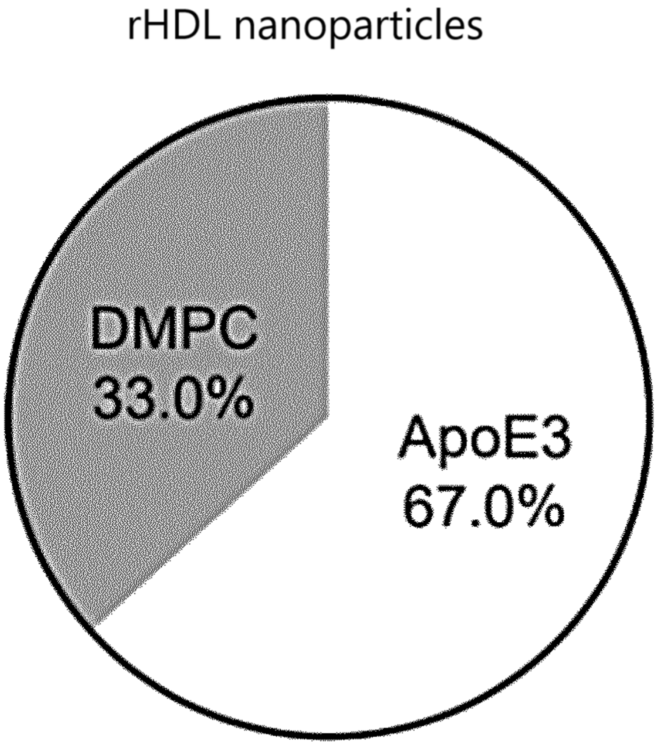

Figure 14

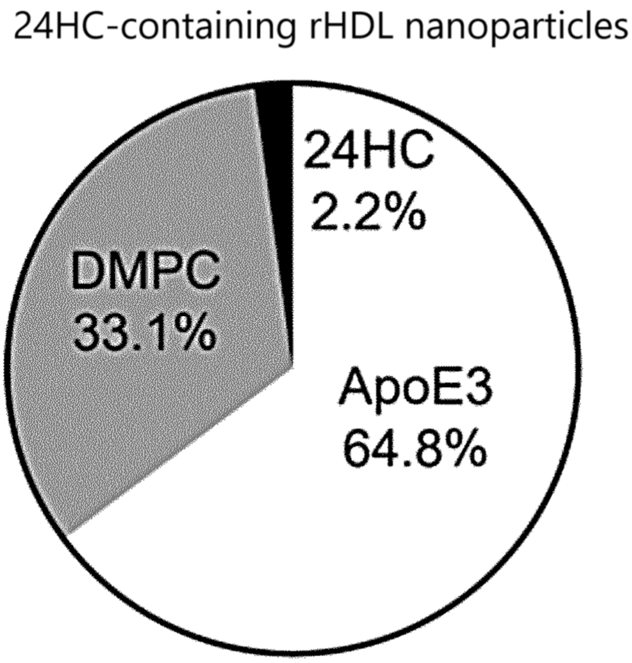
24HC-containing rHDL nanoparticles
24HC
2.2%
DMPC
33.1%
ApoE3
64.8%

Figure 15

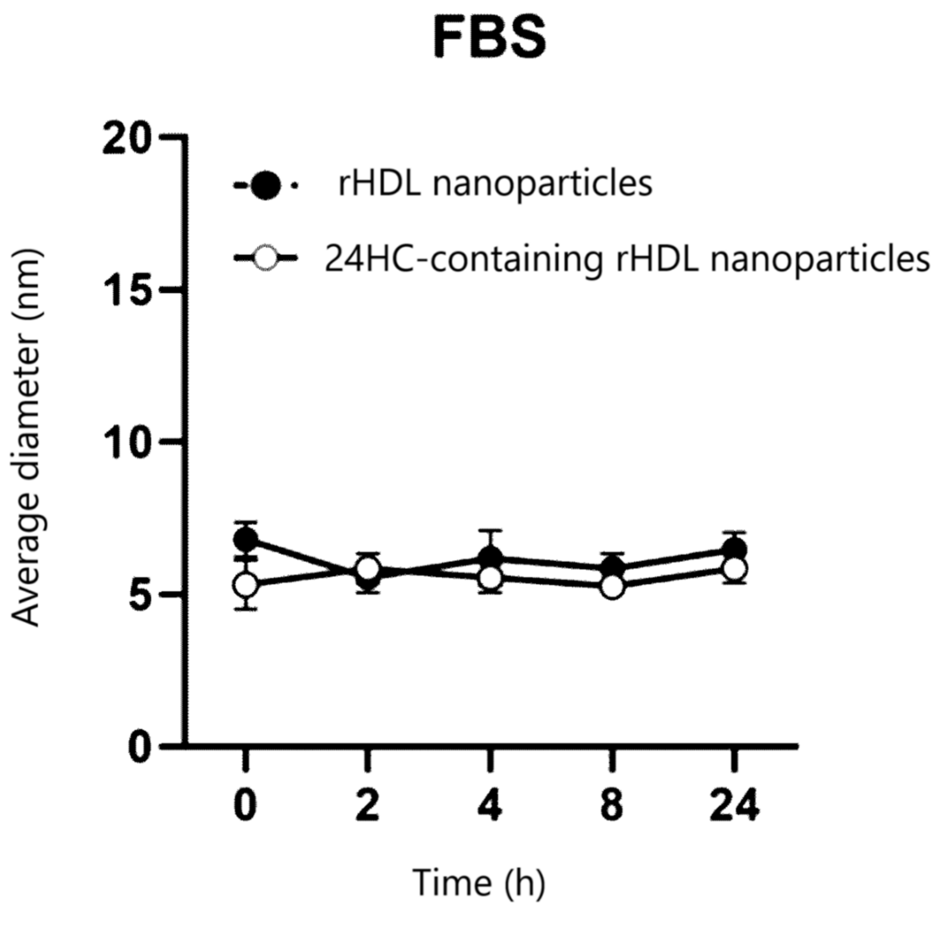
FBS
20
15
10
5
0
rHDL nanoparticles
24HC-containing rHDL nanoparticles
Average diameter (nm)
0
2
4
8
24
Time (h)

Figure 16

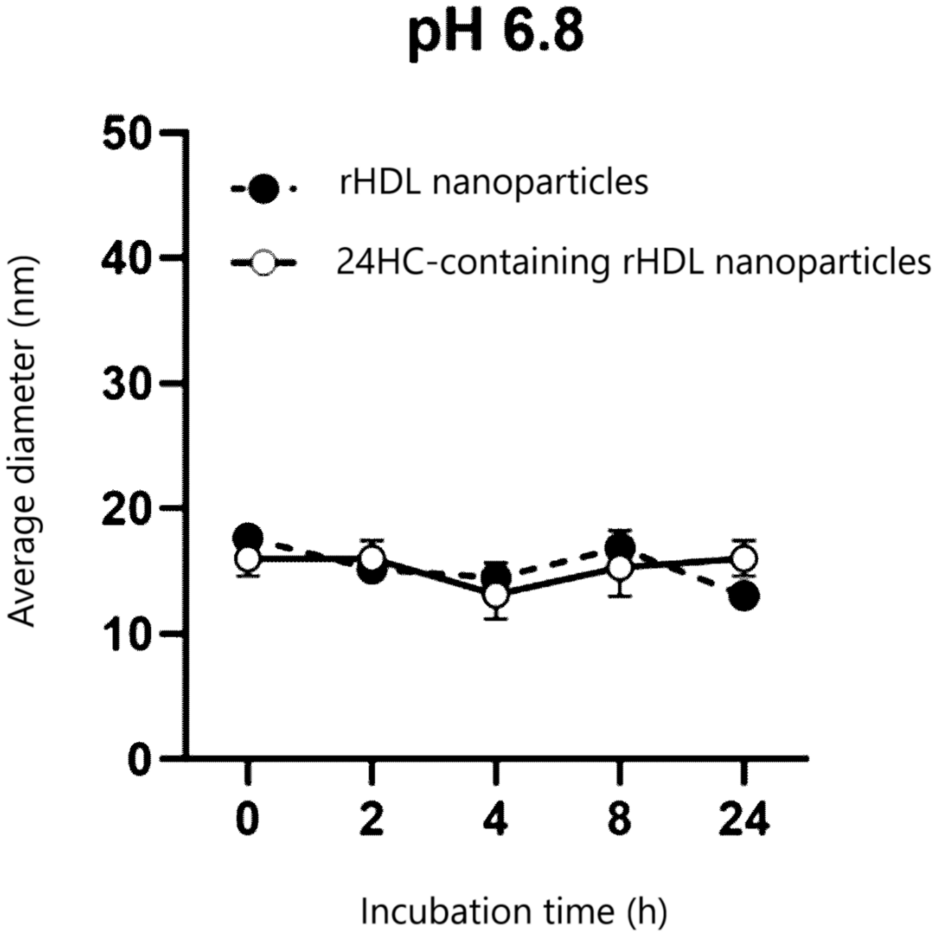
pH 6.8
50
40
30
20
10
0
Average diameter (nm)
rHDL nanoparticles
24HC-containing rHDL nanoparticles
0
2
4
8
24
Incubation time (h)

Figure 17

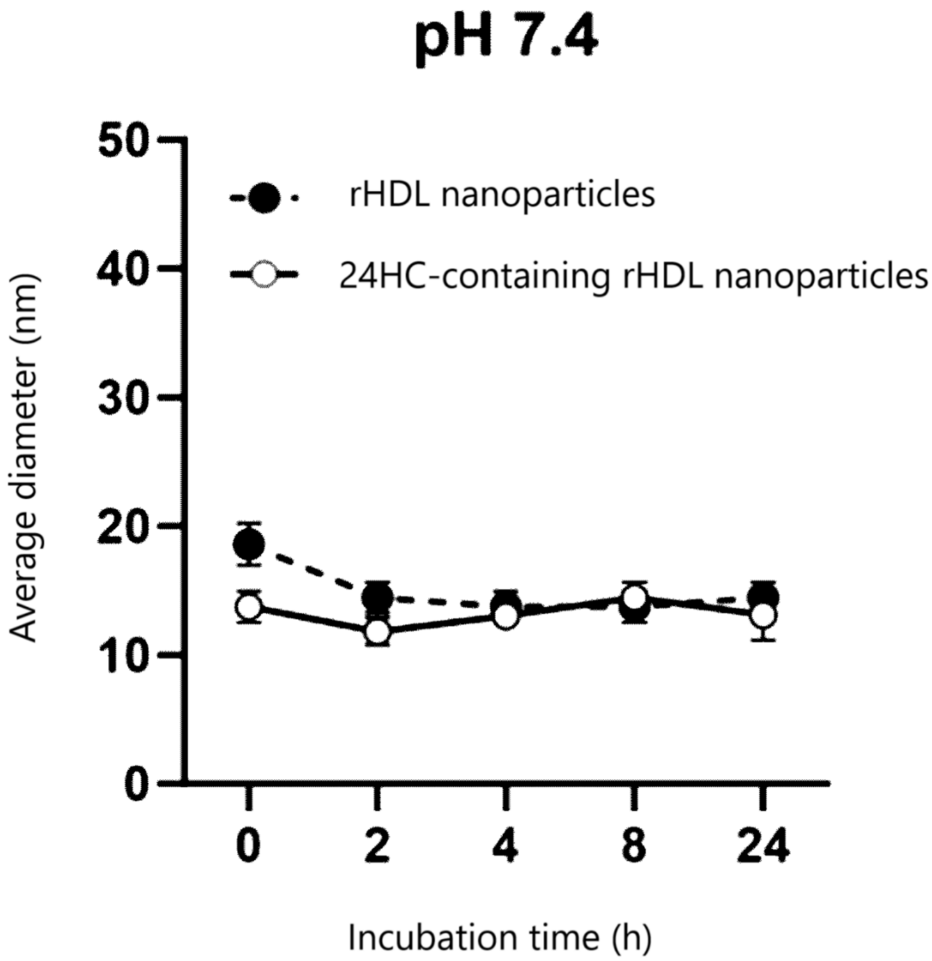
pH 7.4
50
40
30
20
10
0
Average diameter (nm)
rHDL nanoparticles
24HC-containing rHDL nanoparticles
0
2
4
8
24
Incubation time (h)

Figure 18

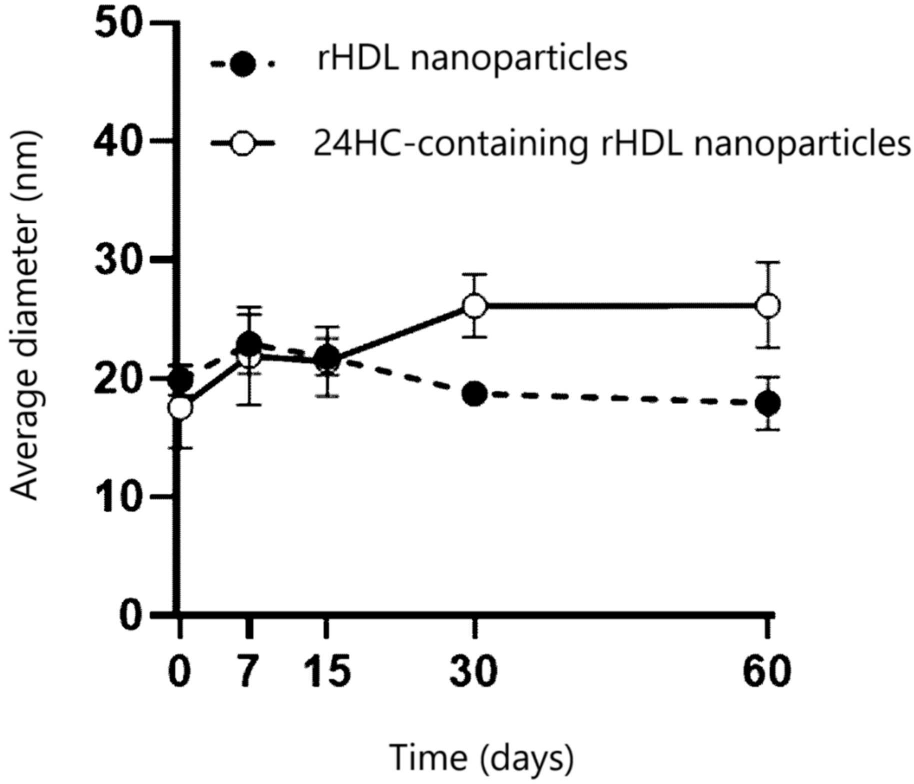
rHDL nanoparticles
24HC-containing rHDL nanoparticles
Average diameter (nm)
50
40
30
20
10
0
0
7
15
30
60
Time (days)

Figure 19

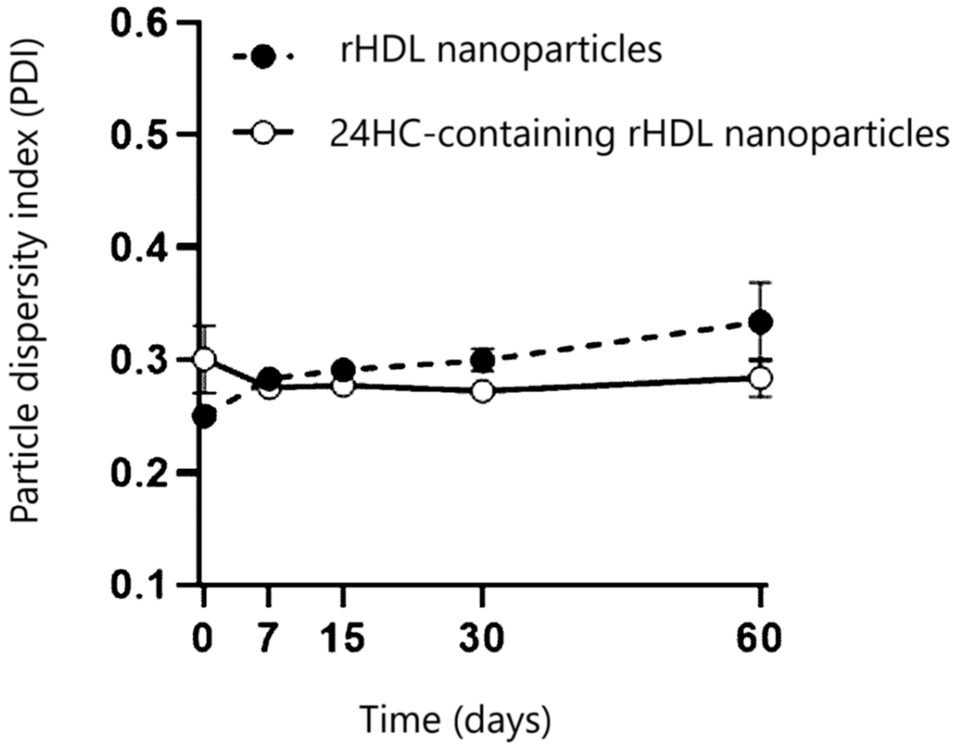
Particle dispersity index (PDI)
0.6
0.5
0.4
0.3
0.2
0.1
rHDL nanoparticles
24HC-containing rHDL nanoparticles
0
7
15
30
60
Time (days)

Figure 20

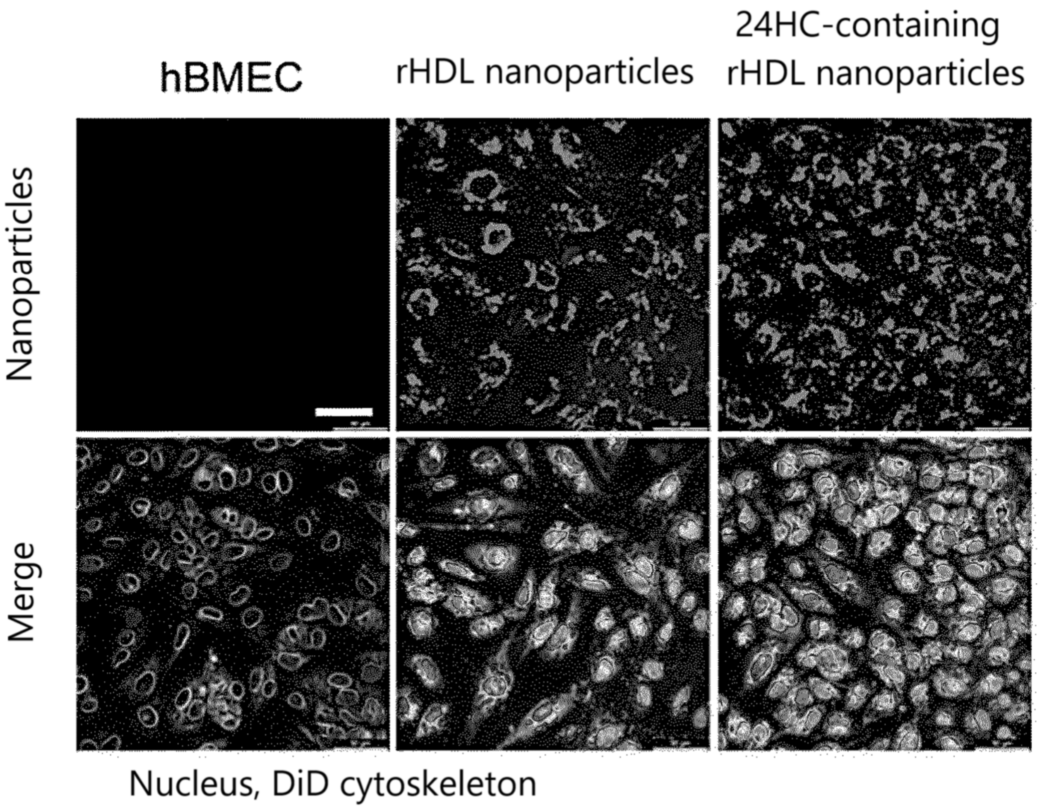
hBMEC
rHDL nanoparticles
24HC-containing rHDL nanoparticles
Nanoparticles
Merge
Nucleus, DiD cytoskeleton

Figure 21

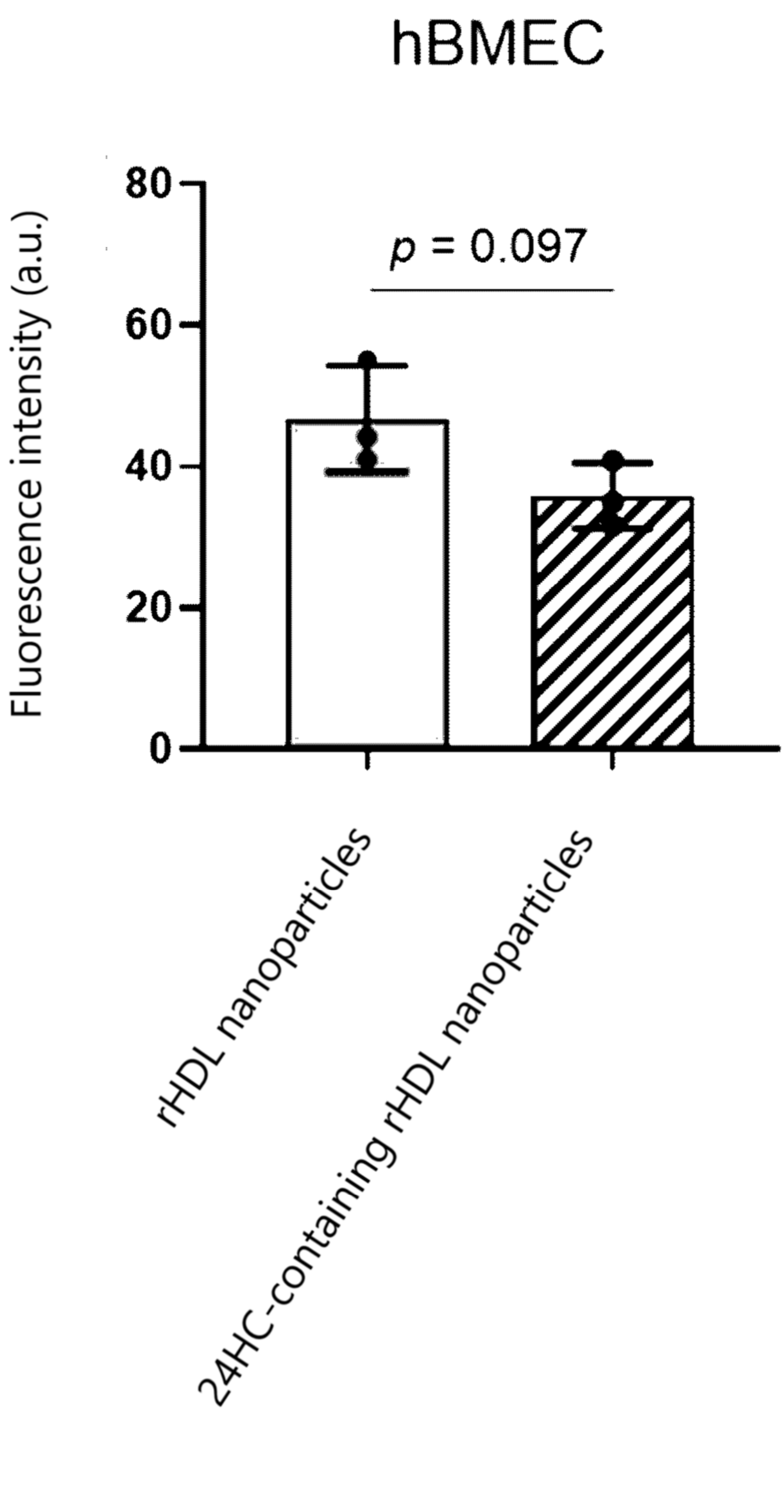
hBMEC
Fluorescence intensity (a.u.)
80
60
40
20
0
p = 0.097
rHDL nanoparticles
24HC-containing rHDL nanoparticles

**Figure 22**

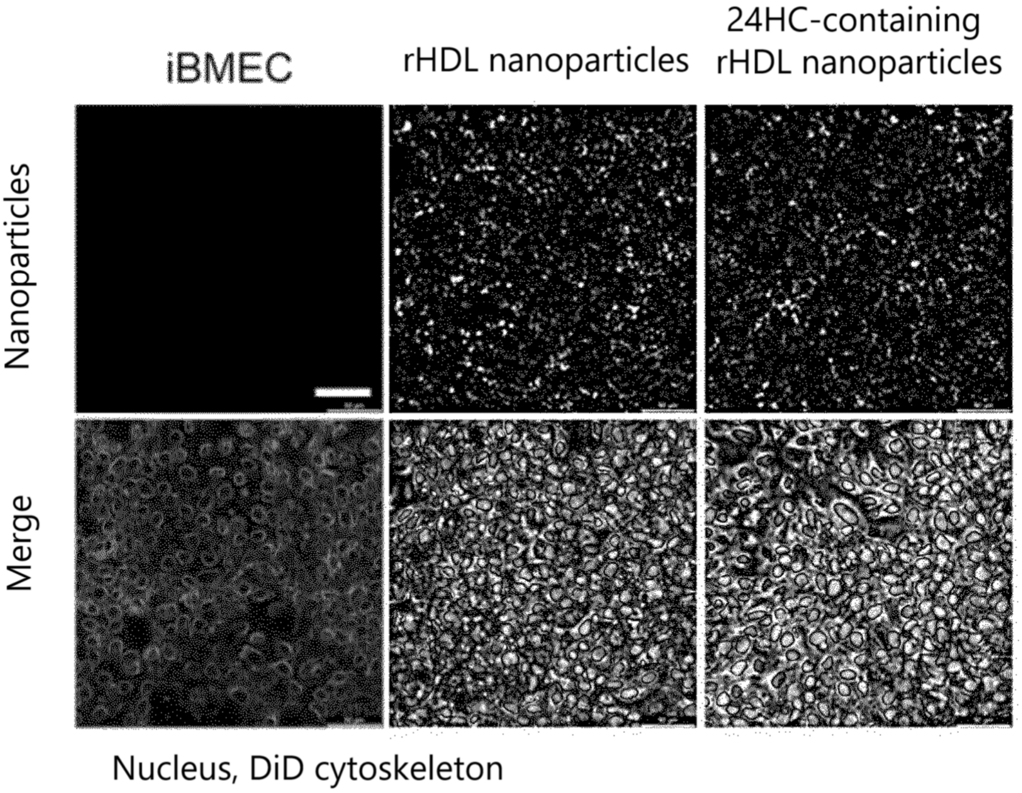
iBMEC
rHDL nanoparticles
24HC-containing
rHDL nanoparticles
Nanoparticles
Merge
Nucleus, DiD cytoskeleton

Figure 23

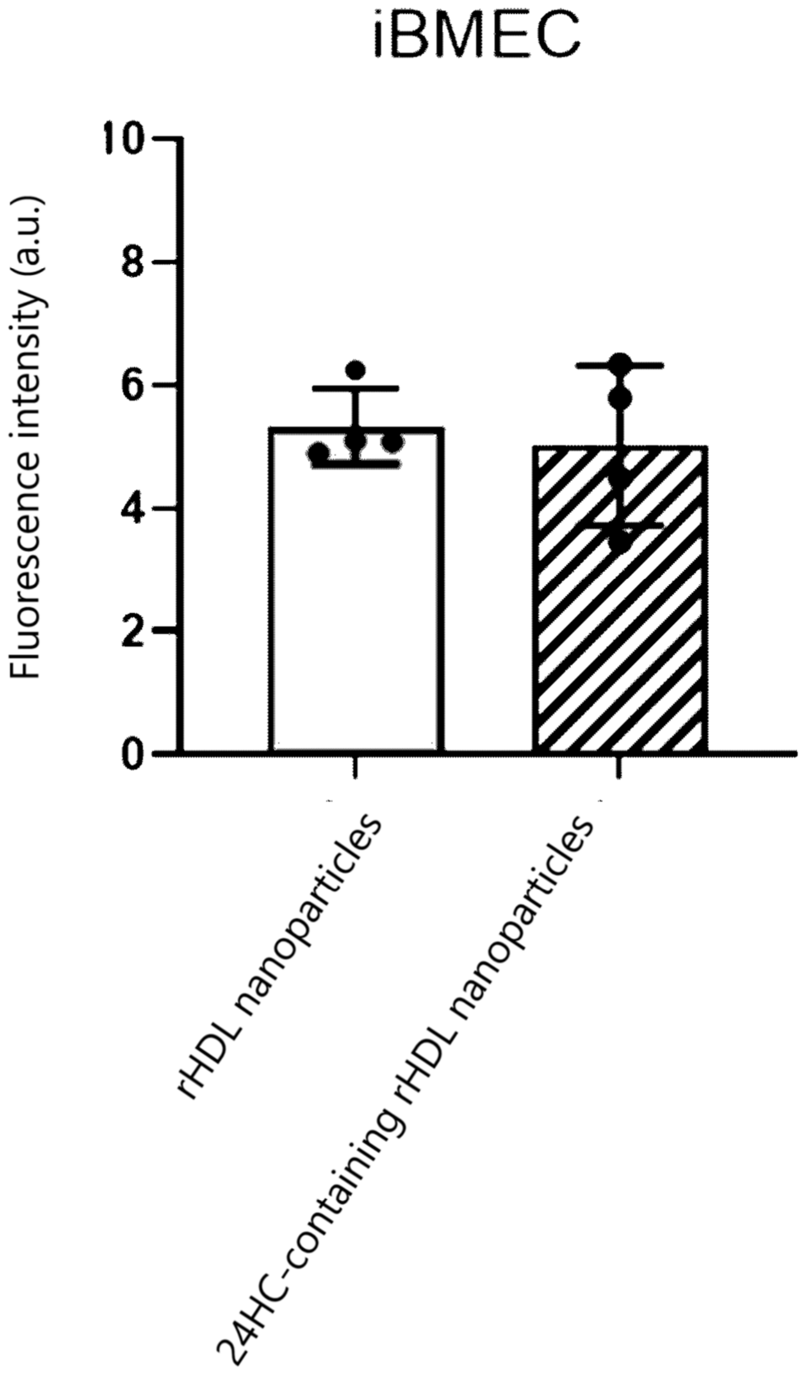
iBMEC
Fluorescence intensity (a.u.)
10
8
6
4
2
0
rHDL nanoparticles
24HC-containing rHDL nanoparticles

Figure 24

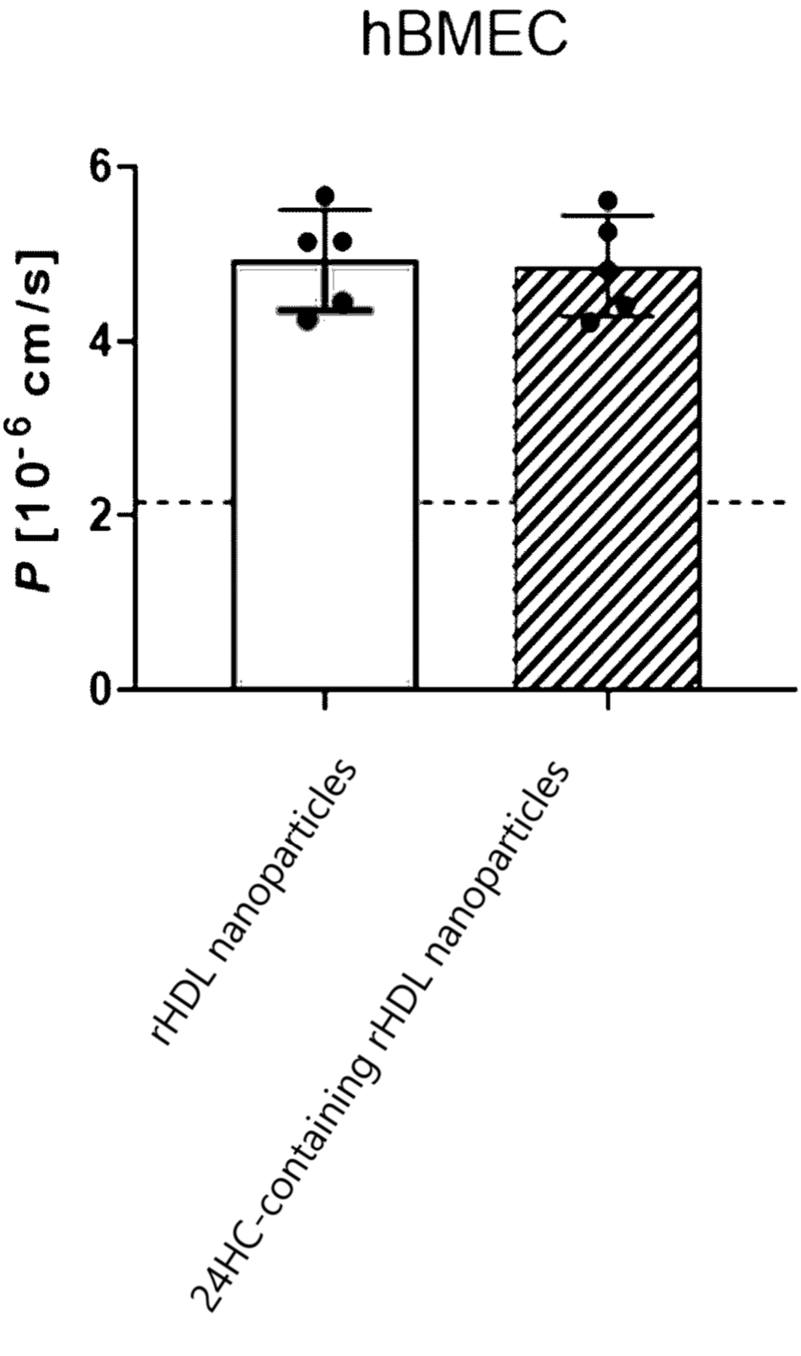
hBMEC
P [10⁻⁶ cm/s]
6
4
2
0
rHDL nanoparticles
24HC-containing rHDL nanoparticles

Figure 25

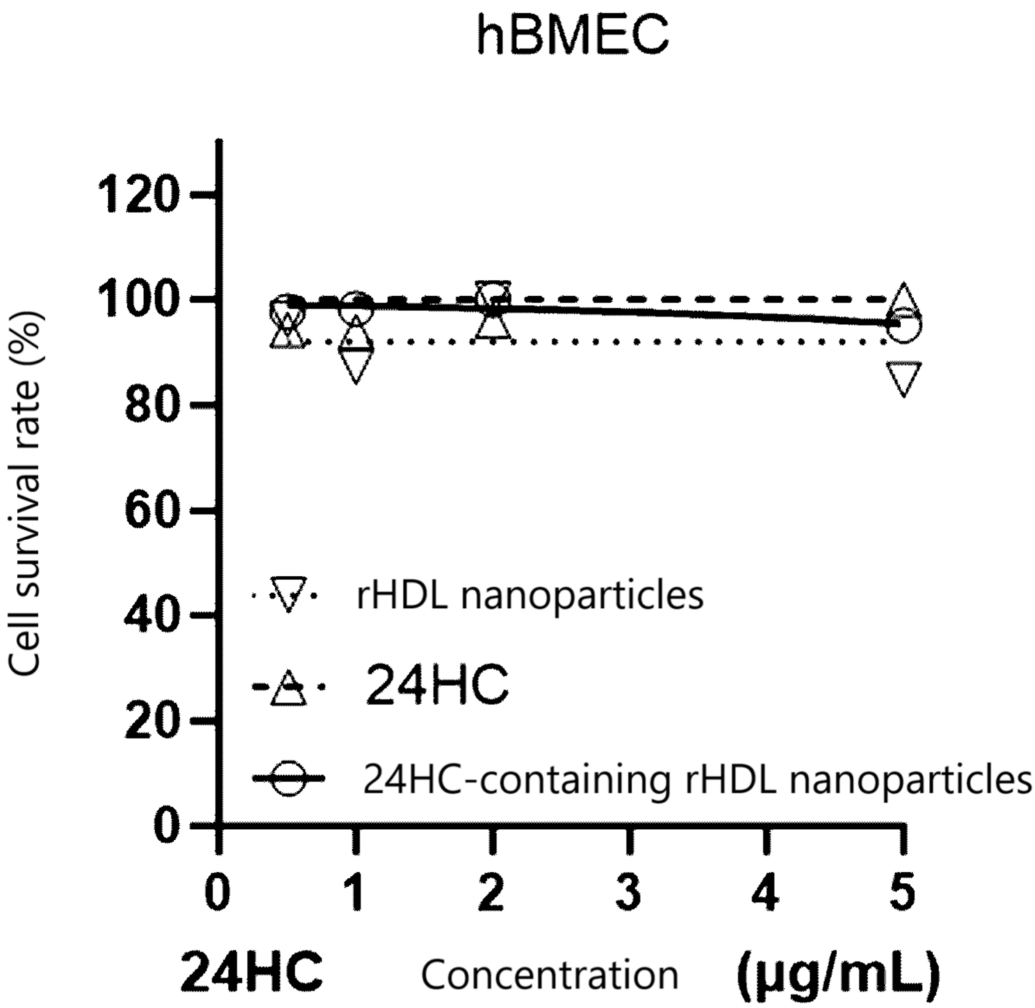
hBMEC
Cell survival rate (%)
120
100
80
60
40
20
0
0
1
2
3
4
5
rHDL nanoparticles
24HC
24HC-containing rHDL nanoparticles
24HC Concentration (µg/mL)

Figure 26

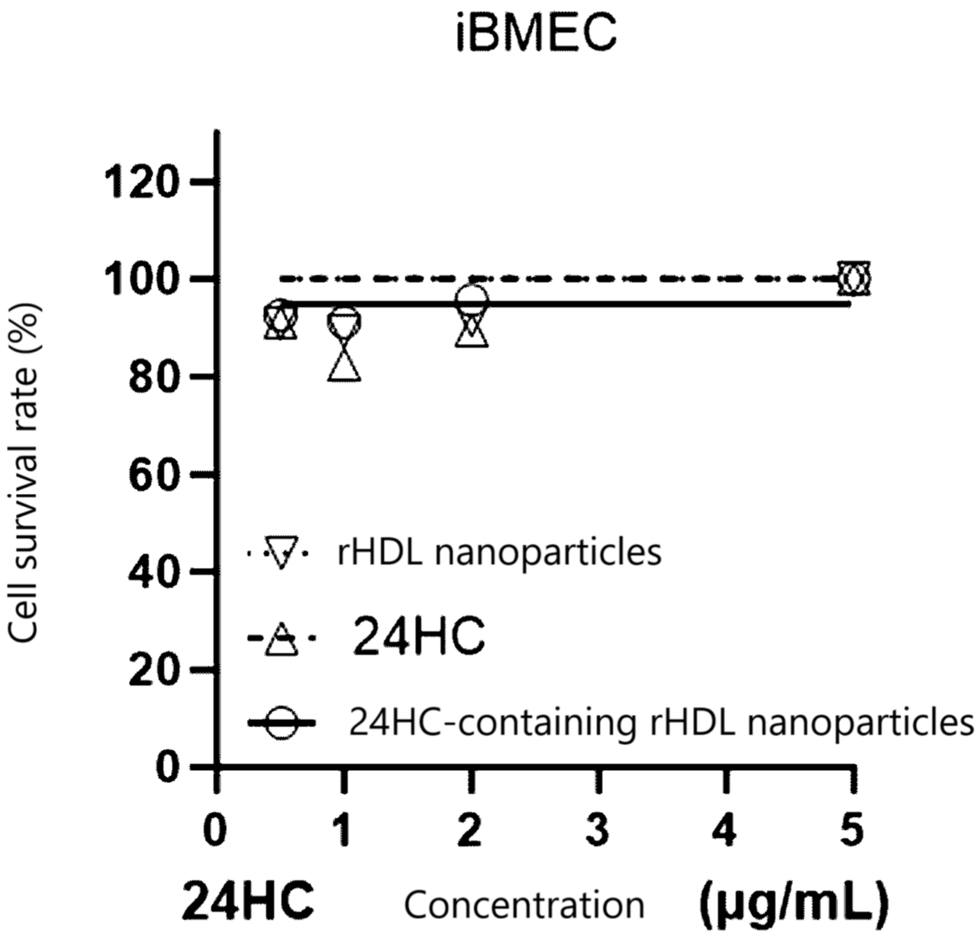
iBMEC
Cell survival rate (%)
120
100
80
60
40
20
0
rHDL nanoparticles
24HC
24HC-containing rHDL nanoparticles
0
1
2
3
4
5
24HC Concentration (μg/mL)

Figure 27

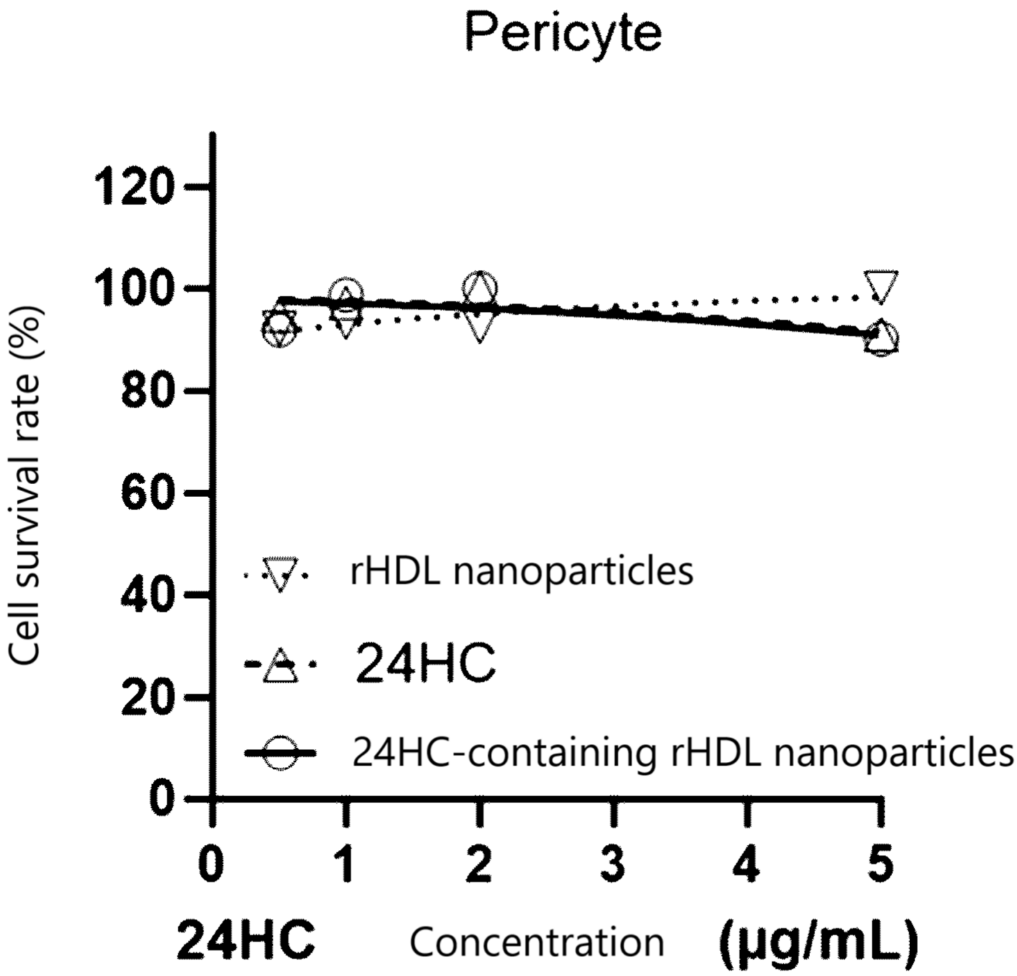
Pericyte
Cell survival rate (%)
120
100
80
60
40
20
0
rHDL nanoparticles
24HC
24HC-containing rHDL nanoparticles
0
1
2
3
4
5
24HC
Concentration
(µg/mL)

Figure 28

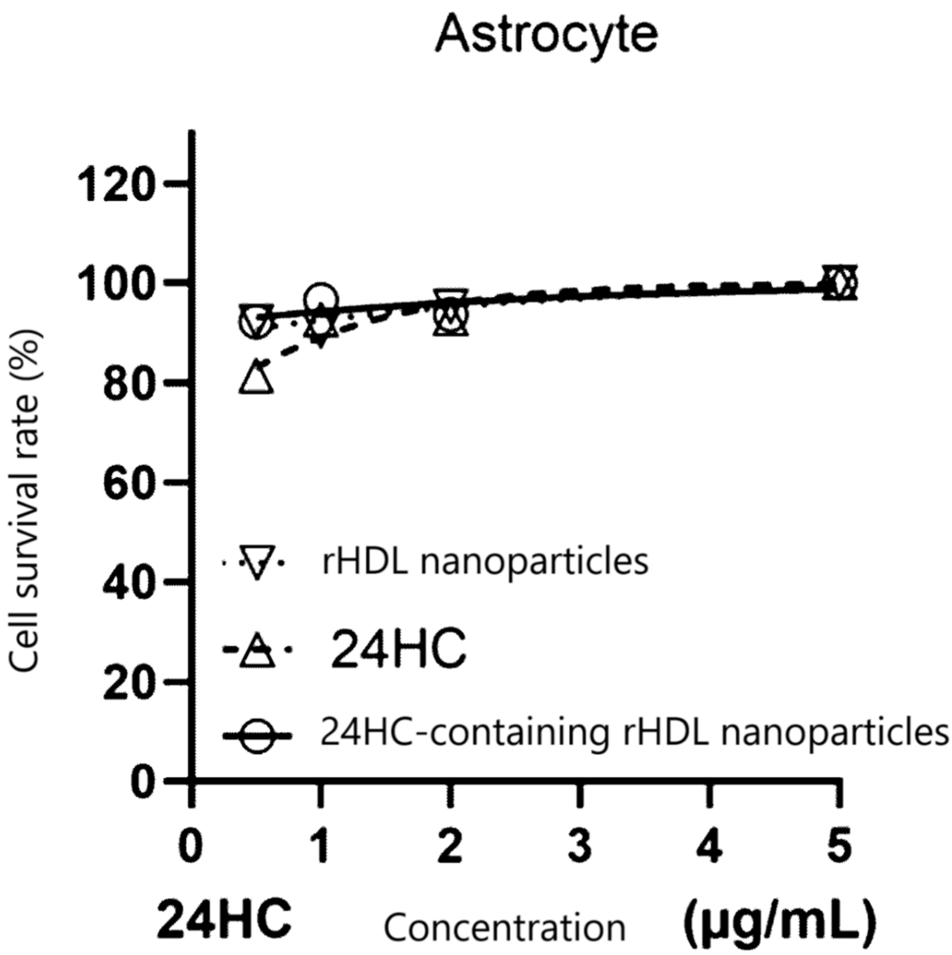
Astrocyte
Cell survival rate (%)
120
100
80
60
40
20
0
rHDL nanoparticles
24HC
24HC-containing rHDL nanoparticles
0
1
2
3
4
5
24HC Concentration (µg/mL)

**Figure 29**

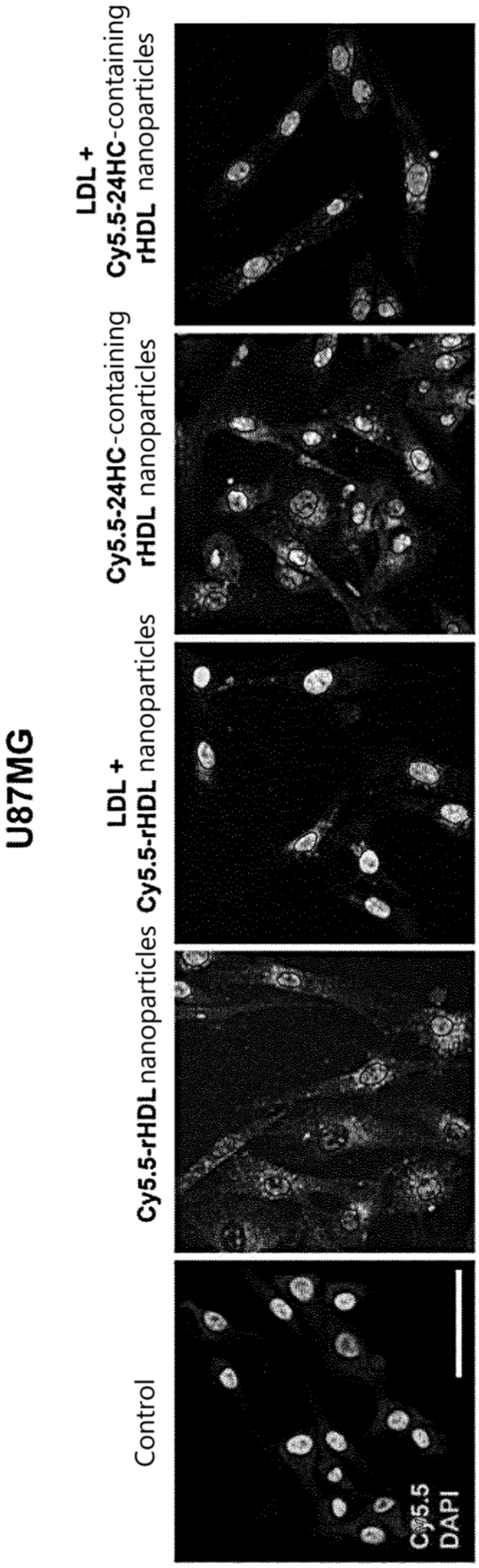
U87MG
Control
Cy5.5
DAPI
Cy5.5-rHDL nanoparticles
LDL +
Cy5.5-rHDL nanoparticles
Cy5.5-24HC-containing
rHDL nanoparticles
LDL +
Cy5.5-24HC-containing
rHDL nanoparticles

Figure 30

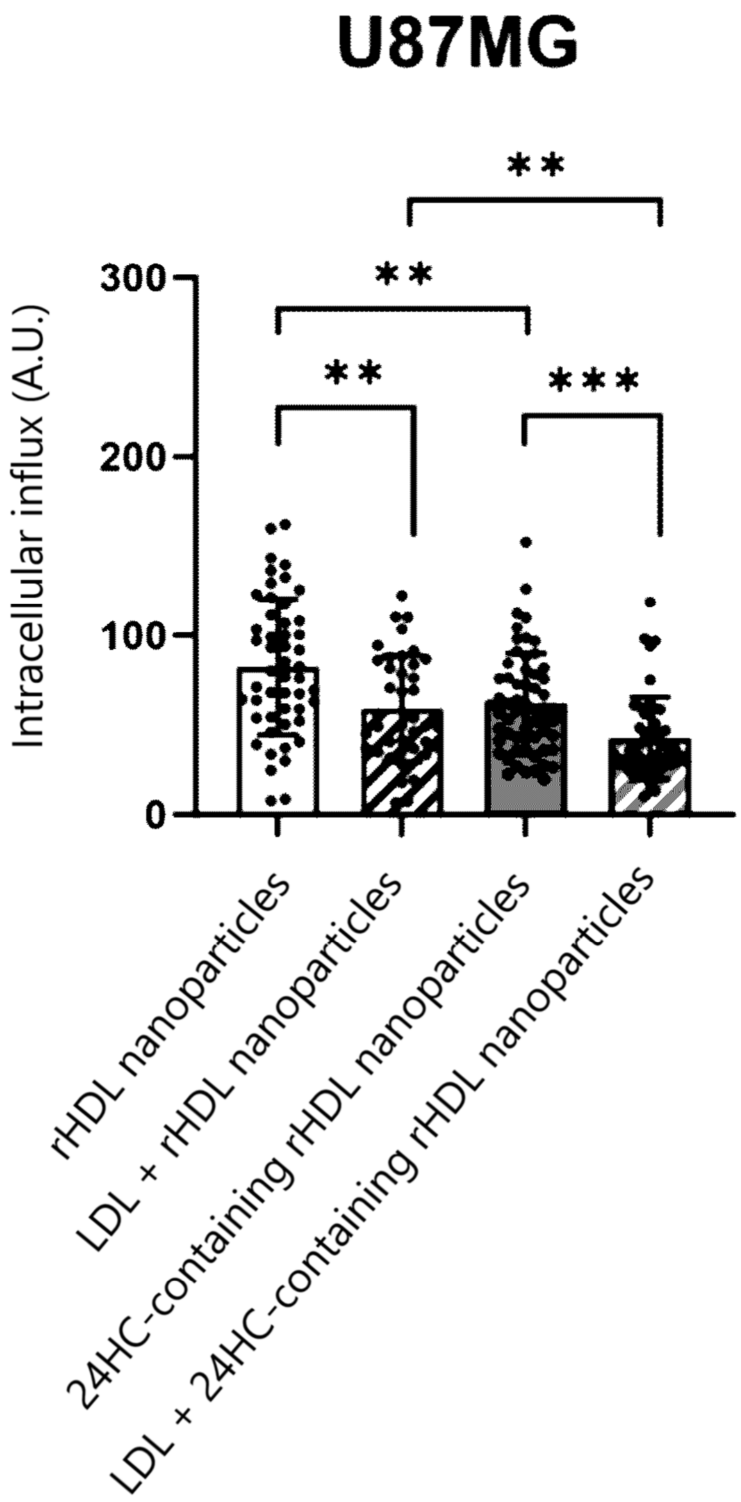
U87MG
**
**
**
***
300
200
100
0
Intracellular influx (A.U.)
rHDL nanoparticles
LDL + rHDL nanoparticles
24HC-containing rHDL nanoparticles
LDL + 24HC-containing rHDL nanoparticles

**Figure 31**

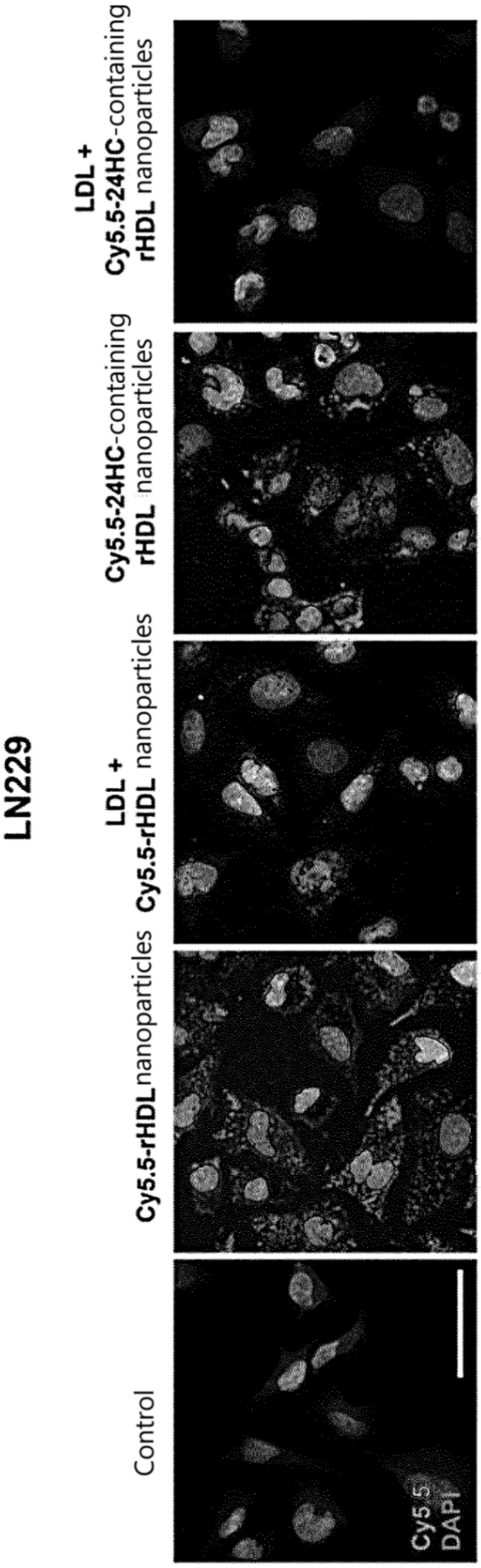
LN229
Control
Cy5.5
DAPI
Cy5.5-rHDL nanoparticles
LDL +
Cy5.5-rHDL nanoparticles
Cy5.5-24HC-containing
rHDL nanoparticles
LDL +
Cy5.5-24HC-containing
rHDL nanoparticles

**Figure 32**

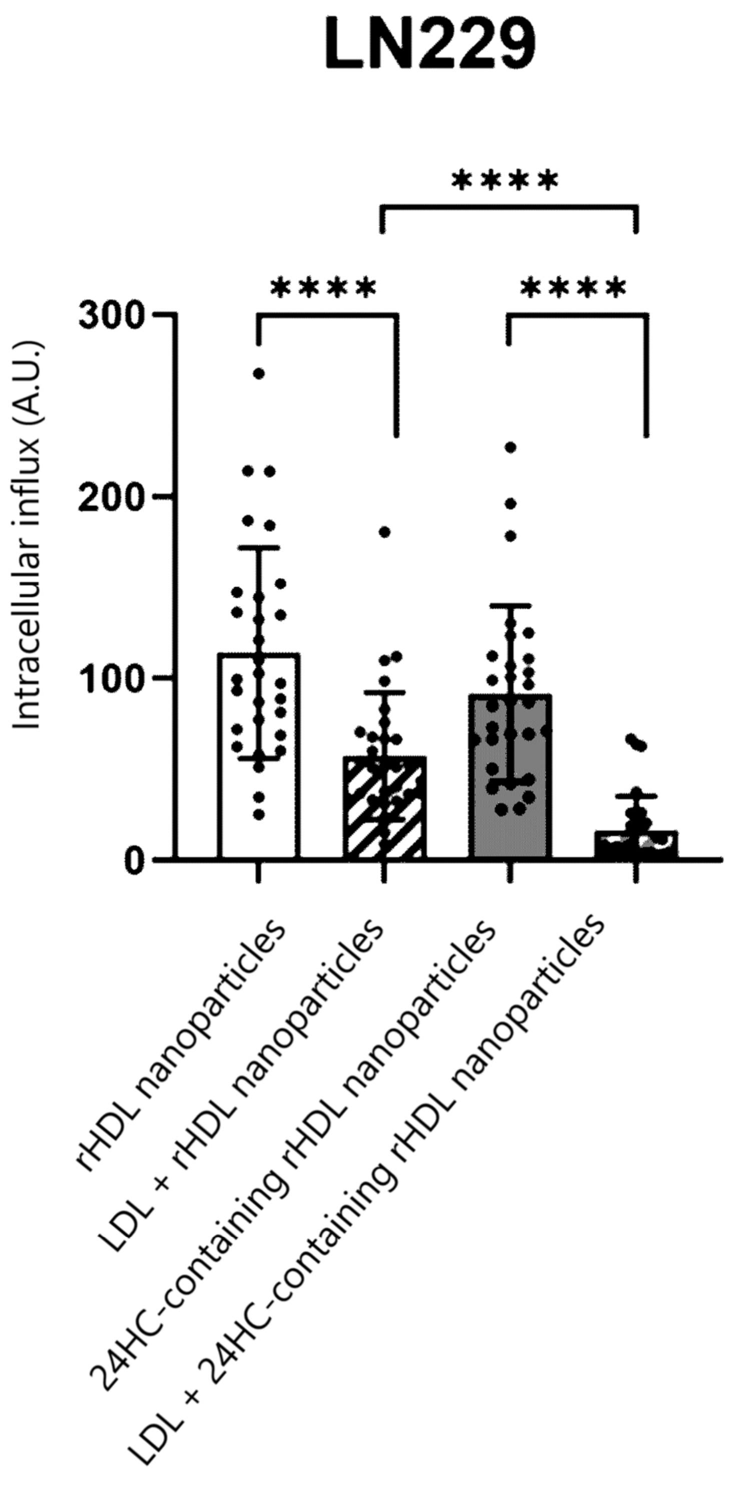
LN229
****
****
****
300
200
100
0
Intracellular influx (A.U.)
rHDL nanoparticles
LDL + rHDL nanoparticles
24HC-containing rHDL nanoparticles
LDL + 24HC-containing rHDL nanoparticles

**Figure 33**

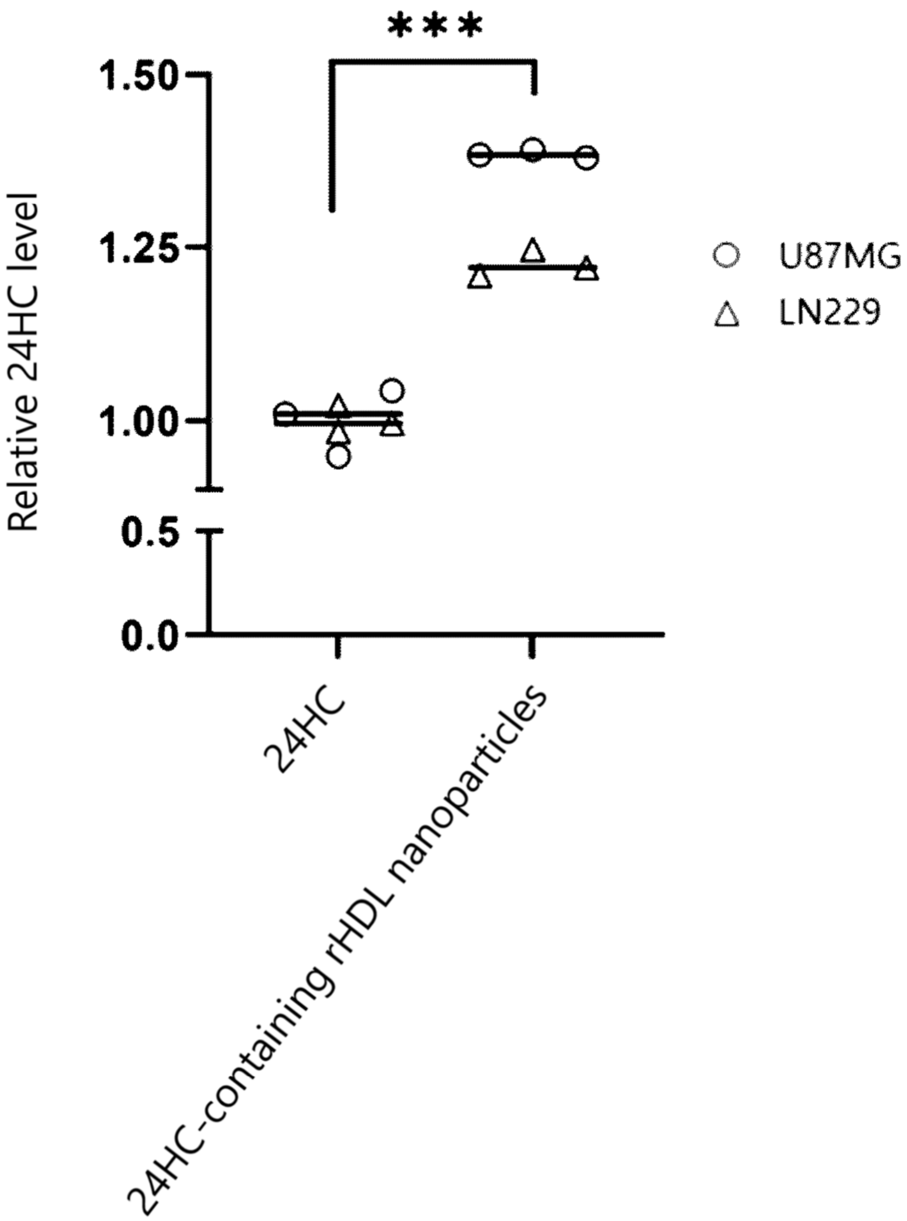
***
1.50
1.25
1.00
0.5
0.0
Relative 24HC level
U87MG
LN229
24HC
24HC-containing rHDL nanoparticles

Figure 34

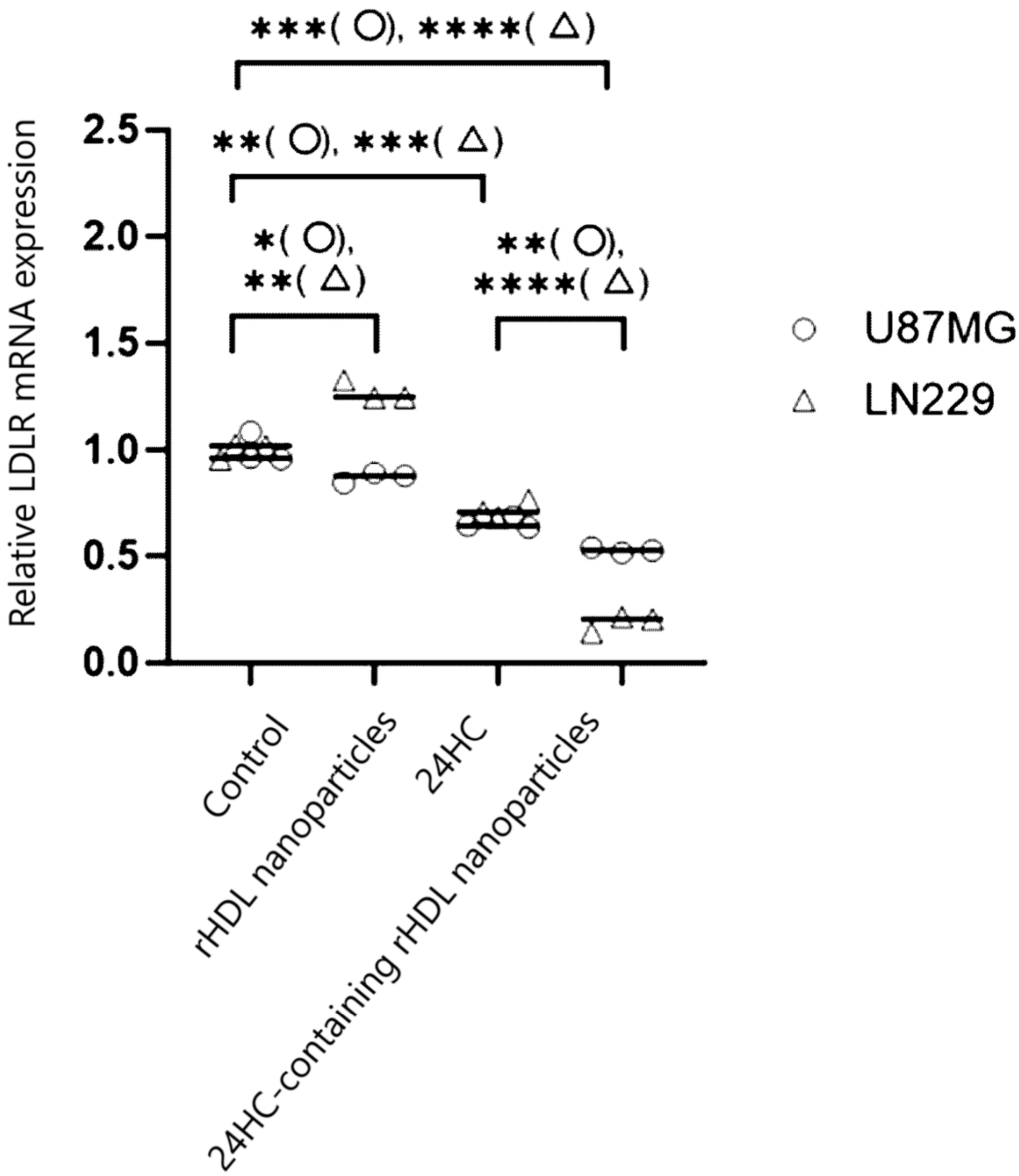
***( ○), ****( △)
**( ○), ***( △)
*( ○),
**( △)
**( ○),
****( △)
2.5
2.0
1.5
1.0
0.5
0.0
Relative LDLR mRNA expression
Control
rHDL nanoparticles
24HC
24HC-containing rHDL nanoparticles
○ U87MG
△ LN229

Figure 35

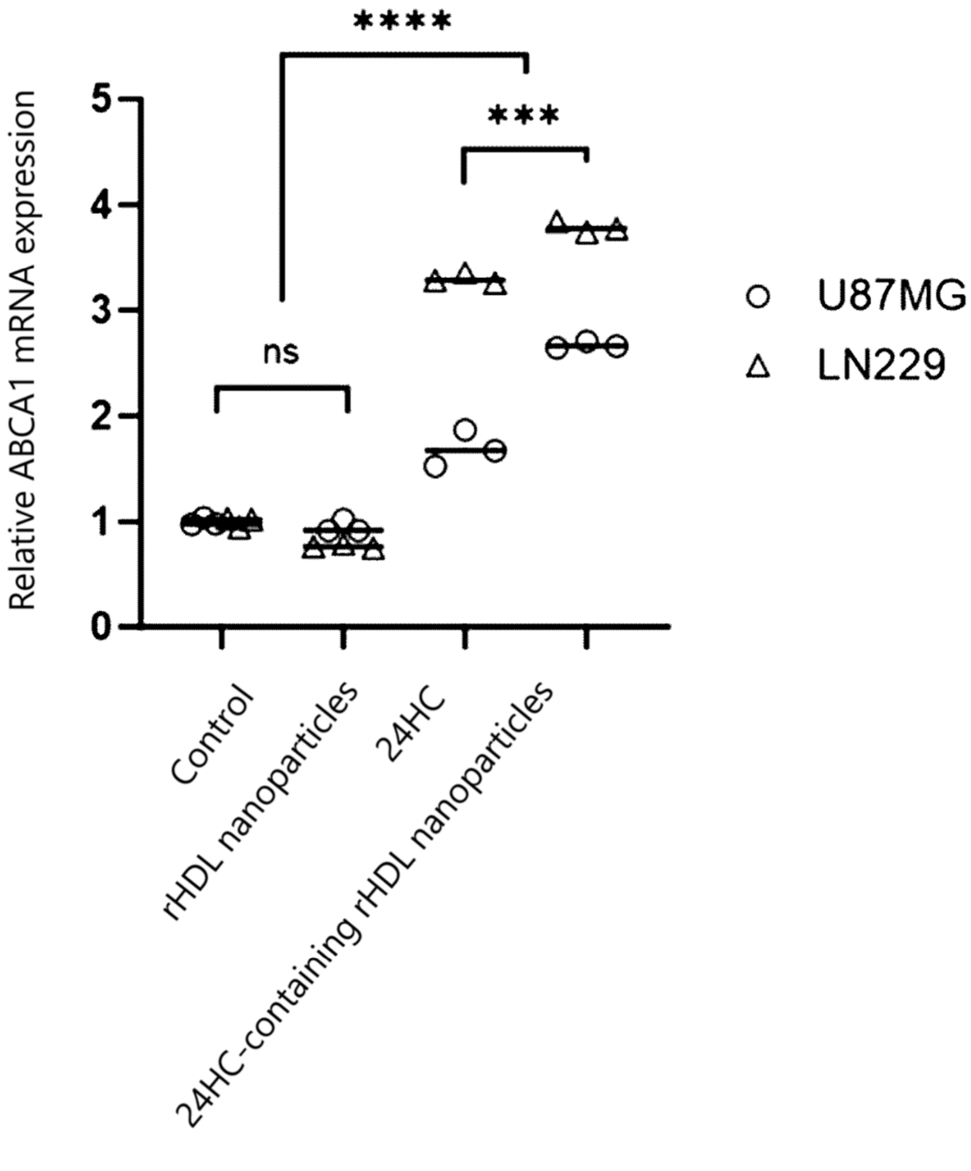
****
***
ns
Relative ABCA1 mRNA expression
5
4
3
2
1
0
Control
rHDL nanoparticles
24HC
24HC-containing rHDL nanoparticles
U87MG
LN229

Figure 36

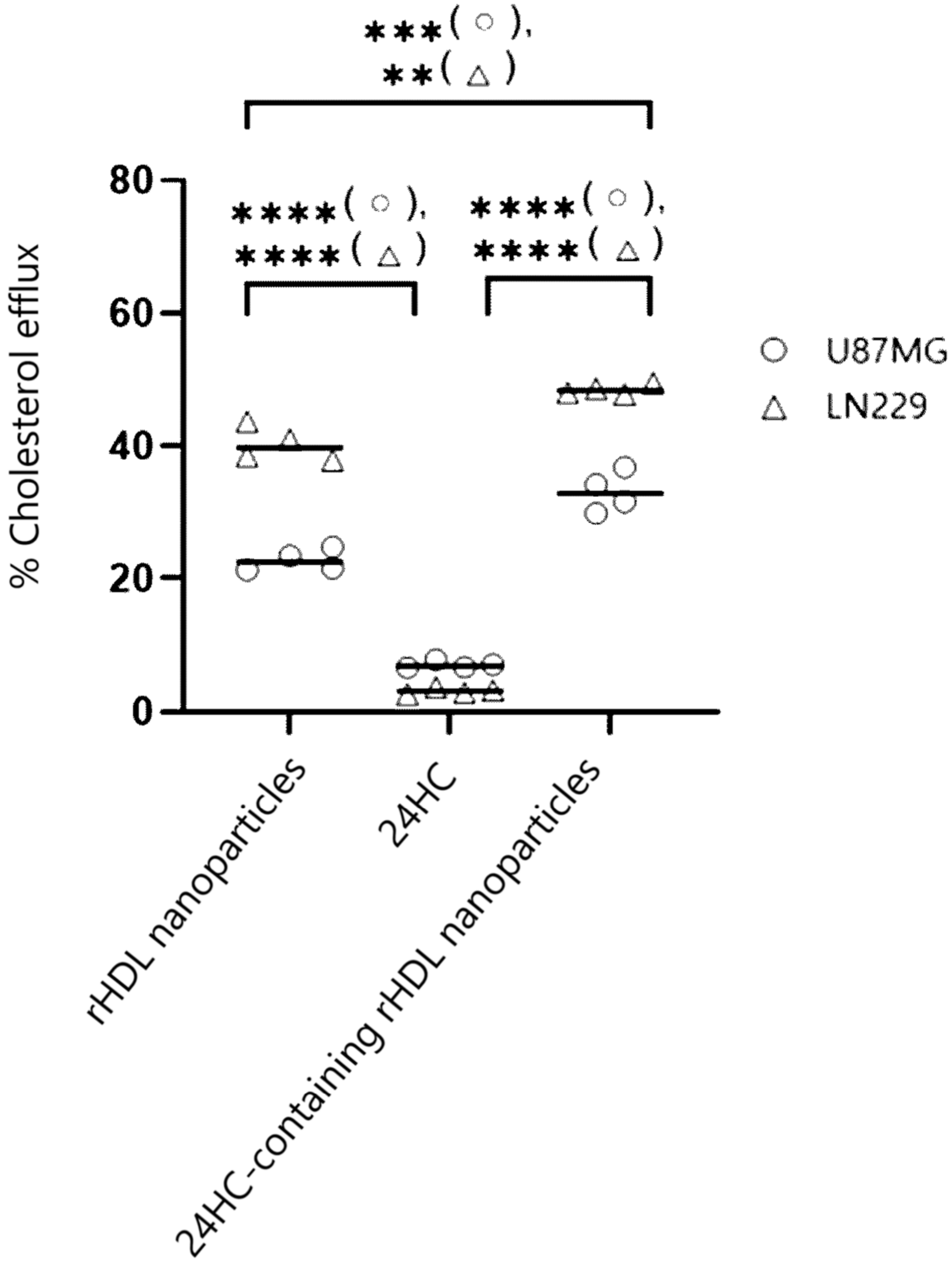
***(○),
**(△)
****(○),
****(△)
****(○),
****(△)
80
60
40
20
0
% Cholesterol efflux
○ U87MG
△ LN229
rHDL nanoparticles
24HC
24HC-containing rHDL nanoparticles

Figure 37

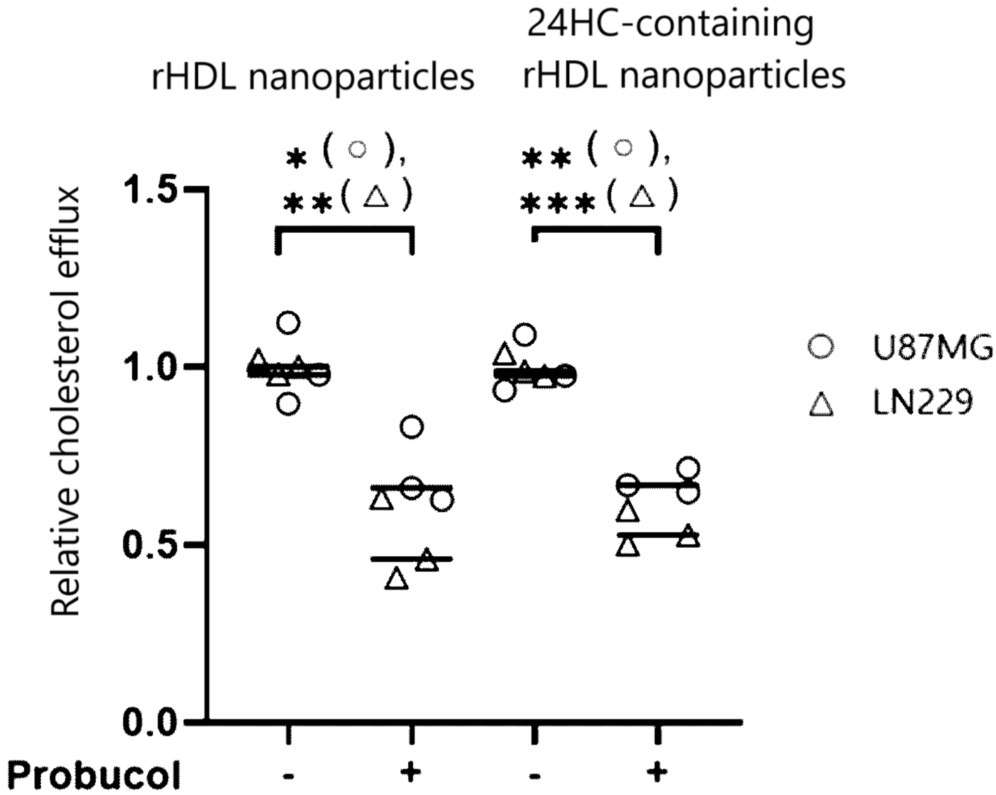
rHDL nanoparticles
24HC-containing rHDL nanoparticles
* ( ○ ),
** ( △ )
** ( ○ ),
*** ( △ )
Relative cholesterol efflux
1.5
1.0
0.5
0.0
Probucol
-
+
-
+
○ U87MG
△ LN229

Figure 38

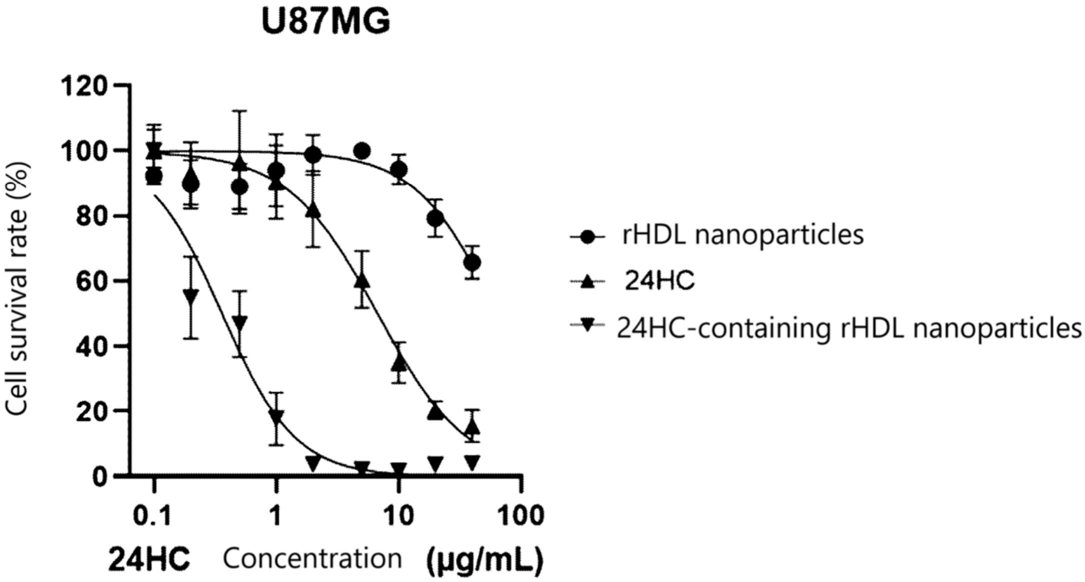


Figure 39

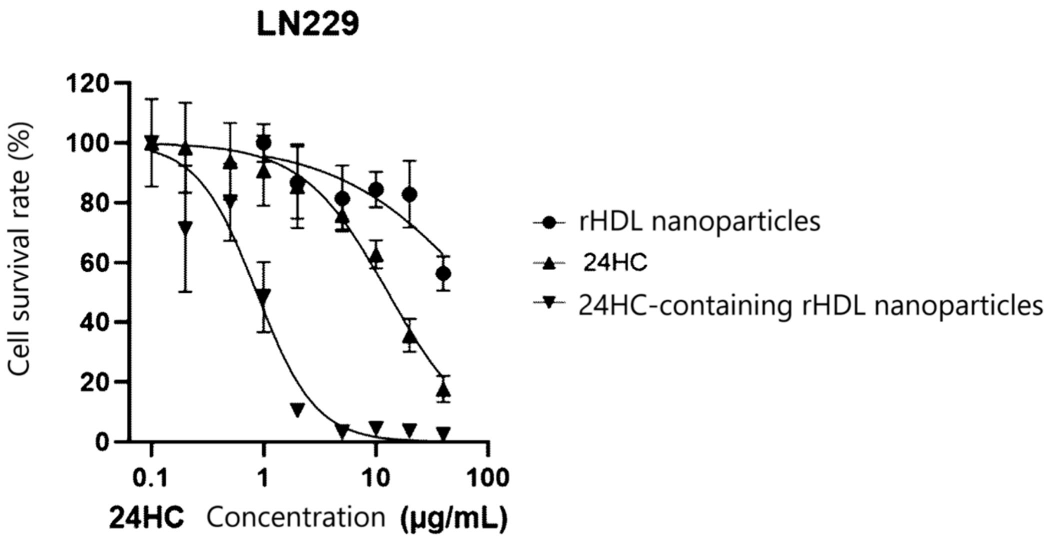

Figure 40

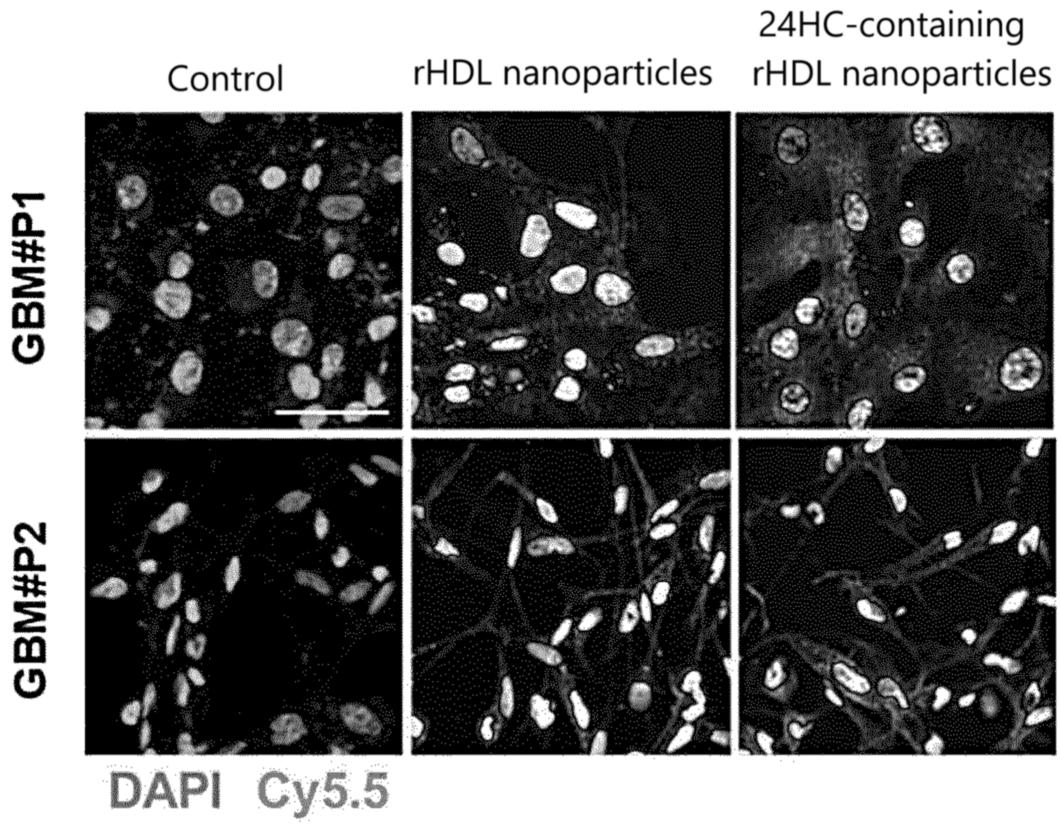
Control
rHDL nanoparticles
24HC-containing
rHDL nanoparticles
GBM#P1
GBM#P2
DAPI Cy5.5

Figure 41

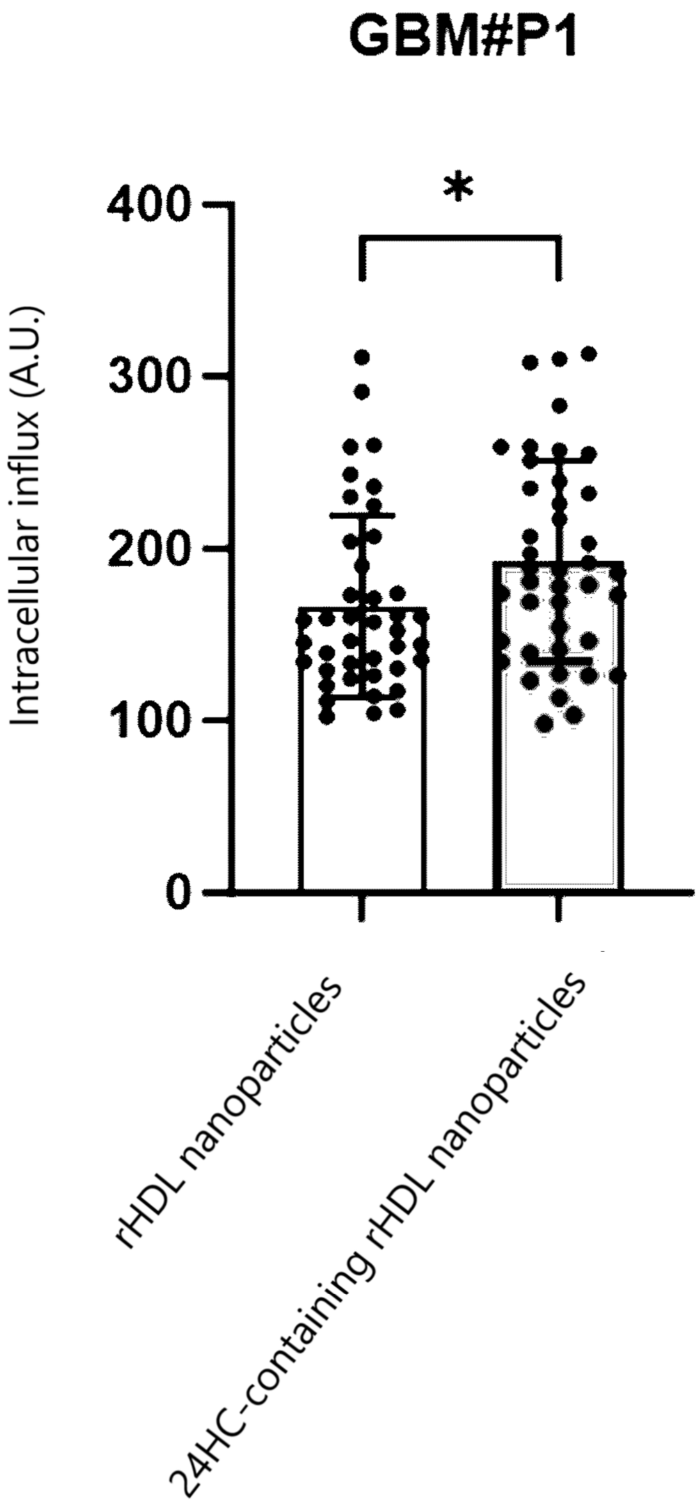
GBM#P1
*
400
300
200
100
0
Intracellular influx (A.U.)
rHDL nanoparticles
24HC-containing rHDL nanoparticles

Figure 42

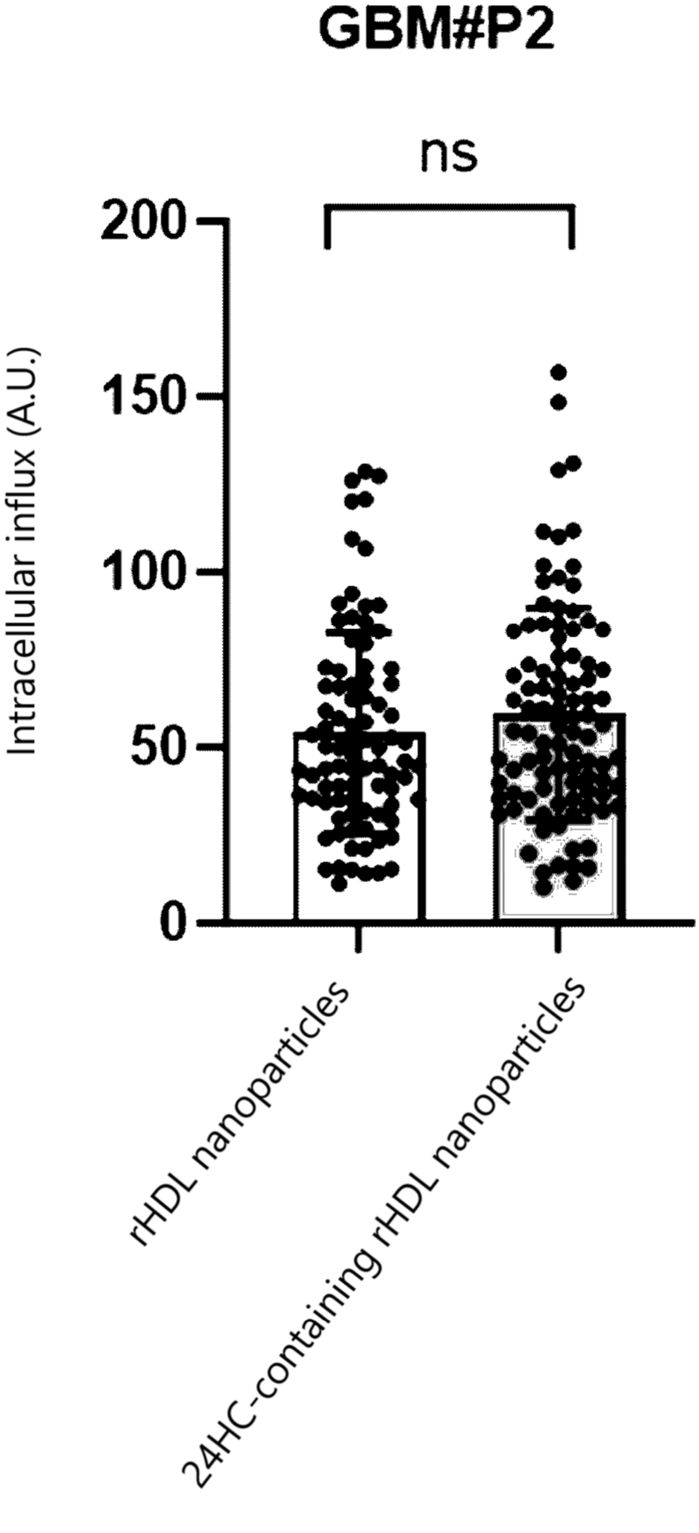
GBM#P2
ns
200
150
100
50
0
Intracellular influx (A.U.)
rHDL nanoparticles
24HC-containing rHDL nanoparticles

Figure 43

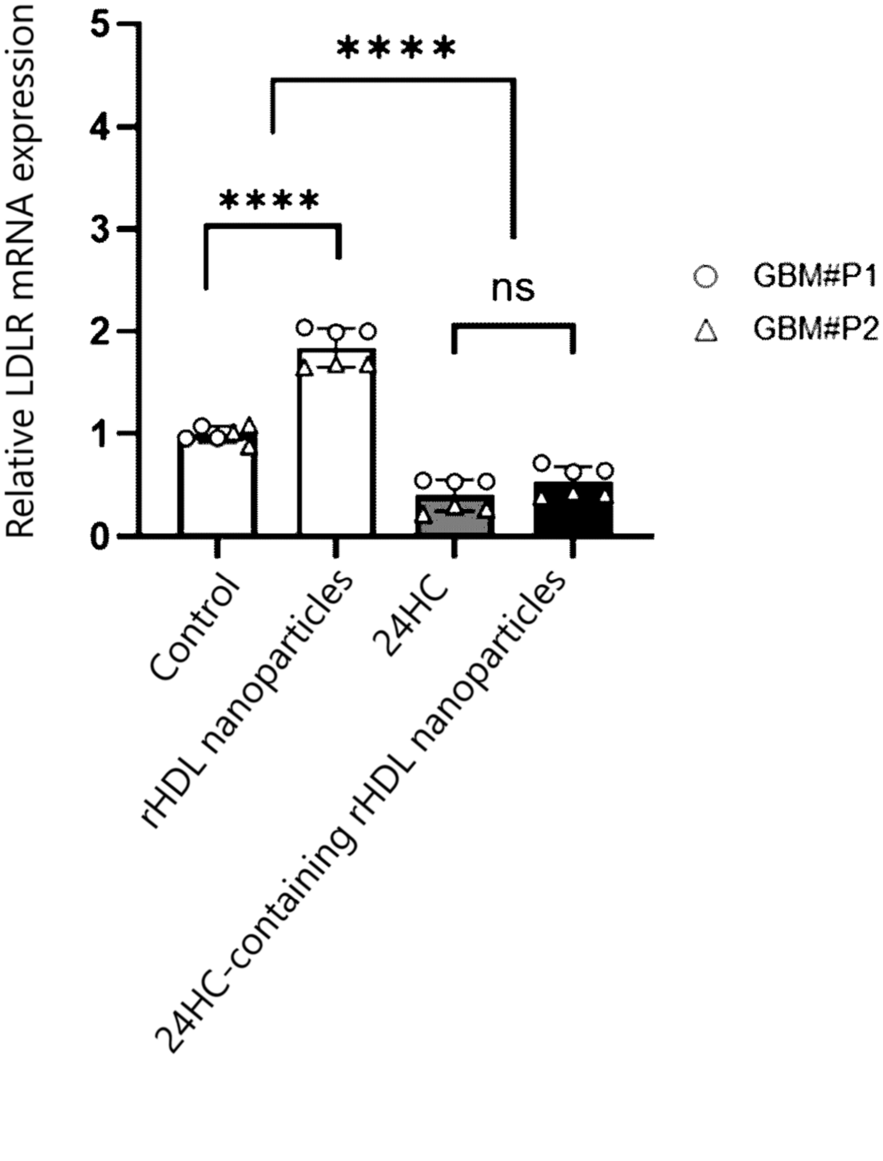

Relative LDLR mRNA expression
5
4
3
2
1
0
****
****
ns
GBM#P1
GBM#P2
Control
rHDL nanoparticles
24HC
24HC-containing rHDL nanoparticles

Figure 44

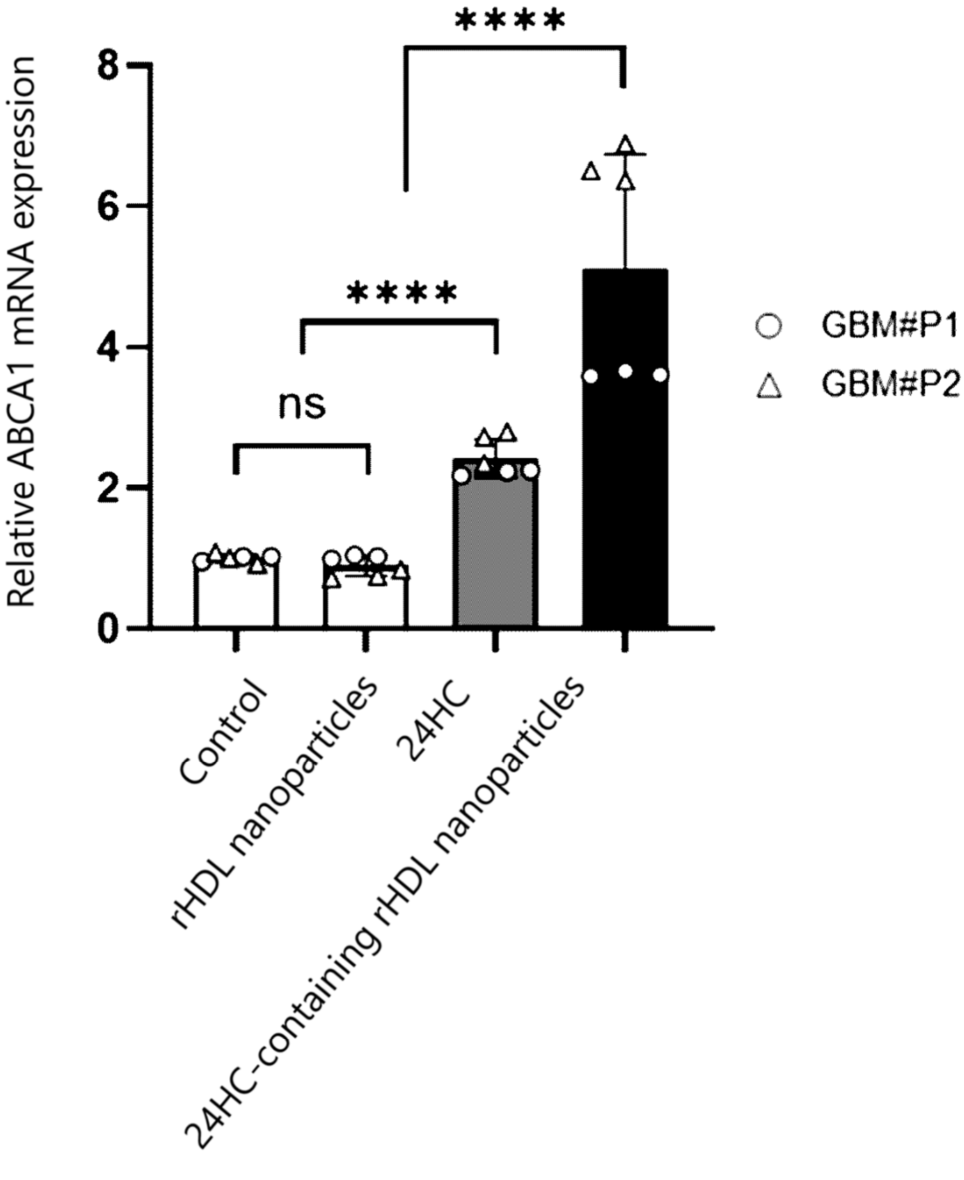
****
****
ns
8
6
4
2
0
Relative ABCA1 mRNA expression
Control
rHDL nanoparticles
24HC
24HC-containing rHDL nanoparticles
GBM#P1
GBM#P2

Figure 45

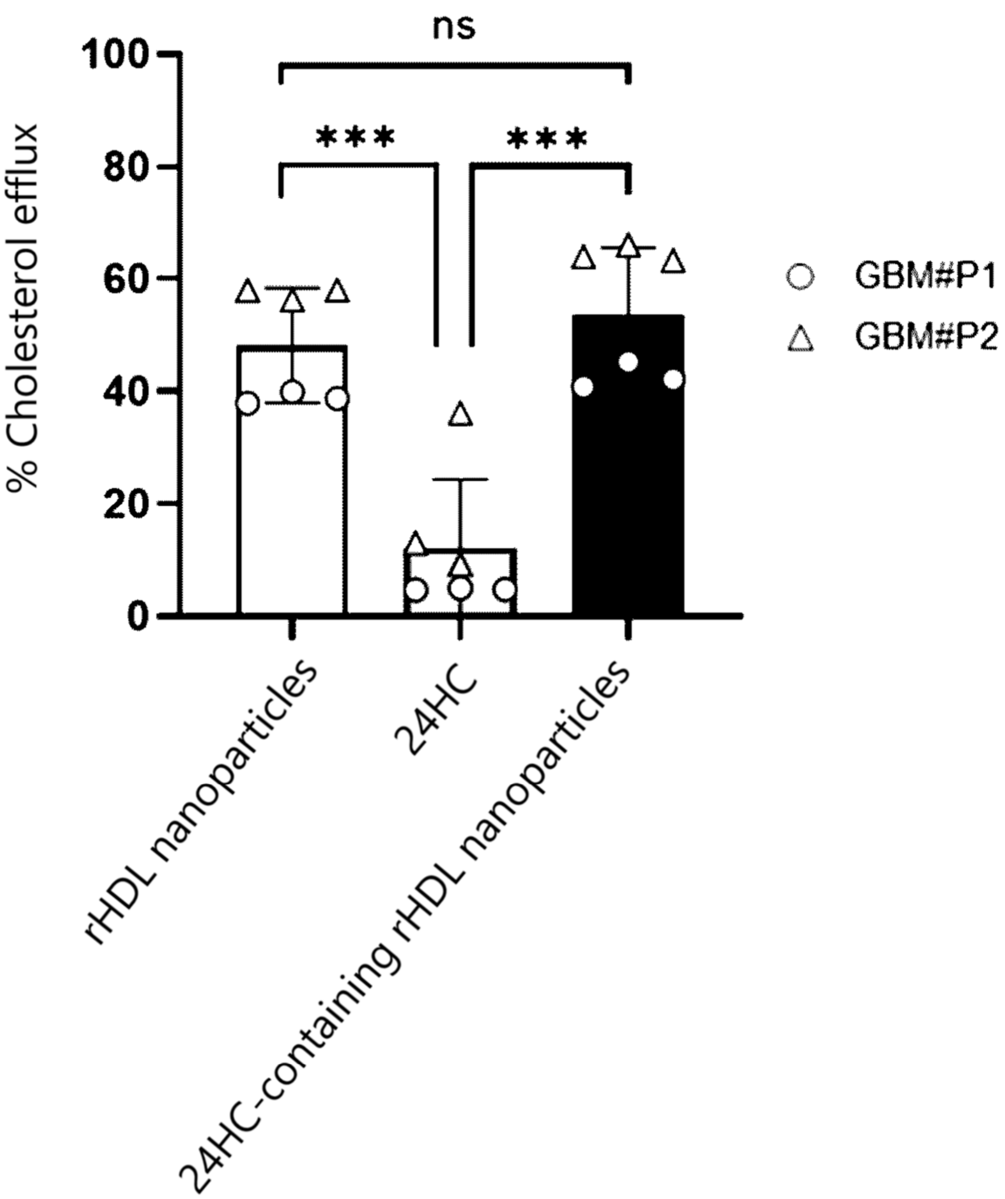
ns
***
***
100
80
60
40
20
0
% Cholesterol efflux
GBM#P1
GBM#P2
rHDL nanoparticles
24HC
24HC-containing rHDL nanoparticles

**Figure 46**

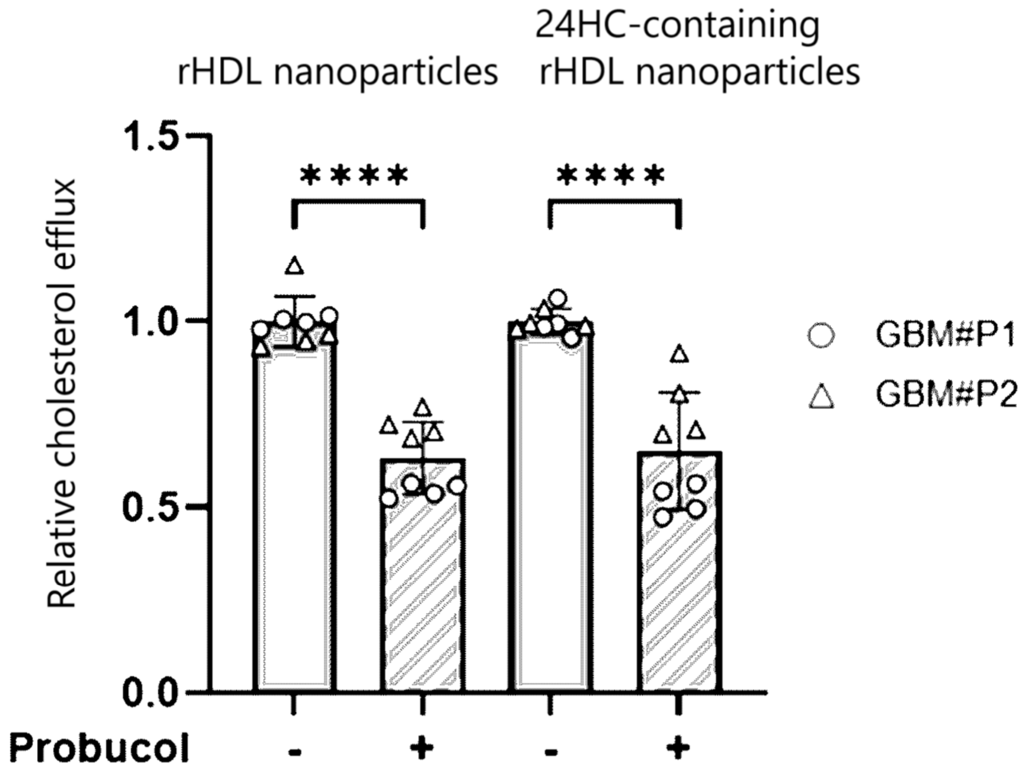
rHDL nanoparticles
24HC-containing rHDL nanoparticles
Relative cholesterol efflux
1.5
1.0
0.5
0.0
****
****
GBM#P1
GBM#P2
Probucol
-
+
-
+

Figure 47

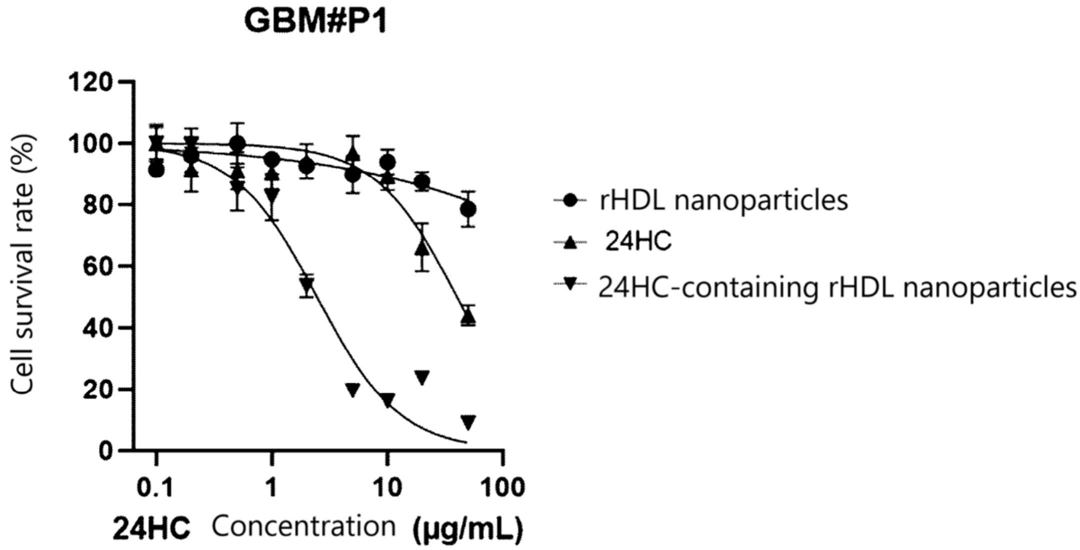


Figure 48

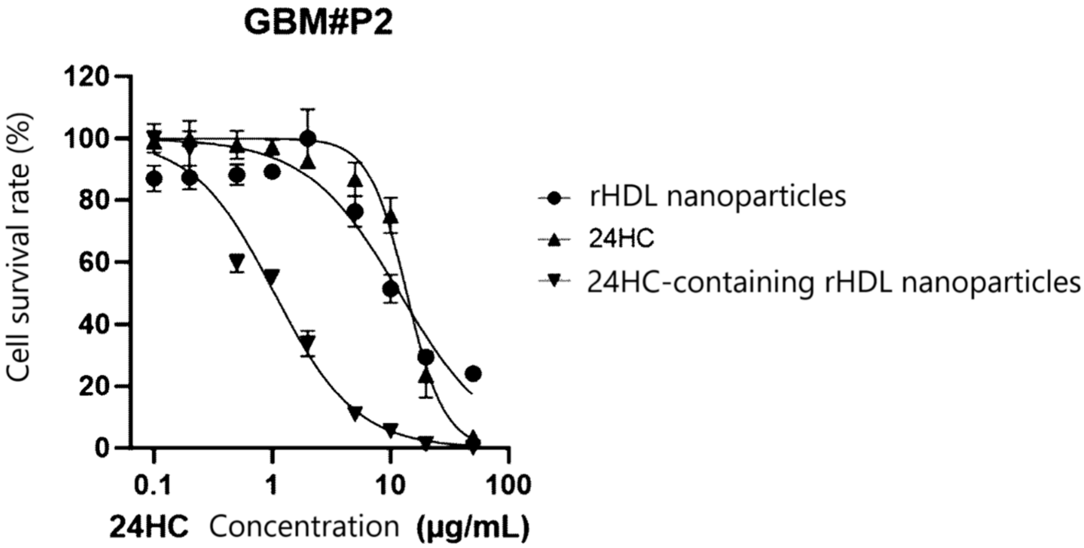

## INTERNATIONAL SEARCH REPORT

International application No. **PCT/KR2024/006052**

**A. CLASSIFICATION OF SUBJECT MATTER**

**A61K 9/51**(2006.01)i; **A61K 31/575**(2006.01)i; **A61P 35/00**(2006.01)i; **A61P 25/28**(2006.01)i

According to International Patent Classification (IPC) or to both national classification and IPC

**B. FIELDS SEARCHED**

Minimum documentation searched (classification system followed by classification symbols)

A61K 9/51(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인지질(phospholipid), 콜레스테롤(cholesterol), 아포지단백질E(apolipoprotein E, ApoE), 재구축 고밀도 지단백(rHDL), 24-하이드록시콜레스테롤(24-hydroxycholesterol), 알츠하이머병(Alzheimer's disease), 뇌종양(brain tumor), 미세유체장치(microfluidic device)

**C. DOCUMENTS CONSIDERED TO BE RELEVANT**

| Category* | Citation of document, with indication, where appropriate, of the relevant passages | Relevant to claim No. |
|---|---|---|
| X<br>Y | HUBIN, E. et al. Apolipoprotein E associated with reconstituted high-density lipoprotein-like particles is protected from aggregation. FEBS Letters. 27 May 2019 (electronic publication), vol. 593, pp. 1144-1153.<br>See abstract; page 1145, left column, third paragraph; page 1146, right column, fifth paragraph – page 1147, left column, first paragraph and page 1149, right column, second paragraph; and figure 1. | 1,7-12<br>2-6,13-27 |
| Y | SODERO, A. O. 24S-hydroxycholesterol: Cellular effects and variations in brain diseases. Journal of Neurochemistry. 2021, vol. 157, pp. 899–918.<br>See page 912, left column, third paragraph; and figure 1. | 2-3,13-19,23,27 |
| Y | MORIN, E. E. et al. Synergetic Effect of rHDL and LXR Agonist on Reduction of Atherosclerosis in Mice. Frontiers in Pharmacology. 16 December 2020, vol. 11, document no. 513031, pp. 1-10.<br>See abstract. | 4-5 |
| Y | CHANG, S. C. et al. ShK toxin: History, structure and therapeutic applications for autoimmune diseases. WikiJournal of Science. 2018, vol. 1, no. 1, pp. 1-13.<br>See page 7, right column, second paragraph. | 6 |

☑ Further documents are listed in the continuation of Box C. ☐ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 August 2024** | **12 August 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2024/006052**

**C. DOCUMENTS CONSIDERED TO BE RELEVANT**

| Category* | Citation of document, with indication, where appropriate, of the relevant passages | Relevant to claim No. |
|---|---|---|
| Y | KIM, Y. T. et al. Single Step Reconstitution of Multifunctional High-Density Lipoprotein-Derived Nanomaterials Using Microfluidics. ACS Nano. 26 November 2013, vol. 7, no. 11, pp. 9975-9983 (inner pp. 1-24).<br>See inner page 4, first paragraph; figure 1; and table 1. | 20-27 |

Form PCT/ISA/210 (second sheet) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **RAIMUND O**. *Life Sci Alliance*, 2022
- **FABIENNE G**. *PNAS*, 2015
- **LEILA PIRMORADI**. *J. Investig. Med.*, 2019
- **MINGZHI HAN**. *EMBO Mol. Med.*, 2020
- **SIGRID NACHTERHAELE**. *Nat. chem. biol.*, 2012
- **RADOSVETA KOLDAMOVA**. *J Biol Chem.*, 2005
- **LEILA PIRMORADI** ; **NAYER SEYFIZADEH** ; **SAEID GHAVAMI** ; **AMIR A ZEKI** ; **SHAHLA SHOJAEI**. Targeting cholesterol metabolism in glioblastoma: a new therapeutic approach in cancer therapy. *J. Investig. Med.*, 2019, vol. 67 (4), 715-719
- **MINGZHI HAN** ; **SHUAI WANG** ; **NING YANG** ; **XU WANG** ; **WENBO ZHAO** ; **HALALA SDIK SAED** ; **THOMAS DAUBON** ; **BIN HUANG** ; **ANJING CHEN** ; **GANG LI**. Therapeutic implications of altered cholesterol homeostasis mediated by loss of CYP46A1 in human glioblastoma. *EMBO Mol. Med.*, 09 January 2020, vol. 12 (1), e10924
- **SIGRID NACHTERGAELE** ; **LAUREL K MYDOCK** ; **KATHIRESAN KRISHNAN** ; **JAYAN RAMMOHAN** ; **PAUL H SCHLESINGER** ; **DOUGLAS F COVEY** ; **RAJAT ROHATGI**. Oxysterols are allosteric activators of the oncoprotein smoothened. *Nat. Chem. Biol.*, 08 January 2012, vol. 8 (2), 211-220
- **RADOSVETA KOLDAMOVA** ; **MATTHIAS STAUFENBIEL** ; **ILIYA LEFTEROV**. Lack of ABCA1 Considerably Decreases Brain ApoE Level and Increases Amyloid Deposition in APP23 Mice. *J Biol Chem.*, 30 December 2005, vol. 280 (52), 43224-43235
- **RAIMUND OBERLE** ; **KRISTINA KUHRER et al.** The HDL particle composition determines its antitumor activity in pancreatic cancer. *Life Sci Alliance*, 16 May 2022, vol. 5 (9), e202101317
- **FABIENNE GUILLAUMOND** ; **GHISLAIN BIDAUT et al.** Cholesterol uptake disruption, in association with chemotherapy, is a promising combined metabolic therapy for pancreatic adenocarcinoma. *PNAS.*, 12 February 2015, vol. 112 (8), 2473-8